(19) Europäisches Patentamt
European Patent Office
Office européen des brevets

(11) **EP 3 458 608 B1**

(12) # EUROPEAN PATENT SPECIFICATION

(45) Date of publication and mention
of the grant of the patent:
**23.07.2025 Bulletin 2025/30**

(21) Application number: **17728306.6**

(22) Date of filing: **16.05.2017**

(51) International Patent Classification (IPC):
***C12Q 1/6886*** (2018.01)

(52) Cooperative Patent Classification (CPC):
**C12Q 1/6886; A61P 35/00; C12Q 2600/106;**
**C12Q 2600/118; C12Q 2600/158**

(86) International application number:
**PCT/US2017/032890**

(87) International publication number:
**WO 2017/201036 (23.11.2017 Gazette 2017/47)**

(54) **STROMAL GENE SIGNATURES FOR DIAGNOSIS AND USE IN IMMUNOTHERAPY**

STROMAGENSIGNATUREN ZUR DIAGNOSE UND VERWENDUNG IN DER IMMUNTHERAPIE

SIGNATURES GÉNÉTIQUES STROMALES DESTINÉES AU DIAGNOSTIC ET À L'UTILISATION EN
IMMUNOTHÉRAPIE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **17.05.2016 US 201662337815 P**

(43) Date of publication of application:
**27.03.2019 Bulletin 2019/13**

(73) Proprietor: **F. Hoffmann-La Roche AG**
**4070 Basel (CH)**

(72) Inventors:
• **HEGDE, Priti**
**South San Francisco, CA 94080-4990 (US)**
• **MOLINERO, Luciana**
**South San Francisco, CA 94080-4990 (US)**
• **MARIATHASAN, Sanjeev**
**South San Francisco, CA 94080-4990 (US)**
• **TURLEY, Shannon**
**South San Francisco, CA 94080-4990 (US)**
• **ASTARITA, Jillian**
**South San Francisco, CA 94080-4990 (US)**
• **CUBAS, Rafael**
**South San Francisco, CA 94080-4990 (US)**
• **YANG, Yagai**
**South San Francisco, CA 94080-4990 (US)**

(74) Representative: **Aoki, Kenichiro et al**
**F. Hoffmann-La Roche AG**
**Patent Department**
**Grenzacherstrasse 124**
**4070 Basel (CH)**

(56) References cited:
**WO-A1-2014/194293      WO-A2-2014/151006**
**WO-A2-2015/118175      US-A1- 2014 271 724**
**US-A1- 2015 071 910**

• **DAVID F. MCDERMOTT ET AL: "Atezolizumab, an**
**Anti-Programmed Death-Ligand 1 Antibody, in**
**Metastatic Renal Cell Carcinoma: Long-Term**
**Safety, Clinical Activity, and Immune Correlates**
**From a Phase Ia Study", JOURNAL OF CLINICAL**
**ONCOLOGY, vol. 34, no. 8, 10 March 2016**
**(2016-03-10), US, pages 833 - 842, XP055391463,**
**ISSN: 0732-183X, DOI: 10.1200/JCO.2015.63.7421**
• **NEUZILLET CINDY ET AL: "Targeting the TGF**
**[beta] pathway for cancer the",**
**PHARMACOLOGY AND THERAPEUTICS, vol.**
**147, 1 March 2015 (2015-03-01), pages 22 - 31,**
**XP029196208, ISSN: 0163-7258, DOI: 10.1016/**
**J.PHARMTHERA.2014.11.001**

EP 3 458 608 B1

• LILLY: "Bristol-Myers Squibb and Lilly Enter Clinical Collaboration Agreement to Evaluate Opdivo (nivolumab) in Combination with Galunisertib in Advanced Solid Tumors", 13 January 2015 (2015-01-13), XP055391705, Retrieved from the Internet <URL:http://files.shareholder.com/downloads/LLY/4825454452x0x802841/8566DD2B-D4B9-4578-83B9-7030CF060309/LLY_News_2015_1_13_Product.pdf> [retrieved on 20170718]

• FEIG C ET AL: "Targeting CXCL12 from FAP-expressing carcinoma-associated fibroblasts synergizes with anti-PD-L1 immunotherapy in pancreatic cancer", PROCEEDINGS NATIONAL ACADEMY OF SCIENCES PNAS, NATIONAL ACADEMY OF SCIENCES, US, vol. 110, no. 50, 10 December 2013 (2013-12-10), pages 20212 - 20217, XP002760317, ISSN: 0027-8424, [retrieved on 20131125], DOI: 10.1073/PNAS.1320318110

**Description**

FIELD OF THE INVENTION

The present invention provides tumor stromal biomarkers and methods for selecting and treating cancer patients with an immunotherapy in combination with a suppressive stromal antagonist.

BACKGROUND OF THE INVENTION

As tumors grow in a tissue they form a tumor microenvironment (TME) comprising a complex mixture of non-malignant cells, including blood endothelial cells (BECs) and lymphatic endothelial cells (LECs), mesenchymal stem cells (MSCs) and their differentiated progeny, cancer-associated fibroblasts (CAFs), pericytes and immune cells, along with the extracellular matrix (ECM) and inflammatory mediators they secrete (Turley, S.J. et al., Nature Reviews Immunology, 2015. 15:669-682). Both healthy tissues and solid tumors have two distinct regions: the parenchyma and a stromal region. The basal lamina, which normally separates these two regions in healthy tissues but is typically poorly defined in tumors. Elements of the TME can interact closely with tumor cells and can influence tumor cell survival, invasiveness and metastatic dissemination, as well as access and responsiveness to therapeutics (Joyce, J. A. & Pollard, J. W. Nat. Rev. Cancer 9:239-252, 2009; Polyak, K. et al., Trends Genet. 25:30-38, 2009; Nakasone, E. S. et al., Cancer Cell 21:488-503, 2012).

The relationship between the TME and the immune system is complex, and tumor cells and various components of their microenvironment can impair protective T cell immunity in diverse ways. For example, T cell migration into the tumor bed can be hindered by the disorganized vasculature and chemotactic cues (Pivarcsi, A. et al., Proc. Natl Acad. Sci. USA 104:19055-19060, 2007) or may encounter inhibitory cells and molecules that can impair their activity. Evidence for the suppressive role of inhibitory factors in the cancer-immunity cycle has been provided by recent clinical studies using neutralizing antibodies that block the inhibitory molecules cytotoxic T lymphocyte antigen 4 (CTLA4; also known as CD152), programmed cell death protein 1 (PD1; also known as CD279) and programmed cell death ligand 1 (PDL1; also known as B7-H1 and CD274) (Turley, S.J. et al., Nature Reviews Immunology, 2015. 15:669-682). The expression of PDL1 is often upregulated on cancer cells, myeloid cells and endothelial cells, and this surface protein inhibits T cell activation and survival upon binding to its receptor PD1 on activated T cells. Therapies that use antibodies to disrupt the PDL1-PD1 interaction to unleash CD8+ T cell-mediated killing of tumor cells have shown promising response rates in several human cancers (Powles, T. et al. Nature 515:558-562, 2014; Herbst, R. S. et al., Nature 515:563-567, 2014;Tumeh, P. C. et al., Nature 515:568-571, 2014). However, many patients do not experience therapeutic benefit, possibly owing to the presence of other immunosuppressive mechanisms in the TME. WO 2014/151006 A2 discloses biomarkers for the treatment of pathological conditions, such as cancer, and method of using PD-1/PD-L1 pathway antagonists.

Accordingly, there is a need for methods for selecting patients who are less likely to respond to immunotherapies and to develop alternative strategies for the treatment of these patients.

BRIEF SUMMARY

The present invention is defined by the apended claims. In the following, all statements of invention not falling under the scope of the claims are to be understood merely as supportive information, even if they are described as "embodiments". They form part of the disclosure, but not of the invention as such.

The present invention provides a method for treating an individual with a disease or disorder, the method comprising: a) determining the presence of a stromal gene signature in a sample from the individual, said signature comprising one or more of *FAP, FN1, MMP2, PDGFRB,* or *THY1,* wherein an increase in the level of expression of the one or more genes in the stroma gene signature relative to a median level identifies an individual for treatment; and b) administering to said individual an effective amount of an immunotherapy and a suppressive stromal antagonist. In some aspects the invention provides a method for improving an immunotherapy of an individual with a disease or disorder, the method comprising: a) determining the presence of a stromal gene signature in a sample from the individual, said signature comprising one or more of *FAP, FN1, MMP2, PDGFRB,* or *THY1,* wherein an increase in the level of expression of the one or more genes in the stroma gene signature relative to a median level identifies an individual for treatment with a suppressive stromal antagonist; and b) administering to said individual identified for treatment with a suppressive stromal antagonist in step a) an effective amount of an immunotherapy and a suppressive stromal antagonist.

In some aspects, the invention provides a method for selecting an individual with a disease or disorder who is less likely to respond to immunotherapy alone, the method comprising determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual for treatment with an immunotherapy and with a suppressive stromal antagonist. In some aspects

the invention provides a method for identifying an individual with a disease or disorder who is more likely to exhibit benefit from treatment with an immunotherapy and with a suppressive stromal antagonist the method comprising determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual having a suppressive stroma wherein the presence of a stromal gene signature in a sample from the individual indicates the individual is more likely to exhibit an increased clinical benefit from an immunotherapy and a suppressive stromal antagonist. In some aspects, the invention provides a method for selecting a treatment an individual with a disease or disorder, the method comprising determining the presence of a stromal gene signature in a sample from the individual, said signature comprising one or more of *FAP, FN1, MMP2, PDGFRB,* or *THY,* wherein an increase in the level of expression of the one or more genes in the stromal gene signature relative to a median level identifies an individual having a suppressive stroma; wherein the presence of a stromal gene signature in a sample from the individual indicates the individual is more likely to exhibit an increased clinical benefit from an immunotherapy and a suppressive stromal antagonist. In some embodiments of the above aspects, the increased clinical benefit further comprises a relative increase in one or more of the following: overall survival (OS), progression free survival (PFS), complete response (CR), partial response (PR) and combinations thereof.

In some aspects, the invention provides a method for monitoring the efficacy of a combination treatment comprising an immunotherapy and a suppressive stromal antagonist, the method comprising determining the presence of a stromal gene signature in a sample from an individual undergoing treatment with an immunotherapy and a suppressive stromal antagonist at one or more time points; wherein the stromal gene signature comprises an increase in the level of expression of one or more genes of *FAP, FN1, MMP2, PDGFRB,* or *THY* relative to a median level; wherein an increased clinical benefit and/or a decrease in the presence of the stromal gene signature indicates an effective treatment. In some aspects, the invention provides a method for monitoring the efficacy of a combination treatment comprising an immunotherapy and a suppressive stromal antagonist, the method comprising a) determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual for treatment; and b) administering to said individual an effective amount of an immunotherapy and a suppressive stromal antagonist; and c) determining the presence of a stromal gene signature in a sample from the individual at one or more time points; wherein an increased clinical benefit and/or a decrease in the presence of the stromal gene signature indicates an effective treatment. In some embodiments of the above aspects, the increased clinical benefit comprises a relative increase in one or more of the following: overall survival (OS), progression free survival (PFS), complete response (CR), partial response (PR) and combinations thereof.

In some embodiments, the disease or disorder is cancer. In some embodiments, the cancer is selected from the group consisting of non-small cell lung cancer, small cell lung cancer, renal cell cancer, colorectal cancer, ovarian cancer, breast cancer, metastatic breast cancer, triple-negative breast cancer, melanoma, pancreatic cancer, gastric carcinoma, bladder cancer, urothelial bladder cancer, esophageal cancer, mesothelioma, melanoma, head and neck cancer, thyroid cancer, sarcoma, prostate cancer, glioblastoma, cervical cancer, thymic carcinoma, leukemia, lymphomas, myelomas, mycoses fungoids, merkel cell cancer, and other hematologic malignancies.

In some embodiments of the above aspects, the cancer is urothelial bladder cancer (UBC) and the stromal gene signature comprises one or more of *FAP, FN1, MMP2,* or *PDGFRB.* In some embodiments, the stromal gene signature for UBC further comprises one or more of *DKK3, PDGFB, NUAK1, FGF1, PDL1M4* or *LRRC32.* In some embodiments, the cancer is non-small cell lung cancer (NSCLC) and the stromal gene signature comprises one or more of *FAP, FN1, MMP2, PDGFRB,* or *THY.* In some embodiments, the cancer is renal cell cancer (RCC) and the stromal gene signature comprises one or more of *FAP, FN1, MMP2, PDGFRB,* or *THY.* In some embodiments, the stromal gene signature for RCC further comprises *LUM* and/or *POSTN.* In some embodiments, the cancer is melanoma and the stromal gene signature comprises one or more of *FAP, FN1, MMP2, PDGFRB,* or *THY1.* In some embodiments, the cancer is triple-negative breast cancer (TNBC) and the stromal gene signature comprises one or more of *FAP, FN1, MMP2, PDGFRB,* or *THY1.* In some embodiments, the stromal gene signature for TNBC further comprises one or more of *MMP11, BGN,* or *COL5A1.* In some embodiments, the cancer is ovarian cancer and the stromal gene signature comprises one or more of *FAP, FN1, MMP2, PDGFRB,* or *THY1.* In some embodiments, the stromal gene signature for ovarian cancer further comprises one or more of *POSTN, LOX,* or *TIMP3.* In some embodiments, the stromal gene signature further comprises *TGFβ.*

In some embodiments of the above aspects, the sample obtained from the individual is selected from the group consisting of tissue, whole blood, plasma, serum and combinations thereof. In some embodiments, the tissue sample is a tumor tissue sample. In some embodiments, the tumor tissue sample comprises tumor cells, tumor infiltrating immune cells, stromal cells and any combinations thereof. In some embodiments, the tissue sample is formalin fixed and paraffin embedded, archival, fresh or frozen. In some embodiments, the sample is whole blood. In some embodiments, the whole blood comprises immune cells, circulating tumor cells and any combinations thereof. In some embodiments, a sample is obtained prior to treatment with the immunotherapy or after treatment with the immunotherapy. In some embodiments, a sample is obtained prior to treatment with the suppressive stromal antagonist.

In the present invention, the immunotherapy comprises a PD-L1 binding antibody.

In some embodiments of the above aspects, the immunotherapy comprises a CD28, OX40, GITR, CD137, CD27, CD40, ICOS, HVEM, NKG2D, MICA, 2B4, IL-2, IL-12, IFN$\gamma$, IFN$\alpha$, TNF$\alpha$, IL-1, CDN, HMGB1, or TLR agonist. In some embodiments, the immunotherapy comprises a CTLA-4, PD-L1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist. In some embodiments, the immunotherapy is a PD-L1 axis antagonist. In some embodiments, the PD-L1 axis binding antagonist is a PD-L1 binding antagonist. In some embodiments, the PD-L1 binding antagonist inhibits the binding of PD-L1 to its ligand binding partners. In some embodiments, the PD-L1 binding antagonist inhibits the binding of PD-L1 to PD-1. In some embodiments, the PD-L1 binding antagonist inhibits the binding of PD-L1 to B7-1. In some embodiments, the PD-L1 binding antagonist inhibits the binding of PD-L1 to both PD-1 and B7-1. In some embodiments, the PD-L1 binding antagonist is an antibody. In some embodiments, the antibody is a monoclonal antibody. In some embodiments, the antibody is a human, humanized or chimeric antibody. In some embodiments, the PD-L1 axis binding antagonist is a PD-1 binding antagonist. In some embodiments, the PD-1 binding antagonist inhibits the binding of PD-1 to its ligand binding partners. In some embodiments, the PD-1 binding antagonist inhibits the binding of PD-1 to PD-L1. In some embodiments, the PD-1 binding antagonist inhibits the binding of PD-1 to PD-L2. In some embodiments, the PD-1 binding antagonist inhibits the binding of PD-1 to both PD-L1 and PD-L2. In some embodiments, the PD-1 binding antagonist is an antibody. In some embodiments, the antibody is a monoclonal antibody. In some embodiments, the antibody is a human, humanized or chimeric antibody.

In the present invention, the suppressive stromal antagonist is a TGF$\beta$ binding antibody.

In some embodiments of the above aspects, the suppressive stromal antagonist is a TGF$\beta$, PDPN, LAIR-1, SMAD, ALK, connective tissue growth factor (CTGF/CCN2), endothelial-1 (ET-1), AP-1, IL-13, PDGF, LOXL2, endoglin (CD105), FAP, podoplanin (GP38), VCAM1 (CD106), THY1, $\beta$1 integrin (CD29), PDGFR$\alpha$ (CD140$\alpha$), PDGFR$\beta$ (CD140$\beta$), vimentin, $\alpha$SMA (ACTA2), desmin, endosialin (CD248) or FSP1 (S100A4) antagonist. In some embodiments, the suppressive stromal antagonist is pirfenidone, galunisertib or nintedanib. In some embodiments, the suppressive stromal antagonist is a TGF$\beta$ antagonist. In some embodiments, the suppressive stromal antagonist is a TGF$\beta$ binding antagonist. In some embodiments, the TGF$\beta$ binding antagonist inhibits the binding of TGF$\beta$ to its ligand binding partners. In some embodiments, the TGF$\beta$ binding antagonist inhibits the binding of TGF$\beta$ to a cellular receptor for TGF$\beta$. In some embodiments, the TGF$\beta$ binding antagonist inhibits activation of TGF$\beta$. In some embodiments, the TGF$\beta$ binding antagonist is an antibody. In some embodiments, the antibody is a monoclonal antibody. In some embodiments, the antibody is a human, humanized or chimeric antibody. In some embodiments, treatment with the suppressive stromal antagonist allows increased immune cell infiltration in a tumor. In some embodiments, the increased immune cell infiltration is an increased infiltration of one or more of T cells, B cells, macrophages, or dendritic cells. In some embodiments, the T cells are CD8+ T cells and/or $T_{eff}$ cells. In some embodiments, the individual is resistant to immunotherapy prior to treatment with the suppressive stromal antagonist. In some embodiments, the individual has already been administered monotherapy immunotherapy.

In some embodiments of the above aspects, the stromal gene signature is detected in the sample using a method selected from the group consisting of FACS, Western blot, ELISA, immunoprecipitation, immunohistochemistry, immunofluorescence, radioimmunoassay, dot blotting, immunodetection methods, HPLC, surface plasmon resonance, optical spectroscopy, one or more reagents for determining the presence of a stromal gene signature in a sample from an individual mass spectrometry, HPLC, qPCR, RT-qPCR, multiplex qPCR or RT-qPCR, RNA-seq, microarray analysis, SAGE, MassARRAY technique, and FISH, and combinations thereof. In some embodiments, the stromal gene signature is detected in the sample by protein expression. In some embodiments, protein expression is determined by immunohistochemistry (IHC). In some embodiments, the stromal gene signature is detected using an antibody. In some embodiments, the stromal gene signature is detected as a weak staining intensity by IHC. In some embodiments, the stromal gene signature is detected as a moderate staining intensity by IHC. In some embodiments, the stromal gene signature is detected as a strong staining intensity by IHC. In some embodiments, the stromal gene signature is detected on tumor cells, tumor infiltrating immune cells, stromal cells and any combinations thereof. In some embodiments, staining is membrane staining, cytoplasmic staining and combinations thereof. In some embodiments, absence of the stromal gene signature is detected as absent or no staining in the sample. In some embodiments, the presence of the stromal gene signature is detected as any staining in the sample. In some embodiments, the stromal gene signature is detected in the sample by nucleic acid expression. In some embodiments, the nucleic acid expression is determined using qPCR, RT-qPCR, multiplex qPCR or RT-qPCR, RNA-seq, microarray analysis, SAGE, MassARRAY technique, or FISH.

In some embodiments of the above aspects, the median levels of the stromal gene signature is selected from the group consisting of (1) the level of the stromal gene signature from a reference population; (2) the level of the stromal gene signature from a population of complete responders and/or partial responders to the immunotherapy; and (3) the level of the stromal gene signature from the individual at a second time point prior to the first time point. In some embodiments, the change in the level(s) of the stromal gene signature in the biological sample compared to the median levels is an increase in the levels.

In some aspects, the present disclosure provides a diagnostic kit comprising one or more reagents for determining the presence of a stromal gene signature in a sample from an individual with a disease or disorder who is less likely to respond to immunotherapy alone, wherein the presence of a stromal gene signature identifies an individual who is more likely to exhibit benefit from treatment with an immunotherapy and a suppressive stromal antagonist. In some aspects, the invention provides a diagnostic kit for selecting a treatment for an individual with a disease or disorder who is less likely to respond to immunotherapy alone, the kit comprising one or more reagents for determining the presence of a stromal gene signature in a sample from an individual in need of an immunotherapy, said signature comprising one or more of *FAP, FN1, MMP2, PDGFRB,* or *THY*; wherein the presence of a stromal gene signature identifies an individual who is more likely to exhibit benefit from treatment with an immunotherapy and a suppressive stromal antagonist. In some aspects, the invention provides a kit for monitoring the efficacy of a combination treatment comprising an immunotherapy and treatment with a suppressive stromal antagonist, the kit comprising one or more reagents for determining the presence of a stromal gene signature in a sample from an individual undergoing treatment with an immunotherapy and a suppressive stromal antagonist, said signature comprising one or more of *FAP, FN1, MMP2, PDGFRB,* or *THY*; wherein an increased clinical benefit and/or a decrease in the presence of the stromal gene signature indicates an effective treatment. In some embodiments, the increased clinical benefit comprises a relative increase in one or more of the following: overall survival (OS), progression free survival (PFS), complete response (CR), partial response (PR) and combinations thereof.

In some embodiments, the cancer is selected from the group consisting of non-small cell lung cancer, small cell lung cancer, renal cell cancer, colorectal cancer, ovarian cancer, breast cancer, metastatic breast cancer, triple-negative breast cancer, melanoma, pancreatic cancer, gastric carcinoma, bladder cancer, urothelial bladder cancer, esophageal cancer, mesothelioma, melanoma, head and neck cancer, thyroid cancer, sarcoma, prostate cancer, glioblastoma, cervical cancer, thymic carcinoma, leukemia, lymphomas, myelomas, mycoses fungoids, merkel cell cancer, and other hematologic malignancies.

In some embodiments of the above kits, the cancer is urothelial bladder cancer (UBC) and the stromal gene signature comprises one or more of *FAP, FN1, MMP2,* or *PDGFRB*. In some embodiments, the stromal gene signature for UBC further comprises one or more of *DKK3, PDGFB, NUAK1, FGF1, PDL1M4* or *LRRC32*. In some embodiments, the cancer is non-small cell lung cancer (NSCLC) and the stromal gene signature comprises one or more of *FAP, FN1, MMP2, PDGFRB,* or *THY*. In some embodiments, the cancer is renal cell cancer (RCC) and the stromal gene signature comprises one or more of *FAP, FN1, MMP2, PDGFRB,* or *THY*. In some embodiments, the stromal gene signature for RCC further comprises *LUM* and/or *POSTN*. In some embodiments, the cancer is melanoma and the stromal gene signature comprises one or more of *FAP, FN1, MMP2, PDGFRB,* or *THY1*. In some embodiments, the cancer is triple-negative breast cancer (TNBC) and the stromal gene signature comprises one or more of *FAP, FN1, MMP2, PDGFRB,* or *THY1*. In some embodiments, the stromal gene signature for TNBC further comprises one or more of *MMP11, BGN,* or *COL5A1*. In some embodiments, the cancer is ovarian cancer and the stromal gene signature comprises one or more of *FAP, FN1, MMP2, PDGFRB,* or *THY1*. In some embodiments, the stromal gene signature for ovarian cancer further comprises one or more of *POSTN, LOX,* or *TIMP3*. In some embodiments, the stromal gene signature further comprises *TGFβ*.

In some embodiments of the above kits, the sample obtained from the individual is selected from the group consisting of tissue, whole blood, plasma, serum and combinations thereof. In some embodiments, the tissue sample is a tumor tissue sample. In some embodiments, the tumor tissue sample comprises tumor cells, tumor infiltrating immune cells, stromal cells and any combinations thereof. In some embodiments, the tissue sample is formalin fixed and paraffin embedded, archival, fresh or frozen. In some embodiments, the sample is whole blood. In some embodiments, the whole blood comprises immune cells, circulating tumor cells and any combinations thereof. In some embodiments, a sample is obtained prior to treatment with the immunotherapy or after treatment with the immunotherapy. In some embodiments, a sample is obtained prior to treatment with the suppressive stromal antagonist. In some embodiments, the immunotherapy comprises a CD28, OX40, GITR, CD137, CD27, CD40, ICOS, HVEM, NKG2D, MICA, 2B4, IL-2, IL-12, IFNγ, IFNα, TNFα, IL-1, CDN, HMGB1, or TLR agonist. In some embodiments, the immunotherapy comprises a CTLA-4, PD-L1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist. In some embodiments, the immunotherapy is a PD-L1 axis antagonist. In some embodiments, the PD-L1 axis binding antagonist is a PD-L1 binding antagonist. In some embodiments, the PD-L1 binding antagonist inhibits the binding of PD-L1 to its ligand binding partners. In some embodiments, the PD-L1 binding antagonist inhibits the binding of PD-L1 to PD-1. In some embodiments, the PD-L1 binding antagonist inhibits the binding of PD-L1 to B7-1. In some embodiments, the PD-L1 binding antagonist inhibits the binding of PD-L1 to both PD-1 and B7-1. In some embodiments, the PD-L1 binding antagonist is an antibody. In some embodiments, the antibody is a monoclonal antibody. In some embodiments, the antibody is a human, humanized or chimeric antibody. In some embodiments, the PD-L1 axis binding antagonist is a PD-1 binding antagonist. In some embodiments, the PD-1 binding antagonist inhibits the binding of PD-1 to its ligand binding partners. In some embodiments, the PD-1 binding antagonist inhibits the binding of PD-1 to PD-L1. In some embodiments, the PD-1 binding antagonist inhibits the binding of PD-1 to PD-L2. In some embodiments, the PD-1 binding antagonist inhibits the binding of PD-1 to both PD-L1 and PD-L2. In some embodiments, the PD-1 binding antagonist is an antibody. In some embodiments, the antibody is a monoclonal antibody. In some embodiments, the antibody is a human, humanized or chimeric antibody.

In some embodiments of the above kits, the suppressive stromal antagonist is a TGFβ, PDPN, LAIR-1, SMAD, ALK, connective tissue growth factor (CTGF/CCN2), endothelial-1 (ET-1), AP-1, IL-13, PDGF, LOXL2, endoglin (CD105), FAP, podoplanin (GP38), VCAM1 (CD106), THY1, β1 integrin (CD29), PDGFRα (CD140α), PDGFRβ (CD140β), vimentin, αSMA (ACTA2), desmin, endosialin (CD248) or FSP1 (S 100A4) antagonist. In some embodiments, the suppressive stromal antagonist is pirfenidone, galunisertib or nintedanib. In some embodiments, the suppressive stromal antagonist is a TGFβ antagonist. In some embodiments, the suppressive stromal antagonist is a TGFβ binding antagonist. In some embodiments, the TGFβ binding antagonist inhibits the binding of TGFβ to its ligand binding partners. In some embodiments, the TGFβ binding antagonist inhibits the binding of TGFβ to a cellular receptor for TGFβ. In some embodiments, the TGFβ binding antagonist inhibits activation of TGFβ. In some embodiments, the TGFβ binding antagonist is an antibody. In some embodiments, the antibody is a monoclonal antibody. In some embodiments, the antibody is a human, humanized or chimeric antibody. In some embodiments, treatment with the suppressive stromal antagonist allows increased immune cell infiltration in a tumor. In some embodiments, the increased immune cell infiltration is an increased infiltration of one or more of T cells, B cells, macrophages, or dendritic cells. In some embodiments, the T cells are CD8+ T cells and/or $T_{eff}$ cells. In some embodiments, the individual is resistant to immunotherapy prior to treatment with the suppressive stromal antagonist. In some embodiments, the individual has already been administered monotherapy immunotherapy.

In some embodiments of the above kits, the stromal gene signature is detected in the sample using a method selected from the group consisting of FACS, Western blot, ELISA, immunoprecipitation, immunohistochemistry, immunofluorescence, radioimmunoassay, dot blotting, immunodetection methods, HPLC, surface plasmon resonance, optical spectroscopy, one or more reagents for determining the presence of a stromal gene signature in a sample from an individual mass spectrometry, HPLC, qPCR, RT-qPCR, multiplex qPCR or RT-qPCR, RNA-seq, microarray analysis, SAGE, MassARRAY technique, and FISH, and combinations thereof. In some embodiments, the stromal gene signature is detected in the sample by protein expression. In some embodiments, protein expression is determined by immunohistochemistry (IHC). In some embodiments, the stromal gene signature is detected using an antibody. In some embodiments, the stromal gene signature is detected as a weak staining intensity by IHC. In some embodiments, the stromal gene signature is detected as a moderate staining intensity by IHC. In some embodiments, the stromal gene signature is detected as a strong staining intensity by IHC. In some embodiments, the stromal gene signature is detected on tumor cells, tumor infiltrating immune cells, stromal cells and any combinations thereof. In some embodiments, staining is membrane staining, cytoplasmic staining and combinations thereof. In some embodiments, absence of the stromal gene signature is detected as absent or no staining in the sample. In some embodiments, the presence of the stromal gene signature is detected as any staining in the sample. In some embodiments, the stromal gene signature is detected in the sample by nucleic acid expression. In some embodiments, the nucleic acid expression is determined using qPCR, RT-qPCR, multiplex qPCR or RT-qPCR, RNA-seq, microarray analysis, SAGE, MassARRAY technique, or FISH.

In some embodiments of the above kits, the median levels of the stromal gene signature is selected from the group consisting of (1) the level of the stromal gene signature from a reference population; (2) the level of the stromal gene signature from a population of complete responders and/or partial responders to the immunotherapy; and (3) the level of the stromal gene signature from the individual at a second time point prior to the first time point. In some embodiments, the change in the level(s) of the stromal gene signature in the biological sample compared to the median levels is an increase in the levels.

BRIEF DESCRIPTION OF THE DRAWINGS

**FIGS. 1A&B** show association of urothelial carcinoma patient responses (**FIG. 1A**) and overall survival (**FIG. 1B**) with expression of urothelial stroma-associated gene Set A.

**FIG. 2** shows association of The Cancer Genome Atlas (TCGA) urothelial carcinoma molecular subtypes (Basal vs Luminal) with expression of urothelial stroma-associated gene Set A.

**FIGS. 3A-G** show association of urothelial carcinoma patient responses with expression of individual urothelial stroma-associated genes TGFB1 (**FIG. 3A**), DKK3 (**FIG. 3B**), PDGFB (**FIG. 3C**), NUAK1 (**FIG. 3D**), FGF1 (**FIG. 3E**), PDLIM4 (**FIG. 3F**), and LRRC32 (**FIG. 3G**).

**FIGS. 4A&B** show association of urothelial carcinoma patient responses (**FIG. 4A**) and overall survival (**FIG. 4B**) with expression of urothelial stroma-associated gene Set B.

**FIG. 5** shows the Spearman correlation of the genes in set A, between each other and all together, for bladder cancer, breast cancer, lung cancer, melanoma, and renal cancer patient samples.

**FIG. 6** shows the mean-Z RNA expression of stroma-associated gene Set A in bladder cancer, breast cancer, lung cancer, melanoma, and renal cancer patient samples.

**FIGS. 7A&B** show association of bladder cancer, breast cancer, lung cancer, melanoma, and renal cancer patient responses (**FIG. 7A**) and overall survival (**FIG. 7B**) with expression of stroma-associated gene Set A.

**FIGS. 8A-E** show association of breast cancer (**FIG. 8A**), renal cancer (**FIG. 8B**), skin cancer (**FIG. 8C**), lung cancer (**FIG. 8D**), and bladder cancer (**FIG. 8E**) patient responses with expression of stroma-associated gene Set A.

**FIGS. 9A-E** show association of overall survival of breast cancer (**FIG. 9A**), bladder cancer (**FIG. 9B**), skin cancer (**FIG. 9C**), lung cancer (**FIG. 9D**), and renal cancer (**FIG. 9E**) patients with expression of stroma-associated gene Set A.

**FIG. 10** shows an experimental diagram of combination therapy with anti-TGFb and anti-PDL1 in a mouse EMT6 breast tumor model.

**FIGS. 11A-C** show tumor volume values in EMT6 breast tumor model mice administered an isotype antibody (**FIG. 11A**), anti-PDL1 antibody (**FIG. 11B**), or anti-PDL1 and anti-TGFb 1D11 antibodies (**FIG. 11C**).

**FIGS. 12A-E** show tumor volume values in EMT6 breast tumor model mice administered an isotype antibody (**FIG. 12A**), anti-PDL1 antibody (**FIG. 12B**), anti-TGFb 2G7 antibody (**FIG. 12C**), anti-TGFb 1D11 antibody (**FIG. 12D**), or anti-PDL1 and anti-TGFb 2G7 antibodies (**FIG. 12E**).

**FIGS. 13A-E** show abundance of CD45+ cells (**FIG. 13A**), CD8 T cells (**FIG. 13B**), granzyme B+ CD8 T cells (**FIG. 13C**), Ki67+ CD8 T cells (**FIG. 13D**), and PD1+ CD8 T cells (**FIG. 13E**) in cells isolated from EMT6 breast tumor model mice administered anti-TGFb 1D11 in combination with anti-PDL1.

**FIGS. 14A&B** show pSMAD MFI (**FIG. 14A**) and percentage of pSMAD+ cells (**FIG. 14B**) in CD45$^-$ cells isolated from EMT6 breast tumor model mice administered anti-PDL1 antibody, anti-TGFb 1D11 antibody, or anti-TGFb 2G7 antibody.

**FIGS. 15A-F** show tumor volume values in EMT6 breast tumor model mice administered anti-PDL1 antibody alone via a dosing regimen of BIWx3 (**FIG. 15A**), TIWx4 (**FIG. 15B**), or BIWx3 (**FIG. 15C**), or anti-PDL1 antibody in combination with anti-TGFb 2G7 via a dosing regimen of BIWx3 (**FIG. 15D**), TIWx4 (**FIG. 15E**), or TIWx3 (**FIG. 15F**)

## DETAILED DESCRIPTION OF THE INVENTION

The invention provides methods for identifying individuals with a disease or disorder who is less likely to respond to immunotherapy alone, the method comprising determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual for treatment with an immunotherapy and with a suppressive stromal antagonist. In some aspects, the invention provides methods for treating an individual with a disease or disorder who is less likely to respond to immunotherapy alone, the method comprising: a) determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual for treatment; and b) administering to said individual an effective amount of an immunotherapy and a suppressive stromal antagonist.

### I. Definitions

The term "detecting" is used herein in the broadest sense to include both qualitative and quantitative measurements of a target molecule. Detecting includes identifying the mere presence of the target molecule in a sample as well as determining whether the target molecule is present in the sample at detectable levels.

The terms "polypeptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.),* as well as other modifications known in the art. The terms "polypeptide" and "protein" as used herein specifically encompass antibodies.

The term "biomarker" as used herein refers to an indicator, *e.g.*, predictive, diagnostic, and/or prognostic, which can be detected in a sample. The biomarker may serve as an indicator of a particular subtype of a disease or disorder (*e.g.,* cancer) characterized by certain, molecular, pathological, histological, and/or clinical features. In some embodiments, a biomarker is a gene. Biomarkers include, but are not limited to, polynucleotides (*e.g.*, DNA, and/or RNA), polynucleotide copy number alterations (*e.g.*, DNA copy numbers), polypeptides, polypeptide and polynucleotide modifications (*e.g.* posttranslational modifications), carbohydrates, and/or glycolipid-based molecular markers.

The terms "biomarker signature," "signature," "biomarker expression signature," or "expression signature" are used interchangeably herein and refer to one or a combination of biomarkers whose expression is an indicator, *e.g.*,

predictive, diagnostic, and/or prognostic. The biomarker signature may serve as an indicator of a particular subtype of a disease or disorder *(e.g.,* cancer) characterized by certain molecular, pathological, histological, and/or clinical features. In some embodiments, the biomarker signature is a "gene signature." The term "gene signature" is used interchangeably with "gene expression signature" and refers to one or a combination of polynucleotides whose expression is an indicator, *e.g.*, predictive, diagnostic, and/or prognostic. In some embodiments, the biomarker signature is a "protein signature." The term "protein signature" is used interchangeably with "protein expression signature" and refers to one or a combination of polypeptides whose expression is an indicator, *e.g.*, predictive, diagnostic, and/or prognostic.

The term "stromal gene signature" refers to any one or a combination or subcombination of genes associated with stroma; *e.g.*, associated with tumor stroma. The gene expression pattern of a stromal gene signature in a patient correlates with the presence of stroma *(e.g.,* fibrosis) in or around a tissue *(e.g.,* a tumor). Each individual gene or member of a stromal gene signature is a "stroma signature gene." Stroma signature genes may be expressed by stromal cells, by tumor cells or by other cells where expression of the stroma signature gene is associated with high levels of stroma *(e.g.,* high levels of fibrosis) in a given tissue. These genes include, without limitation: FAP, FN1, MMP2, BGN, LOXL2, PDPN, PDGFRB, COL4A1, COL4A2, COL5A1, COL8A1, THY1, DKK3, PDGFB, NUAK1, FGF1, PDLIM4, LRRC32, POSTN, LOX, TIMP3, and TGFβ.

A sample, cell, tumor, or cancer which "expresses" one or stromal gene signatures at an increased expression level relative to a median level of expression *(e.g.,* the median level of expression of the one or more stromal gene signatures in the type of cancer (or in a cancer type, wherein the "cancer type" is meant to include cancerous cells *(e.g.,* tumor cells, tumor tissues) as well as non-cancerous cells *(e.g.,* stromal cells, stromal tissues) that surround the cancerous/tumor environment) is one in which the expression level of one or more stromal gene signatures is considered to be a "high stromal gene signature expression level" to a skilled person for that type of cancer. Generally, such a level will be in the range from about 50% up to about 100% or more *(e.g.,* 50%, 55%, 60%, 65%, 70%, 75%, 80%, 85%, 90%, 95%, 100%, or more) relative to stromal gene signature levels in a population of samples, cells, tumors, or cancers of the same cancer type. For instance, the population that is used to arrive at the median expression level may be particular cancer samples *(e.g.,* bladder cancer, breast cancer, colorectal cancer, gastric cancer, liver cancer, melanoma, lung cancer *(e.g.,* non-small cell lung carcinoma), ovarian cancer, or renal cell carcinoma) generally, or subgroupings thereof, such as chemotherapy-resistant cancer, platinum-resistant cancer, as well as advanced, refractory, or recurrent cancer samples. Without being bound by theory, in some embodiments a stromal gene signature includes the expression of stroma-associated genes that impede or inhibit an immunotherapy. In some embodiments, a stromal gene signature is increased expression of tumor stroma-associated genes in an individual compared to the expression of tumor stroma-associated genes from a cancer patient who displays a complete or partial response to an immunotherapy.

By "determining the expression level" used in reference to a particular biomarker *(e.g.,* one or more stroma gene signatures), means expression of the biomarker(s) *(e.g.,* one or more stroma gene signatures) in a cancer-associated biological environment *(e.g.,* expression of the biomarker(s) in the tumor cells), tumor-associated cells *(e.g.,* tumor-associated stromal cells), as determined using a diagnostic test, any of the detection methods described herein, or the similar.

The "amount or "level" of a biomarker associated with an increased clinical benefit to an individual is a detectable level in a biological sample. These can be measured by methods known to one skilled in the art and also disclosed herein. The expression level or amount of biomarker assessed can be used to determine the response to the treatment.

The terms "level of expression" or "expression level" in general are used interchangeably and generally refer to the amount of a biomarker in a biological sample. "Expression" generally refers to the process by which information *(e.g.,* gene-encoded and/or epigenetic) is converted into the structures present and operating in the cell. Therefore, as used herein, "expression" may refer to transcription into a polynucleotide, translation into a polypeptide, or even polynucleotide and/or polypeptide modifications *(e.g.,* posttranslational modification of a polypeptide). Fragments of the transcribed polynucleotide, the translated polypeptide, or polynucleotide and/or polypeptide modifications *(e.g.,* posttranslational modification of a polypeptide) shall also be regarded as expressed whether they originate from a transcript generated by alternative splicing or a degraded transcript, or from a post-translational processing of the polypeptide, *e.g.,* by proteolysis. "Expressed genes" include those that are transcribed into a polynucleotide as mRNA and then translated into a polypeptide, and also those that are transcribed into RNA but not translated into a polypeptide (for example, transfer and ribosomal RNAs).

"Elevated expression," "elevated expression levels," or "elevated levels" refers to an increased expression or increased levels of a biomarker in an individual relative to a control, such as an individual or individuals who are not suffering from the disease or disorder *(e.g.,* cancer), individuals who are complete or partial responders to an immunotherapy, or an internal control *(e.g.,* housekeeping biomarker).

"Reduced expression," "reduced expression levels," or "reduced levels" refers to a decrease expression or decreased levels of a biomarker in an individual relative to a control, such as an individual or individuals who are not suffering from the disease or disorder *(e.g.,* cancer) , individuals who are complete or partial responders to an immunotherapy, or an internal control *(e.g.,* housekeeping biomarker). In some embodiments, reduced expression is little or no expression.

The term "housekeeping biomarker" refers to a biomarker or group of biomarkers (*e.g.*, polynucleotides and/or polypeptides) which are typically similarly present in all cell types. In some embodiments, the housekeeping biomarker is a "housekeeping gene." A "housekeeping gene" refers herein to a gene or group of genes which encode proteins whose activities are essential for the maintenance of cell function and which are typically similarly present in all cell types.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The term "immunotherapy" refers the use of a therapeutic agent that modulates an immune response. An immunotherapy may be an activating immunotherapy or a suppressing immunotherapy. The term "activating immunotherapy" refers to the use of a therapeutic agent that induces, enhances, or promotes an immune response, including, *e.g.*, a T cell response. The term "suppressing immunotherapy" refers to the use of a therapeutic agent that interferes with, suppresses, or inhibits an immune response, including, *e.g.*, a T cell response. In some embodiments, the present invention provides a means for determining a stromal cell signature to determine if an individual may benefit from a combination of an activating immunotherapy with a suppressive stromal antagonist.

The term "PD-L1 axis binding antagonist" is a molecule that inhibits the interaction of a PD-L1 axis binding partner with either one or more of its binding partner, so as to remove T-cell dysfunction resulting from signaling on the PD-1 signaling axis - with a result being to restore or enhance T-cell function. As used herein, a PD-L1 axis binding antagonist includes a PD-L1 binding antagonist and a PD-1 binding antagonist as well as molecules that interfere with the interaction between PD-L1 and PD-1 (*e.g.*, PD-L2-Fc).

The term "PD-L1 binding antagonists" is a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In one embodiment, a PD-L1 binding antagonist reduces the negative signal mediated by or through cell surface proteins expressed on T lymphocytes, and other cells, mediated signaling through PD-L1 or PD-1so as render dysfunctional T-cell less non-dysfunctional.

The term "PD-1 binding antagonists" is a molecule that decreases, blocks, inhibits, abrogates or interferes with signal transduction resulting from the interaction of PD-1 with one or more of its binding partners, such as PD-L1, PD-L2. In some embodiments, the PD-1 binding antagonist is a molecule that inhibits the binding of PD-1 to its binding partners. In a specific aspect, the PD-1 binding antagonist inhibits the binding of PD-1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with PD-L1 and/or PD-L2. In one embodiment, a PD-1 binding antagonist reduces the negative signal mediated by or through cell surface proteins expressed on T lymphocytes, and other cells, mediated signaling through PD-1 or PD-L1 so as render a dysfunctional T-cell less non-dysfunctional.

The terms "Programmed Death Ligand 1" and "PD-L1" refer herein to a native sequence PDL1 polypeptide, polypeptide variants and fragments of a native sequence polypeptide and polypeptide variants (which are further defined herein). The PD-L1 polypeptide described herein may be that which is isolated from a variety of sources, such as from human tissue types or from another source, or prepared by recombinant or synthetic methods.

A "native sequence PD-L1 polypeptide" comprises a polypeptide having the same amino acid sequence as the corresponding PD-L1 polypeptide derived from nature. "PD-L1 polypeptide variant", or variations thereof, means a PD-L1 polypeptide, generally an active PD-L1 polypeptide, as defined herein having at least about 80% amino acid sequence identity with any of the native sequence PD-L1 polypeptide sequences as disclosed herein. Such PD-L1 polypeptide variants include, for instance, PD-L1 polypeptides wherein one or more amino acid residues are added, or deleted, at the N- or C-terminus of a native amino acid sequence. Ordinarily, a PD-L1 polypeptide variant will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a native sequence PD-L1 polypeptide sequence as disclosed herein. Ordinarily, PD-L1 variant polypeptides are at least about 10 amino acids in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 281, 282, 283, 284, 285, 286, 287, 288, 289amino acids in length, or more. Optionally, PD-L1 variant polypeptides will have no more than one conservative amino acid substitution as compared to a native PD-L1 polypeptide sequence, alternatively no more than 2, 3, 4, 5, 6, 7, 8, 9, or 10 conservative amino acid substitution as compared to the native PD-L1 polypeptide sequence.

The term "PD-L1 antagonist" as defined herein is any molecule that partially or fully blocks, inhibits, or neutralizes a biological activity and/or function mediated by a native sequence PD-L1. In certain embodiments such antagonist binds to PD-L1. According to one embodiment, the antagonist is a polypeptide. According to another embodiment, the antagonist is an anti- PD-L1 antibody. According to another embodiment, the antagonist is a small molecule antagonist. According to another embodiment, the antagonist is a polynucleotide antagonist.

A "suppressive stromal antagonist" as defined herein is any molecule that partially or fully blocks, inhibits, or neutralizes a biological activity and/or function of a gene or gene product associated with stroma (e.g, tumor associated stroma). In some embodiments, the suppressive stromal antagonist partially or fully blocks, inhibits, or neutralizes a biological activity and/or function of a gene or gene product associated with fibrotic tumors. In some embodiments, treatment with a suppressive stromal antagonist results in the reduction of stroma thereby resulting in an increase activity of an immunotherapy; for example, by increasing the ability of activating immune cells (*e.g.,* proinflammatory cells) to infiltrate a fibrotic tissue (*e.g.,* a fibrotic tumor).

The term "TGFβ antagonists" is a molecule that decreases, blocks, inhibits, abrogates or interferes with signal transduction resulting from the interaction of TGFβ with one or more of its interaction partners, such as a TGFβ cellular receptor. In some embodiments, a TGFβ binding antagonist is a molecule that inhibits the binding of TGFβ to its binding partners. In some embodiments, the TGFβ antagonist inhibits the activation of TGFβ. In some embodiments, the TGFβ antagonists include anti-TGFβ antibodies, antigen binding fragments thereof, immunoadhesins, fusion proteins, oligopeptides and other molecules that decrease, block, inhibit, abrogate or interfere with signal transduction resulting from the interaction of TGFβ with one or more of its interaction partners.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (*e.g.,* methylphosphonates, phosphotriesters, phosphoamidates, carbamates, *etc.*) and with charged linkages (*e.g.,* phosphorothioates, phosphorodithioates, *etc.*), those containing pendant moieties, such as, for example, proteins (*e.g.*, nucleases, toxins, antibodies, signal peptides, ply-L-lysine, *etc.),* those with intercalators (*e.g.,* acridine, psoralen, *etc.*), those containing chelators (*e.g.,* metals, radioactive metals, boron, oxidative metals, *etc.*), those containing alkylators, those with modified linkages (*e.g.,* alpha anomeric nucleic acids, *etc.*), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro-or 2'-azido-ribose, carbocyclic sugar analogs, $\alpha$-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithio-ate"), "(O)NR$_2$ ("amidate"), P(O)R, P(O)OR', CO or CH$_2$ ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, single stranded, polynucleotides that are, but not necessarily, less than about 250 nucleotides in length. Oligonucleotides may be synthetic. The terms "oligonucleotide" and "polynucleotide" are not mutually exclusive. The description above for polynucleotides is equally and fully applicable to oligonucleotides.

The term "primer" refers to a single stranded polynucleotide that is capable of hybridizing to a nucleic acid and following polymerization of a complementary nucleic acid, generally by providing a free 3'-OH group.

The term "small molecule" refers to any molecule with a molecular weight of about 2000 daltons or less, preferably of about 500 daltons or less.

The term "diagnosis" is used herein to refer to the identification or classification of a molecular or pathological state, disease or condition (*e.g.*, cancer). For example, "diagnosis" may refer to identification of a particular type of cancer. "Diagnosis" may also refer to the classification of a particular subtype of cancer, *e.g.,* by histopathological criteria, or by molecular features (*e.g.,* a subtype characterized by expression of one or a combination of biomarkers (*e.g.,* particular genes or proteins encoded by said genes)).

The term "aiding diagnosis" is used herein to refer to methods that assist in making a clinical determination regarding the presence, or nature, of a particular type of symptom or condition of a disease or disorder (*e.g.,* cancer). For example, a method of aiding diagnosis of a disease or condition (*e.g.,* cancer) can comprise measuring certain biomarkers in a biological sample from an individual.

The term "sample," as used herein, refers to a composition that is obtained or derived from a subject and/or individual of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof.

By "tissue sample" or "cell sample" is meant a collection of similar cells obtained from a tissue of a subject or individual. The source of the tissue or cell sample may be solid tissue as from a fresh, frozen and/or preserved organ, tissue sample, biopsy, and/or aspirate; blood or any blood constituents such as plasma; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. The tissue sample may contain compounds which are not naturally intermixed with the tissue in nature such as preservatives, anticoagulants, buffers, fixatives, nutrients, antibiotics, or the like.

A "reference sample", "reference cell", "reference tissue", "control sample", "control cell", or "control tissue", as used herein, refers to a sample, cell, tissue, standard, or level that is used for comparison purposes. In one embodiment, a reference sample, reference cell, reference tissue, control sample, control cell, or control tissue is obtained from a healthy and/or non-diseased part of the body (*e.g.,* tissue or cells) of the same subject or individual. For example, healthy and/or non-diseased cells or tissue adjacent to the diseased cells or tissue (*e.g.,* cells or tissue adjacent to a tumor). In another embodiment, a reference sample is obtained from an untreated tissue and/or cell of the body of the same subject or individual. In yet another embodiment, a reference sample, reference cell, reference tissue, control sample, control cell, or control tissue is obtained from a healthy and/or non-diseased part of the body (*e.g.,* tissues or cells) of an individual who is not the subject or individual. In even another embodiment, a reference sample, reference cell, reference tissue, control sample, control cell, or control tissue is obtained from an untreated tissue and/or cell of the body of an individual who is not the subject or individual. In some embodiments, the reference sample is an individual who is a complete responder or partial responder to an immunotherapy. In some embodiments, the reference sample is a database of samples from individuals who are complete responders or partial responders to an immunotherapy.

For the purposes herein a "section" of a tissue sample is meant a single part or piece of a tissue sample, *e.g.* a thin slice of tissue or cells cut from a tissue sample. It is understood that multiple sections of tissue samples may be taken and subjected to analysis, provided that it is understood that the same section of tissue sample may be analyzed at both morphological and molecular levels, or analyzed with respect to both polypeptides and polynucleotides.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of polypeptide analysis or protocol, one may use the results of the polypeptide expression analysis or protocol to determine whether a specific therapeutic regimen should be performed. With respect to the embodiment of polynucleotide analysis or protocol, one may use the results of the polynucleotide expression analysis or protocol to determine whether a specific therapeutic regimen should be performed.

"Individual response" or "response" can be assessed using any endPoint indicating a benefit to the individual, including, without limitation, (1) inhibition, to some extent, of disease progression (*e.g.,* cancer progression), including slowing down and complete arrest; (2) a reduction in tumor size; (3) inhibition (*i.e.,* reduction, slowing down or complete stopping) of cancer cell infiltration into adjacent peripheral organs and/or tissues; (4) inhibition (*i.e.* reduction, slowing down or complete stopping) of metatasis; (5) relief, to some extent, of one or more symptoms associated with the disease or disorder (*e.g.,* cancer); (6) increase or extend in the length of survival, including overall survival and progression free survival; and/or (9) decreased mortality at a given Point of time following treatment.

An "effective response" of a patient or a patient's "responsiveness" to treatment with a medicament and similar wording refers to the clinical or therapeutic benefit imparted to a patient at risk for, or suffering from, a disease or disorder, such as cancer. In one embodiment, such benefit includes any one or more of: extending survival (including overall survival and progression free survival); resulting in an objective response (including a complete response or a partial response); or improving signs or symptoms of cancer. In one embodiment, the stromal gene signature is used to identify the patient who is predicted to have an increase likelihood of being responsive to treatment with a combination of an immunotherapy and a suppressive stromal antagonist compared to treatment with the immunotherapy in the absence of treatment with a suppressive stromal antagonist.

"Survival" refers to the patient remaining alive, and includes overall survival as well as progression free survival.

Overall survival refers to the patient remaining alive for a defined period of time, such as 1year, 5 years, *etc* from the time of diagnosis or treatment.

Progression free survival refers to the patient remaining alive, without the cancer progressing or getting worse.

By "extending survival" is meant increasing overall or progression free survival in a treated patient relative to an untreated patient (*i.e.* relative to a patient not treated with the medicament), or relative to a patient who does not express a biomarker at the designated level, and/or relative to a patient treated with an approved anti-tumor agent. An objective response refers to a measurable response, including complete response (CR) or partial response (PR).

By complete response or "CR" is intended the disappearance of all signs of cancer in response to treatment. This does not always mean the cancer has been cured.

Partial response or "PR" refers to a decrease in the size of one or more tumors or lesions, or in the extent of cancer in the body, in response to treatment.

The term "substantially the same," as used herein, denotes a sufficiently high degree of similarity between two numeric values, such that one of skill in the art would consider the difference between the two values to be of little or no biological and/or statistical significance within the context of the biological characteristic measured by said values (*e.g.*, $K_d$ values or expression). The difference between said two values is, for example, less than about 50%, less than about 40%, less than about 30%, less than about 20%, and/or less than about 10% as a function of the reference/comparator value.

The phrase "substantially different," as used herein, denotes a sufficiently high degree of difference between two numeric values such that one of skill in the art would consider the difference between the two values to be of statistical significance within the context of the biological characteristic measured by said values (*e.g.*, $K_d$ values). The difference between said two values is, for example, greater than about 10%, greater than about 20%, greater than about 30%, greater than about 40%, and/or greater than about 50% as a function of the value for the reference/comparator molecule.

An "effective amount" of an agent refers to an amount effective, at dosages and for periods of time necessary, to achieve the desired therapeutic result.

A "therapeutically effective amount" refers to an amount of a therapeutic agent to treat or prevent a disease or disorder in a mammal. In the case of cancers, the therapeutically effective amount of the therapeutic agent may reduce the number of cancer cells; reduce the primary tumor size; inhibit (*i.e.,* slow to some extent and preferably stop) cancer cell infiltration into peripheral organs; inhibit (*i.e.,* slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), the response rates (RR), duration of response, and/or quality of life.

The terms "cancer" and "cancerous" refer to or describe the physiological condition in mammals that is typically characterized by unregulated cell growth. Included in this definition are benign and malignant cancers. By "early stage cancer" or "early stage tumor" is meant a cancer that is not invasive or metastatic or is classified as a Stage 0, I, or II cancer. Examples of cancer include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. More particular examples of such cancers include squamous cell cancer (*e.g.* epithelial squamous cell cancer), lung cancer including small-cell lung cancer (SCLC), non-small cell lung cancer (NSCLC), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, urothelial bladder cancer, hepatoma, breast cancer (including metastatic breast cancer), colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, merkel cell cancer, mycoses fungoids, testicular cancer, esophageal cancer, tumors of the biliary tract, as well as head and neck cancer and hematological malignancies. In some embodiments, the cancer is triple-negative metastatic breast cancer, including any histologically confirmed triple-negative (ER-, PR-, HER2-) adenocarcinoma of the breast with locally recurrent or metastatic disease (where the locally recurrent disease is not amenable to resection with curative intent).

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed during the course of clinical pathology. Desirable effects of treatment include, but are not limited to, preventing occurrence recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies are used to delay development of a disease or to slow the progression of a disease.

The term "anti-cancer therapy" refers to a therapy useful in treating cancer. Examples of anticancer therapeutic agents include, but are limited to, *e.g.*, chemotherapeutic agents, growth inhibitory agents, cytotoxic agents, agents used in radiation therapy, anti-angiogenesis agents, apoptotic agents, antitubulin agents, and other agents to treat cancer, anti-CD20 antibodies, platelet derived growth factor inhibitors (*e.g.*, Gleevec™ (Imatinib Mesylate)), a COX-2 inhibitor (*e.g.,* celecoxib), interferons, cytokines, antagonists (*e.g.,* neutralizing antibodies) that bind to one or more of the following targets PDGFR-beta, BlyS, APRIL, BCMA receptor(s), TRAIL/Apo2, and other bioactive and organic chemical agents, *etc.* Combinations thereof are also included in the invention. The term "cytotoxic agent" as used herein refers to a substance that inhibits or prevents the function of cells and/or causes destruction of cells. The term is intended to include radioactive isotopes (*e.g.*, At211, I131, I125, Y90, Re186, Re188, Sm153, Bi212, P32 and radioactive isotopes of Lu), chemotherapeutic agents *e.g.*, methotrexate, adriamycin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" refers to a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (*e.g.*, calicheamicin, especially calicheamicin gamma1I and calicheamicin omega1 (see, *e.g.*, Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolinodoxorubicin, doxorubicin HCl liposome injection (DOXIL®), liposomal doxorubicin TLC D-99 (MYOCET®), pegylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, e.g., paclitaxel (TAXOL®), albumin-engineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin (e.g., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); BAY439006 (sorafenib; Bayer); SU-11248 (sunitinib, SUTENT®, Pfizer); perifosine, COX-2 inhibitor (e.g., celecoxib or etoricoxib), proteasome inhibitor (e.g., PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors (see definition below); tyrosine kinase inhibitors (see definition below); serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX®), 4-hydroxytamoxifen, toremifene (FARESTON®), idoxifene, droloxifene, raloxifene (EVISTA®), trioxifene, keoxifene, and selective estrogen receptor modulators (SERMs) such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN®), and nonsteroidal aromatase inhibitors such as anastrazole (ARIMIDEX®), letrozole (FEMARA®) and aminoglutethimide, and other aromatase inhibitors include vorozole (RIVISOR®), megestrol acetate (MEGASE®), fadrozole, and 4(5)-imidazoles; lutenizing hormone-releasing hormone agonists, including leuprolide (LUPRON® and ELIGARD®), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); antiandrogens such as flutamide, nilutamide and bicalutamide; and pharmaceutically acceptable salts, acids or derivatives of any of the above; as well as combinations of two or more of the above.

The term "prodrug" as used in this application refers to a precursor or derivative form of a pharmaceutically active substance that is less cytotoxic to tumor cells compared to the parent drug and is capable of being enzymatically activated or converted into the more active parent form. See, e.g., Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamidecontaining prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

A "growth inhibitory agent" when used herein refers to a compound or composition which inhibits growth of a cell (e.g., a cell whose growth is dependent upon PD-L1 expression either in vitro or in vivo). Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one-time administration and typical dosages range from 10 to 200 units (Grays) per day.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (*e.g.,* mice and rats). In certain embodiments, the individual or subject is a human.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

By "reduce or inhibit" is meant the ability to cause an overall decrease of 20%, 30%, 40%, 50%, 60%, 70%, 75%, 80%, 85%, 90%, 95%, or greater. Reduce or inhibit can refer to the symptoms of the disorder being treated, the presence or size of metastases, or the size of the primary tumor.

The term "package insert" is used to refer to instructions customarily included in commercial packages of therapeutic products, that contain information about the indications, usage, dosage, administration, combination therapy, contraindications and/or warnings concerning the use of such therapeutic products.

An "article of manufacture" is any manufacture (*e.g.*, a package or container) or kit comprising at least one reagent, *e.g.,* a medicament for treatment of a disease or disorder (*e.g.,* cancer), or a probe for specifically detecting a biomarker described herein. In certain embodiments, the manufacture or kit is promoted, distributed, or sold as a unit for performing the methods described herein.

A "target audience" is a group of people or an institution to whom or to which a particular medicament is being promoted or intended to be promoted, as by marketing or advertising, especially for particular uses, treatments, or indications, such as individuals, populations, readers of newspapers, medical literature, and magazines, television or internet viewers, radio or internet listeners, physicians, drug companies, *etc. The* phrase "based on" when used herein means that the information about one or more biomarkers is used to inform a treatment decision, information provided on a package insert, or marketing/promotional guidance, *etc.*

As is understood by one skilled in the art, reference to "about" a value or parameter herein includes (and describes) embodiments that are directed to that value or parameter per se. For example, description referring to "about X" includes description of "X".

A "capture antibody" refers to an antibody that specifically binds a target molecule in a sample. Under certain conditions, the capture antibody forms a complex with the target molecule such that the antibody-target molecule complex can be separated from the rest of the sample. In certain embodiments, such separation may include washing away substances or material in the sample that did not bind the capture antibody. In certain embodiments, a capture antibody may be attached to a solid support surface, such as, for example but not limited to, a plate or a bead.

A "detection antibody" refers to an antibody that specifically binds a target molecule in a sample or in a sample-capture antibody combination material. Under certain conditions, the detection antibody forms a complex with the target molecule or with a target molecule-capture antibody complex. A detection antibody is capable of being detected either directly through a label, which may be amplified, or indirectly, *e.g.*, through use of another antibody that is labeled and that binds the detection antibody. For direct labeling, the detection antibody is typically conjugated to a moiety that is detectable by some means, for example, including but not limited to, biotin or ruthenium.

The terms "label" or "detectable label" refers to any chemical group or moiety that can be linked to a substance that is to be detected or quantitated, *e.g.*, an antibody. Typically, a label is a detectable label that is suitable for the sensitive detection or quantification of a substance. Examples of detectable labels include, but are not limited to, luminescent labels, *e.g.,* fluorescent, phosphorescent, chemiluminescent, bioluminescent and electrochemiluminescent labels, radioactive labels, enzymes, particles, magnetic substances, electroactive species and the like. Alternatively, a detectable label may signal its presence by participating in specific binding reactions. Examples of such labels include haptens, antibodies, biotin, streptavidin, His-tag, nitrilotriacetic acid, glutathione S-transferase, glutathione and the like.

The term "detection means" refers to a moiety or technique used to detect the presence of the detectable antibody through signal reporting that is then read out in an assay. Typically, detection means employ reagents that amplify an immobilized label such as the label captured onto a microtiter plate, *e.g.,* avidin or streptavidin-HRP.

"Photoluminescence" refers to a process whereby a material luminesces subsequent to the absorption by that material of light (alternatively termed electromagnetic radiation). Fluorescence and phosphorescence are two different types of photoluminescence. "Chemiluminescent" processes involve the creation of the luminescent species by a chemical reaction. "Electro-chemiluminescence" or "ECL" is a process whereby a species, *e.g.,* an antibody, luminesces upon the exposure of that species to electrochemical energy in an appropriate surrounding chemical environment.

An antibody "which binds" an antigen of interest, *e.g.* a host cell protein, is one that binds the antigen with sufficient affinity such that the antibody is useful as an assay reagent, *e.g.,* as a capture antibody or as a detection antibody. Typically, such an antibody does not significantly cross-react with other polypeptides.

With regard to the binding of a polypeptide to a target molecule, the term "specific binding" or "specifically binds to" or is "specific for" a particular polypeptide or an epitope on a particular polypeptide target means binding that is measurably different from a non-specific interaction. Specific binding can be measured, for example, by determining binding of a target molecule compared to binding of a control molecule, which generally is a molecule of similar structure that does not have binding activity.

"Affinity" refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity which reflects a 1:1 interaction between members of a binding pair (*e.g.,* antibody and antigen). The affinity of a molecule X for its partner Y can generally be represented by the dissociation constant ($K_d$). Affinity can be measured by common methods known in the art, including those described herein.

"Active" or "activity" for the purposes herein refers to form(s) of a polypeptide which retain a biological and/or an immunological activity of native or naturally-occurring polypeptide, wherein "biological" activity refers to a biological function (either inhibitory or stimulatory) caused by a native or naturally-occurring polypeptide other than the ability to induce the production of an antibody against an antigenic epitope possessed by a native or naturally-occurring polypeptide and an "immunological" activity refers to the ability to induce the production of an antibody against an antigenic epitope possessed by a native or naturally-occurring polypeptide.

The term "antagonist" is used in the broadest sense, and includes any molecule that partially or fully blocks, inhibits, or neutralizes a biological activity of a native polypeptide. In a similar manner, the term "agonist" is used in the broadest sense and includes any molecule that mimics a biological activity of a native polypeptide. Suitable agonist or antagonist molecules specifically include agonist or antagonist antibodies or antibody fragments, fragments or amino acid sequence variants of native polypeptides, *etc.* Methods for identifying agonists or antagonists of a polypeptide may comprise contacting a polypeptide with a candidate agonist or antagonist molecule and measuring a detectable change in one or more biological activities normally associated with the polypeptide.

The term "antibody" herein is used in the broadest sense and specifically covers monoclonal antibodies, polyclonal antibodies, half-antibodies, multispecific antibodies (e.g. bispecific antibodies) formed from at least two intact antibodies, and antibody fragments so long as they exhibit the desired biological activity. The term "immunoglobulin" (Ig) is used interchangeable with antibody herein.

Antibodies are naturally occurring immunoglobulin molecules which have varying structures, all based upon the immunoglobulin fold. For example, IgG antibodies have two "heavy" chains and two "light" chains that are disulfide-bonded to form a functional antibody. As another example, one heavy chain and one light chain linked by one or more disulfide bonds forms a functional half-antibody. Each heavy and light chain itself comprises a "constant" (C) and a "variable" (V) region. The V regions determine the antigen binding specificity of the antibody, whilst the C regions provide structural support and function in non-antigen-specific interactions with immune effectors. The antigen binding specificity of an antibody or antigen-binding fragment of an antibody is the ability of an antibody to specifically bind to a particular antigen.

The antigen binding specificity of an antibody is determined by the structural characteristics of the V region. The variability is not evenly distributed across the 110-amino acid span of the variable domains. Instead, the V regions consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, Md. (1991)). The constant domains are not involved directly in binding an antibody to an antigen, but exhibit various effector functions, such as participation of the antibody in antibody dependent cellular cytotoxicity (ADCC).

Each V region typically comprises three complementarity determining regions ("CDRs", each of which contains a "hypervariable loop"), and four framework regions. An antibody binding site, the minimal structural unit required to bind with substantial affinity to a particular desired antigen, will therefore typically include the three CDRs, and at least three, preferably four, framework regions interspersed there between to hold and present the CDRs in the appropriate conformation. Classical four chain antibodies have antigen binding sites which are defined by $V_H$ and $V_L$ domains in cooperation. Certain antibodies, such as camel and shark antibodies, lack light chains and rely on binding sites formed by heavy chains only. Single domain engineered immunoglobulins can be prepared in which the binding sites are formed by heavy chains or light chains alone, in absence of cooperation between $V_H$ and $V_L$.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (*see* Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" when used herein refers to the amino acid residues of an antibody that are responsible for antigen binding. The hypervariable region may comprise amino acid residues from a "complementarity determining region" or "CDR" (*e.g.,* around about residues 24-34 (L1), 50-56 (L2) and 89-97 (L3) in the $V_L$, and around about 31-35B (H1), 50-65 (H2) and 95-102 (H3) in the $V_H$ (Kabat et al., Sequences of Proteins of Immunological Interest, 5th Ed. Public Health Service, National Institutes of Health, Bethesda, Md. (1991)) and/or those residues from a "hypervariable loop" (*e.g.* residues 26-32 (L1), 50-52 (L2) and 91-96 (L3) in the $V_L$, and 26-32 (H1), 52A-55 (H2) and 96-101 (H3) in the $V_H$ (Chothia and Lesk J. Mol. Biol. 196:901-917 (1987)).

"Framework" or "FR" residues are those variable domain residues other than the hypervariable region residues as herein defined.

"Hinge region" in the context of an antibody or half-antibody is generally defined as stretching from Glu216 to Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions.

The "lower hinge region" of an Fc region is normally defined as the stretch of residues immediately C-terminal to the hinge region, *i.e.* residues 233 to 239 of the Fc region. Prior to the present invention, FcγR binding was generally attributed to amino acid residues in the lower hinge region of an IgG Fc region.

The "CH2 domain" of a human IgG Fc region usually extends from about residues 231 to about 340 of the IgG. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. (Burton, Molec. Immunol.22:161-206 (1985)).

The "CH3 domain" comprises the stretch of residues C-terminal to a CH2 domain in an Fc region (*i.e.* from about amino acid residue 341 to about amino acid residue 447 of an IgG).

"Antibody fragments" comprise a portion of an intact antibody, preferably comprising the antigen binding region thereof. Examples of antibody fragments include Fab, Fab', F(ab')$_2$, and Fv fragments; diabodies; tandem diabodies (taDb), linear antibodies(e.g., U.S. Patent No. 5,641,870, Example 2; Zapata et al., Protein Eng. 8(10):1057-1062 (1995)); one-armed antibodies, single variable domain antibodies, minibodies, single-chain antibody molecules; multispecific antibodies formed from antibody fragments (*e.g.*, including but not limited to, Db-Fc, taDb-Fc, taDb-CH3, (scFV)4-Fc, di-scFv, bi-scFv, or tandem (di,tri)-scFv); and Bi-specific T-cell engagers (BiTEs).

Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Pepsin treatment of an antibody yields a single large F(ab')2 fragment which roughly corresponds to two disulfide linked Fab fragments having divalent antigen-binding activity and is still capable of cross-linking antigen. Fab' fragments differ from Fab fragments by having additional few residues at the carboxy terminus of the CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

"Fv" is the minimum antibody fragment that contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the $V_H$-$V_L$ dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab')$_2$ antibody fragments originally were produced as pairs of Fab' fragments that have hinge cysteines between them. Other chemical couplings of antibody fragments are also known.

The "light chains" of antibodies (immunoglobulins) from any vertebrate species can be assigned to one of two clearly distinct types, called kappa ($\kappa$) and lambda ($\lambda$), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), *e.g.,* IgG1, IgG2, IgG3, IgG4, IgA, and IgA2. The heavy chain constant domains that correspond to the different classes of antibodies are called $\alpha$, $\delta$, $\varepsilon$, $\gamma$, and $\mu$, respectively. The subunit structures and three-dimensional configurations of different classes of immunoglobulins are well known.

"Single-chain Fv" or "scFv" antibody fragments comprise the $V_H$ and $V_L$ domains of antibody, wherein these domains are present in a single polypeptide chain. In some embodiments, the Fv polypeptide further comprises a polypeptide linker between the $V_H$ and $V_L$ domains that enables the scFv to form the desired structure for antigen binding. For a review of scFv *see* Plückthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy chain variable domain ($V_H$) connected to a light chain variable domain ($V_L$) in the same polypeptide chain ($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

A "bispecific antibody" is a multispecific antibody comprising an antigen-binding domain that is capable of specifically binding to two different epitopes on one biological molecule or is capable of specifically binding to epitopes on two different biological molecules. A bispecific antibody may also be referred to herein as having "dual specificity" or as being "dual specific." In some embodiments, a bispecific antibody comprises two half antibodies, wherein each half antibody comprises a single heavy chain variable region and optionally at least a portion of a heavy chain constant region, and a single light chain variable region and optionally at least a portion of a light chain constant region. In certain embodiments, a bispecific antibody comprises two half antibodies, wherein each half antibody comprises a single heavy chain variable region and a single light chain variable region and does not comprise more than one single heavy chain variable region and does not comprise more than one single light chain variable region. In some embodiments, a bispecific antibody comprises two half antibodies, wherein each half antibody comprises a single heavy chain variable region and a single light chain variable region, and wherein the first half antibody binds to a first antigen and not to a second antigen and the second half antibody binds to the second antigen and not to the first antigen.

The expression "single domain antibodies" (sdAbs) or "single variable domain (SVD) antibodies" generally refers to antibodies in which a single variable domain (VH or VL) can confer antigen binding. In other words, the single variable domain does not need to interact with another variable domain in order to recognize the target antigen. Examples of single domain antibodies include those derived from camelids (lamas and camels) and cartilaginous fish (*e.g.,* nurse sharks) and those derived from recombinant methods from humans and mouse antibodies (Nature (1989) 341:544-546; Dev Comp Immunol (2006) 30:43-56; Trend Biochem Sci (2001) 26:230-235; Trends Biotechnol (2003):21:484-490; WO 2005/035572; WO 03/035694; FEBS Lett (1994) 339:285-290; WO00/29004; WO 02/051870).

The term "monoclonal antibody" as used herein refers to an antibody obtained from a population of substantially homogeneous antibodies, *i.e.,* the individual antibodies comprising the population are identical and/or bind the same epitope, except for possible variants that may arise during production of the monoclonal antibody, such variants generally being present in minor amounts. In contrast to polyclonal antibody preparations that typically include different antibodies directed against different determinants (epitopes), each monoclonal antibody is directed against a single determinant on the antigen. In addition to their specificity, the monoclonal antibodies are advantageous in that they are uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the methods provided herein may be made by the hybridoma method first described by Kohler et al., Nature 256:495 (1975), or may be made by recombinant DNA methods (*see*, *e.g.,* U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA 81:6851-6855 (1984)). Chimeric antibodies of interest herein include "primatized" antibodies comprising variable domain antigen-binding sequences derived from a non-human primate (*e.g.* Old World Monkey, such as baboon, rhesus or cynomolgus monkey) and human constant region sequences (US Pat No. 5,693,780).

"Humanized" forms of non-human (*e.g.,* murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence, except for FR substitution(s) as noted above. The humanized antibody optionally also will comprise at least a portion of an immunoglobulin constant region, typically that of a human immunoglobulin. For further details, *see* Jones et al., Nature 321:522-525 (1986); Riechmann et al., Nature 332:323-329 (1988); and Presta, Curr. Op. Struct. Biol. 2:593-596 (1992).

For the purposes herein, an "intact antibody" is one comprising heavy and light variable domains as well as an Fc region. The constant domains may be native sequence constant domains (e.g. human native sequence constant domains) or amino acid sequence variant thereof. Preferably, the intact antibody has one or more effector functions.

"Native antibodies" are usually heterotetrameric glycoproteins of about 150,000 Daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

A "naked antibody" is an antibody (as herein defined) that is not conjugated to a heterologous molecule, such as a cytotoxic moiety or radiolabel.

As used herein, the term "immunoadhesin" designates molecules which combine the binding specificity of a heterologous protein (an "adhesin") with the effector functions of immunoglobulin constant domains. Structurally, the immunoadhesins comprise a fusion of an amino acid sequence with a desired binding specificity, which amino acid sequence is other than the antigen recognition and binding site of an antibody (*i.e.,* is "heterologous" compared to a constant region of an antibody), and an immunoglobulin constant domain sequence (*e.g.,* CH2 and/or CH3 sequence of an IgG). Exemplary adhesin sequences include contiguous amino acid sequences that comprise a portion of a receptor or a ligand that binds to a protein of interest. Adhesin sequences can also be sequences that bind a protein of interest, but are not receptor or ligand sequences (*e.g.*, adhesin sequences in peptibodies). Such polypeptide sequences can be selected or identified by various methods, include phage display techniques and high throughput sorting methods. The immunoglobulin constant domain sequence in the immunoadhesin can be obtained from any immunoglobulin, such as IgG-1, IgG-2, IgG-3, or IgG-4 subtypes, IgA (including IgA-1 and IgA-2), IgE, IgD, or IgM.

In some aspects of the disclosure, antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors.

"Complement dependent cytotoxicity" or "CDC" refers to the ability of a molecule to lyse a target in the presence of complement. The complement activation pathway is initiated by the binding of the first component of the complement system (C1q) to a molecule (*e.g.* polypeptide (*e.g.*, an antibody)) complexed with a cognate antigen. To assess complement activation, a CDC assay, *e.g.* as described in Gazzano-Santoro et al., J. Immunol. Methods 202:163 (1996) may be performed.

"Antibody-dependent cell-mediated cytotoxicity" and "ADCC" refer to a cell-mediated reaction in which non-specific cytotoxic cells that express Fc receptors (FcRs) *(e.g.* Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells in summarized is Table 3 on page 464 of Ravetch and Kinet, Annu. Rev. Immunol 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al., Proc. Natl. Acad. Sci. (USA) 95:652-656 (1998).

"Human effector cells" are leukocytes that express one or more FcRs and perform effector functions. In some embodiments, the cells express at least FcγRIII and carry out ADCC effector function. Examples of human leukocytes that mediate ADCC include peripheral blood mononuclear cells (PBMC), natural killer (NK) cells, monocytes, cytotoxic T cells and neutrophils; with PBMCs and NK cells being preferred.

The terms "Fc receptor" or "FcR" are used to describe a receptor that binds to the Fc region of an antibody. In some embodiments, the FcR is a native sequence human FcR. Moreover, a preferred FcR is one that binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (*see* Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976) and Kim et al., J. Immunol. 24:249 (1994)).

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. In certain embodiments, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, *e.g.,* Kindt et al., Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, *e.g.,* Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise. It is understood that aspects and variations of the invention described herein include "consisting" and/or "consisting essentially of" aspects and variations.

## II. Methods of Diagnosis and Treatment

Provided herein are methods for determining if an individual may benefit from treatment with a combination of an immunotherapy and a suppressive stromal antagonist. In some embodiments, the individual is less likely to respond to the immunotherapy alone. In some aspects, the invention provides a method for treating an individual with a disease or disorder, the method comprising: a) determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual for treatment; and b) administering to said individual an effective amount of an immunotherapy and a suppressive stromal antagonist.

In some aspects, the invention provides a method for improving an immunotherapy of an individual with a disease or disorder, the method comprising: a) determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual for treatment with a suppressive stromal antagonist; and b) administering to said individual identified for treatment with a suppressive stromal antagonist in step a) an effective amount of an immunotherapy and a suppressive stromal antagonist.

In some aspects, the invention provides a method for selecting an individual with a disease or disorder who is less likely to respond to immunotherapy alone, the method comprising determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual for treatment with an immunotherapy and with a suppressive stromal antagonist.

In some aspects, the invention provides a method for identifying an individual with a disease or disorder who is more likely to exhibit benefit from treatment with an immunotherapy and with a tumor stromal fibrotic antagonist, the method comprising determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual having a suppressive stroma wherein the presence of a stromal gene signature in a sample from the individual indicates the individual is more likely to exhibit an increased clinical benefit from an immunotherapy and a suppressive stromal antagonist. In some aspects, the invention provides a method for selecting a treatment an individual with a disease or disorder, the method comprising determining the presence of a stromal gene signature in a sample from the individual, said signature comprising one or more of *FAP, FN1, MMP2, PDGFRB,* or *THY,* wherein an increase in the level of expression of the one or more genes in the stromal gene signature relative to a median level identifies an individual having a suppressive stroma; wherein the presence of a stromal gene signature in a sample from the individual indicates the individual is more likely to exhibit an increased clinical benefit from an immunotherapy and a suppressive stromal antagonist. In some embodiments, the increased clinical benefit further comprises a relative increase in one or more of the following: overall survival (OS), progression free survival (PFS), complete response (CR), partial response (PR) and combinations thereof.

In some aspects, the invention provides a method for monitoring the efficacy of a combination treatment comprising an immunotherapy and a suppressive stromal antagonist, the method comprising determining the presence of a stromal gene signature in a sample from an individual undergoing treatment with an immunotherapy and a suppressive stromal antagonist at one or more time points; wherein the stromal gene signature comprises an increase in the level of expression of one or more genes of *FAP, FN1, MMP2, PDGFRB,* or *THY* relative to a median level; wherein an increased clinical benefit and/or a decrease in the presence of the stromal gene signature indicates an effective treatment. In some aspects, the invention provides a method for monitoring the efficacy of a combination treatment comprising an immunotherapy and a suppressive stromal antagonist, the method comprising a) determining the presence of a stromal gene signature in a sample from the individual, said signature comprising one or more of *FAP, FN1, MMP2, PDGFRB,* or *THY,* wherein an increase in the level of expression of the one or more genes in the stroma gene signature relative to a median level identifies an individual for treatment; and b) administering to said individual an effective amount of an immunotherapy and a suppressive stromal antagonist; and c) determining the presence of a stromal gene signature in a sample from the individual at one or more time points; wherein an increased clinical benefit and/or a decrease in the presence of the stromal gene signature indicates an effective treatment. In some embodiments, the increased clinical benefit comprises a relative increase in one or more of the following: overall survival (OS), progression free survival (PFS), complete response (CR), partial response (PR) and combinations thereof.

In some aspects of the invention, the stromal gene signature comprises two or more of *FAP, FN1, MMP2, PDGFRB,* or *THY.* In some embodiments, the stromal gene signature comprises three or more of *FAP, FN1, MMP2, PDGFRB,* or *THY.* In some embodiments, the stromal gene signature comprises four or more of *FAP, FN1, MMP2, PDGFRB,* or *THY.* In some embodiments, the stromal gene signature comprises five or more of *FAP, FN1, MMP2, PDGFRB,* or *THY.* In some embodiments, the stromal gene signature comprises *FAP, FN1, MMP2, PDGFRB, THY1* and *TGFβ.* In some embodiments, the stromal gene signature comprises one or more of *PDPN, FAP, TGFB1, NNMT, TNFAIP6, DKK3, MMP2, MMP8, MMP9, BGN, COL4A1, COL4A2, COL5A1, PDGFRB, NUAK1, FN1, FGF1, PDLIM4* or *LRR32C.* In some embodiments, the stromal gene signature comprises one or more of *FAP, FN1, MMP2, BGN, LOXL2, PDPN, PDGFRB, COL12a1, COL5A1, COL8A2, THY1,* or *PALLD.* In some embodiments, the stromal gene signature further comprises *TGFβ.*

In some aspects, the invention provides methods for determining if an individual with a disease or disorder may benefit from treatment with a combination of an immunotherapy and a suppressive stromal antagonist and methods of treatment. In some embodiments, the invention further provides methods of treatment for individuals identified by the methods of the invention. In the present invention, the disease or disorder is cancer. In some embodiments, the cancer is selected from the group consisting of non-small cell lung cancer, small cell lung cancer, renal cell cancer, colorectal cancer, ovarian cancer, breast cancer, metastatic breast cancer, triple-negative breast cancer, melanoma, pancreatic cancer, gastric carcinoma, bladder cancer, urothelial bladder cancer, esophageal cancer, mesothelioma, melanoma, head and neck cancer, thyroid cancer, sarcoma, prostate cancer, glioblastoma, cervical cancer, thymic carcinoma, leukemia, lymphomas, myelomas, mycoses fungoids, merkel cell cancer, and other hematologic malignancies.

In some embodiments, the cancer is urothelial bladder cancer (UBC) and the stromal gene signature comprises one or more of *FAP, FN1, MMP2, PDGFRB,* or *THY1.* In some embodiments, the cancer is UBC and the stromal gene signature comprises *FAP, FN1, MMP2,* and *PDGFRB.* In some embodiments, the stromal gene signature for UBC further comprises one or more of *DKK3, PDGFB, NUAK1, FGF1, PDL1M4* or *LRRC32.* In some embodiments, the stromal gene signature for UBC comprises one or more of *FAP, FN1, MMP2, PDGFRB, DKK3, PDGFB, NUAK1, FGF1, PDL1M4* or *LRRC32.* In some embodiments, the stromal gene signature for UBC comprises one or more, two or more, three or more, four or more, five or more, six or more, seven or more, eight or more, nine or more or ten or more of *FAP, FN1, MMP2, PDGFRB, DKK3, PDGFB, NUAK1, FGF1, PDL1M4* or *LRRC32.* In some embodiments, the stromal gene signature for UBC comprises *FAP, FN1, MMP2, PDGFRB, DKK3, PDGFB, NUAK1, FGF1, PDL1M4* and *LRRC32.* In some embodiments, the stromal gene signature further comprises *TGFβ.*

In some embodiments, the cancer is non-small cell lung cancer (NSCLC) and the stromal gene signature comprises one or more of *FAP, FN1, MMP2, PDGFRB,* or *THY.* In some embodiments, the cancer is NSCLC and the stromal gene signature comprises one or more, two or more, three or more, four or more, or five or more of *FAP, FN1, MMP2, PDGFRB,* or *THY.* In some embodiments, the cancer is NSCLC and the stromal gene signature comprises one or more of *FAP, FN1, MMP2, PDGFRB,* and *THY1.* In some embodiments, the stromal gene signature further comprises *TGFβ.*

In some embodiments, the cancer is renal cell cancer (RCC) and the stromal gene signature comprises one or more of *FAP, FN1, MMP2, PDGFRB,* or *THY.* In some embodiments, the cancer is RCC and the stromal gene signature comprises *FAP, FN1, MMP2, PDGFRB,* and *THY1.* In some embodiments, the stromal gene signature for RCC further comprises *LUM* and/or *POSTN.* In some embodiments, the cancer is RCC and the stromal gene signature comprises one or more, two or more, three or more, four or more, five or more, six or more, or seven or more of *FAP, FN1, MMP2, PDGFRB, THY1, TGFβ, LUM* or *POSTN.* In some embodiments, the cancer is RCC and the stromal gene signature comprises *FAP, FN1, MMP2, PDGFRB, THY1, LUM,* and *POSTN.* In some embodiments, the stromal gene signature further comprises *TGFβ.*

In some embodiments, the cancer is melanoma and the stromal gene signature comprises one or more of *FAP, FN1, MMP2, PDGFRB,* or *THY1.* In some embodiments, the cancer is melanoma and the stromal gene signature comprises one or more, two or more, three or more, or four or more of *FAP, FN1, MMP2, PDGFRB,* or *THY1.* In some embodiments, the cancer is melanoma and the stromal gene signature comprises *FAP, FN1, MMP2, PDGFRB,* and *THY1.*

In some embodiments the cancer is triple-negative breast cancer (TNBC) and the stromal gene signature comprises one or more of *FAP, FN1, MMP2, PDGFRB,* or *THY1.* In some embodiments the cancer is TNBC and the stromal gene signature comprises *FAP, FN1, MMP2, PDGFRB,* and *THY1.* In some embodiments the stromal gene signature for TNBC further comprises one or more of *MMP11, BGN,* or *COL5A1.* In some embodiments the cancer is TNBC and the stromal gene signature comprises one or more, two or more, three or more, four or more, five or more, six or more, seven or more of *FAP, FN1, MMP2, PDGFRB, THY1, MMP11, BGN,* or *COL5A1.* In some embodiments the cancer is TNBC and the stromal gene signature comprises *FAP, FN1, MMP2, PDGFRB, THY1, MMP11, BGN,* and *COL5A1.*

In some embodiments, the cancer is ovarian cancer and the stromal gene signature comprises one or more of *FAP, FN1, MMP2, PDGFRB,* or *THY1.* In some embodiments, the cancer is ovarian cancer and the stromal gene signature comprises *FAP, FN1, MMP2, PDGFRB,* and *THY1.* In some embodiments, the stromal gene signature for ovarian cancer further comprises one or more of *POSTN, LOX,* or *TIMP3.* In some embodiments, the cancer is ovarian cancer and the stromal gene signature comprises one or more, two or more, three or more, four or more, five or more, six or more, seven or more, eight or more of *FAP, FN1, MMP2, PDGFRB, THY1, POSTN, LOX,* or *TIMP3.* In some embodiments, the cancer is ovarian cancer and the stromal gene signature comprises *FAP, FN1, MMP2, PDGFRB, THY1, POSTN, LOX,* and *TIMP3.* In some embodiments, the stromal gene signature further comprises *TGFβ.*

In some embodiments, the sample obtained from the individual is selected from the group consisting of tissue, whole blood, plasma, serum and combinations thereof. In some embodiments, the sample is a tissue sample. In some embodiments, the sample is a tumor tissue sample. In some embodiments, the tumor tissue sample comprises tumor cells, tumor infiltrating immune cells, stromal cells or any combinations thereof.

In some embodiments, the tissue sample is formalin fixed and paraffin embedded, archival, fresh or frozen. In some embodiments, the sample is whole blood. In some embodiments, the whole blood comprises immune cells, circulating tumor cells and any combinations thereof.

In some embodiments, the sample is obtained prior to treatment with an immunotherapy. In some embodiments, the sample is obtained prior to treatment with the immunotherapy or after treatment with the immunotherapy. In some embodiments, the sample is obtained prior to treatment with the suppressive stromal antagonist.

In some embodiments, the individual with a disease or disorder may benefit from treatment with a combination of an immunotherapy and a suppressive stromal antagonist. In some embodiments, the individual is less likely to respond to the immunotherapy alone. Immunotherapies are described in the art (Chen, DS and Mellman, 2013, Immunity 39:1-10). In some respects, immunotherapy targets may be considered stimulators or inhibitors. In some embodiments, the immunotherapy comprises a CD28, OX40, GITR, CD137, CD27, CD40, ICOS, HVEM, NKG2D, MICA, 2B4, IL-2, IL-12, IFNγ, IFNα, TNFα, IL-1, CDN, HMGB1, or TLR agonist. In other embodiments, the immunotherapy comprises a CTLA-4, PD-L1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist.

In some embodiments of the methods and kits described herein, the immunotherapy is a PD-L1 axis antagonist. In some embodiments, the PD-Ll axis binding antagonist is a PD-Ll binding antagonist. In some embodiments, the PD-Ll binding antagonist inhibits the binding of PD-Ll to its ligand binding partners. In some embodiments, the PD-L1 binding antagonist inhibits the binding of PD-Ll to PD-1. In some embodiments, the PD-L1 binding antagonist inhibits the binding of PD-Ll to B7-1. In some embodiments, the PD-L1 binding antagonist inhibits the binding of PD-Ll to both PD-1 and B7-1.

In some embodiments of any of the methods and kits of the invention, the PD-L1 binding antagonist is an antibody. In some embodiments, the antibody is a monoclonal antibody. In some embodiments, the antibody is a human, humanized or chimeric antibody.

In other embodiments, the PD-Ll axis binding antagonist is a PD-1 binding antagonist. In some embodiments, the PD-1 binding antagonist inhibits the binding of PD-1 to its ligand binding partners. In some embodiments, the PD-1 binding antagonist inhibits the binding of PD-1 to PD-Ll. In some embodiments, the PD-1 binding antagonist inhibits the binding of PD-1 to PD-L2. In some embodiments, the PD-1 binding antagonist inhibits the binding of PD-1 to both PD-Ll and PD-L2.

Examples of anti-PD-L1 antibodies useful for the methods of this invention, and methods for making thereof are described in PCT patent application WO 2010/077634 A1. Diagnostic methods used in treating PD-1 and PD-L1 related conditions are described in PCT patent application WO 2014/151006 A2.

In some embodiments, the anti-PD-Ll antibody is capable of inhibiting binding between PD-L1 and PD-1 and/or between PD-L1 and B7-1. In some embodiments, the anti-PD-Ll antibody is a monoclonal antibody. In some embodiments, the anti-PD-Ll antibody is an antibody fragment selected from the group consisting of Fab, Fab'-SH, Fv, scFv, and (Fab')2 fragments. In some embodiments, the anti-PD-Ll antibody is a humanized antibody. In some embodiments, the anti-PD-Ll antibody is a human antibody.

The invention provides methods to determine the presence of a stromal gene signature in a sample. In some embodiments, the presence of a stromal gene signature indicates a clinical benefit of an immunotherapy in combination with treatment with a suppressive stromal antagonist. The combination of an immunotherapy with a suppressive stromal antagonist may provide clinical benefit compared to treatment with an immunotherapy alone. For example, treatment of a fibrotic tumor presenting a stromal gene signature may benefit from the combination of an immunotherapy and a suppressive stromal antagonist.

As used herein, "suppressive stromal antagonist" is any molecule that partially or fully blocks, inhibits, or neutralizes a biological activity and/or function of a gene or gene product associated with stroma (*e.g,* tumor associated stroma). Targets for stromal gene antagonists are known in the art; for example, see Turley, S.J. et al., Nature Reviews Immunology, 2015. 15:669-682 and Rosenbloom, J. et al., Biochimica et Biophysica Acta 2013, 1832:1088-1103. In some embodiments, the suppressive stromal antagonist targets TGFβ (*e.g.,* TGFB1 and TGFβ2). In some embodiments, the suppressive stromal antagonist targets a surface glycoprotein, including but not limited to endoglin (CD105), 3G5 antigen, FAP, CSPG4 (NG2), and/or podopolin (GP38). In some embodiments, the suppressive stromal antagonist targets an adhesion molecule, including but not limited to PECAM (CD31), VCAM (CD106), ICAM1 (CD54), THY1 (CD90) and/or β1 integrin (CD29). In some embodiments, the suppressibe stromal antagonist targets a growth factor receptor including, but not limited to VEGFR1 (FLT1), VE GFR2 (KDR), VEGFR3 (FLT4), PDGFRα (CD140α), and/or PDGFRβ (CD140β). In some embodiments, the suppressive stromal antagonist targets an intracellular structural protein including but not limited to vimentin, αSMA (ACTA2) and/or desmin. Other targets for suppressive stromal antagonist include but are not limited to 5NT (CD73 or NT5E) RGS3, endosialin (CD248) and FSP1 (S100A4). In some embodiments, the suppressive stromal antagonist targets a cancer associated fibroblast associated polypeptide. Examples of cancer associated fibroblast associated polypeptides include, but are not limited to endoglin (CD105), FAP, Podopalin, VCAM1, THY1, β1 integrin, PDGFRα, PDGFRβ, vimentin, αSMA, desmin, endosialin, and/or FSP1.

In some embodiments, the suppressive stromal antagonist targets TGF-β expression and activation. Examples include but are not limited to Pirfenidone, αvβ6 antibodies, ATI and ATII receptor blockers, ACE inhibitors, CAT-192 (anti-TGF-β1 monoclonal AB), and caveolin scaffolding domain (CSD). In some embodiments, the suppressive stromal antagonist targets TGF-β signaling pathways including canonical signaling pathways TBR1 (exemplary inhibitor is SM305) and SMAD3 (exemplary inhibitor is SIS3). In some embodiments, the suppressive stromal antagonist targets TGF-β signaling pathways including noncanonical signaling pathways including c-Abl (exemplary inhibitor is imatinib mesylate), PDGFR (exemplary inhibitor is dasatinib), c-kit (exemplary inhibitor is Nilotinib), and PKC-δ (exemplary inhibitors include small specific polypeptides and rottlerin derivatives. In some embodiments, the suppressive stromal antagonist targets CTGF (exemplary inhibitors include monoclonal CTGF antibodies). In some embodiments, the suppressive stromal antagonist targets inhibition of fibrocyte homing including CXCL12 (exemplary inhibitor includes CXCL12 antibodies), CXCR4 (exemplary inhibitors include AMD3100), and CCR2 (exemplary inhibitors include PF-04136309. In some embodiments, the suppressive stromal antagonist targets Src (exemplary inhibitors includes dasatinib and SU6656). In some embodiments, the suppressive stromal antagonist targets VEGFR (exemplary inhibitors include Nintedanib and BIBF1120. In some embodiments, the suppressive stromal antagonist targets FGFR (exemplary inhibitor includes Sorafenib). In some embodiments, the suppressive stromal antagonist targets IL-13 (exemplary inhibitor includes humanized monoclonal antibodies to IL-13. In some embodiments, the suppressive stromal antagonist targets IL-6 receptor (exemplary inhibitor includes Tocilizumab). In some embodiments, the suppressive stromal antagonist targets TLR (exemplary inhibitors include TLR inhibitors E5564, TAK-242). In some embodiments, the suppressive stromal antagonist targets Nox4 (ROS) (exemplary inhibitor includes GKT136901). In some embodiments, the suppressive stromal antagonist targets ET-1 (exemplary inhibitors include Bosentan and other ET receptor blockers). In some embodiments, the suppressive stromal antagonist is a TGFβ, PDPN, LAIR-1, SMAD, ALK, connective tissue growth factor (CTGF/CCN2), endothelial-1 (ET-1), AP-1, IL-13, PDGF, LOXL2, endoglin (CD105), FAP, podoplanin (GP38), VCAM1 (CD106), THY1, β1 integrin (CD29), PDGFRα (CD140α), PDGFRβ (CD140β), vimentin, αSMA (ACTA2), desmin, endosialin (CD248) or FSP1 (S 100A4) antagonist.

In some embodiments, the suppressive stromal antagonist is pirfenidone, galunisertib, dasetininb, nintedanib, nilotinib, rottlerin and derivatives, or sorafenib.

In some embodiments, the suppressive stromal antagonist is a TGFβ antagonist. In some embodiments, the TGFβ antagonist inhibits the binding of TGFβ to its ligand binding partners. In some embodiments, the TGFβ antagonist inhibits the binding of TGFβ to a cellular receptor of TGFβ. In some embodiments, the TGFβ antagonist inhibits activation of TGFβ. In some embodiments, the TGFβ antagonist targets TGFβ1. In some embodiments, the TGFβ antagonist targets TGFβ2. In some embodiments, the TGFβ antagonist targets TGFβ3. In some embodiments, the TGFβ antagonist targets TGFβ receptor 1. In some embodiments, the TGFβ antagonist targets one or more of TGFβ1, TGFβ2 or TGFβ1. In some embodiments, the TGFβ antagonist targets TGFβ receptor 2. In some embodiments, the TGFβ antagonist targets TGFβ receptor 3. In some embodiments, the TGFβ antagonist targets one or more of TGFβ receptor 1, TGFβ receptor 2 or TGFβ receptor 3.

In some embodiments, the TGFβ antagonist is an anti-TGFβ antibody. In some embodiments, the anti-TGFβ antibody is capable of inhibiting binding between anti-TGFβ and one or more of its ligands. In some embodiments, the anti-TGFβ antibody is capable of inhibiting activation of TGFβ. In some embodiments, the anti-TGFβ antibody is a monoclonal antibody. In some embodiments, the anti-TGFβ antibody is an antibody fragment selected from the group consisting of Fab, Fab'-SH, Fv, scFv, and (Fab')2 fragments. In some embodiments, the anti-TGFβ antibody is a humanized antibody. In some embodiments, the anti-TGFβ antibody is a human antibody.

Examples of anti-TGFβ antibodies useful for the methods of this invention, and methods for making thereof are described in US Patent No. 5,571,714, WO 92/08480, WO 92/00330, WO 95/26203, WO97/13844, WO 00/066631, WO 05/097832, WO 06/086469, WO 06/116002, WO 07/076391, WO 12/167143, WO 13/134365, and WO 14/164709.

In some embodiments, treatment with the suppressive stromal antagonist allows increased immune cell infiltration in a tissue. Without being bound by theory, the suppressive stromal antagonist modulates the stromal in the target tissue to facilitate infiltration of immune cells to the target tissue. For example, treatment of a fibrotic tumor expressing a stromal gene signature with a suppressive stromal antagonist modulates the stroma in and around the tumor to allow infiltration of immune cells (*e.g.,* modulated by the immunotherapy) to the tumor. In some embodiments, the increased immune cell infiltration is an increased infiltration of one or more of T cells, B cells, macrophages, or dendritic cells. In some embodiments, the T cells are CD8+ T cells and/or $T_{eff}$ cells. In some embodiments, the individual is resistant to immunotherapy prior to treatment with the suppressive stromal antagonist. In some embodiments, the individual has already been administered monotherapy immunotherapy.

Presence and/or expression levels/amount of one or more genes of a stromal gene signature can be determined qualitatively and/or quantitatively based on any suitable criterion known in the art, including but not limited to DNA, mRNA, cDNA, proteins, protein fragments and/or gene copy number. In certain embodiments, presence and/or expression levels/amount of a biomarker in a first sample is increased or elevated as compared to presence/absence and/or expression levels/amount in a second sample. In certain embodiments, presence/absence and/or expression levels/amount of a biomarker in a first sample is decreased or reduced as compared to presence and/or expression levels/amount in a second sample. In certain embodiments, the second sample is a reference sample, reference cell, reference tissue, control sample, control cell, or control tissue. Additional disclosures for determining presence/absence and/or expression levels/amount of a gene are described herein. In some embodiments, the stromal gene signature comprises one or more of *FAP, FN1, MMP2, PDGFRB,* or *THY.*

In some embodiments of any of the methods, elevated expression refers to an overall increase of about any of 10%, 20%, 30%, 40%, 50%, 60%, 70%, 80%, 90%, 95%, 96%, 97%, 98%, 99% or greater, in the level of one or more genes of a stromal gene signature (*e.g.*, protein or nucleic acid *(e.g.,* gene or mRNA)), detected by standard art known methods such as those described herein, as compared to a reference sample, reference cell, reference tissue, control sample, control cell, or control tissue. In certain embodiments, the elevated expression refers to the increase in expression level/amount of a biomarker in the sample wherein the increase is at least about any of 1.5X, 1.75X, 2X, 3X, 4X, 5X, 6X, 7X, 8X, 9X, 10X, 25X, 50X, 75X, or 100X the expression level/amount of the respective biomarker in a reference sample, reference cell, reference tissue, control sample, control cell, or control tissue. In some embodiments, elevated expression refers to an overall increase of greater than about 1.5 fold, about 1.75 fold, about 2 fold, about 2.25 fold, about 2.5 fold, about 2.75 fold, about 3.0 fold, or about 3.25 fold as compared to a reference sample, reference cell, reference tissue, control sample, control cell, control tissue, or internal control (*e.g.*, housekeeping gene). In some embodiments, the expression of the gene signature is relative to a median level of expression. In some embodiments, the median level of expression is the level of expression from a tissue that is not considered fibrotic. In some embodiments, the median level of expression reflects the level of expression in a tissue that responds, or partially responds to immunotherapy. In some embodiments, the median level of expression reflects the level of expression of stromal-associated genes in a tumor from an individual that was a complete responder or partial responder to the immunotherapy. In some embodiments, the median level of expression is derived from a database of patients that responded to the immunotherapy. In some embodiments, the median level of expression is derived from a database of patients with a particular cancer that responded to an immunotherapy. For example, the stromal gene signature for a patient with urothelial bladder cancer is compared to a database of urothelial bladder cancer patients who were complete responders or partial responders to an immunotherapy. In some embodiments, the stromal gene signature comprises one or more of FAP, FN1, MMP2, PDGFRB, or THY.

In some embodiments of any of the methods, reduced expression refers to an overall reduction of about any of 10%, 20%, 30%, 40%, 50%, 60%, 70%, 80%, 90%, 95%, 96%, 97%, 98%, 99% or greater, in the level of the stromal gene signature (e.g., protein or nucleic acid *(e.g.,* gene or mRNA)), detected by standard art known methods such as those described herein, as compared to a reference sample, reference cell, reference tissue, control sample, control cell, or control tissue. In certain embodiments, reduced expression refers to the decrease in expression level/amount of a biomarker in the sample wherein the decrease is at least about any of 0.9X, 0.8X, 0.7X, 0.6X, 0.5X, 0.4X, 0.3X, 0.2X, 0.1X, 0.05X, or 0.01X the expression level/amount of the respective biomarker in a reference sample, reference cell, reference tissue, control sample, control cell, or control tissue. In some embodiments, the stromal gene signature comprises one or more of *FAP, FN1, MMP2, PDGFRB,* or *THY.*

Presence and/or expression level/amount of various genes of a stromal gene signature in a sample can be analyzed by a number of methodologies, many of which are known in the art and understood by the skilled artisan, including, but not limited to, immunohistochemistry ("IHC"), Western blot analysis, immunoprecipitation, molecular binding assays, ELISA, ELIFA, fluorescence activated cell sorting ("FACS"), MassARRAY, proteomics, quantitative blood based assays (as for example Serum ELISA), biochemical enzymatic activity assays, in situ hybridization, Southern analysis, Northern analysis, whole genome sequencing, polymerase chain reaction ("PCR") including quantitative real time PCR ("qRT-PCR") and other amplification type detection methods, such as, for example, branched DNA, SISBA, TMA and the like), RNA-Seq, FISH, microarray analysis, gene expression profiling, and/or serial analysis of gene expression ("SAGE"), as well as any one of the wide variety of assays that can be performed by protein, gene, and/or tissue array analysis. Typical protocols for evaluating the status of genes and gene products are found, for example in Ausubel et al., eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used.

In some embodiments, presence and/or expression level/amount of one or more genes in a stromal gene signature is determined using a method comprising: (a) performing gene expression profiling, PCR (such as rtPCR or qRT-PCR), RNA-seq, microarray analysis, SAGE, MassARRAY technique, or FISH on a sample (such as a subject cancer sample); and b) determining presence and/or expression level/amount of a stromal gene signature product in the sample. In some embodiments, the microarray method comprises the use of a microarray chip having one or more nucleic acid molecules that can hybridize under stringent conditions to a nucleic acid molecule encoding a gene mentioned above or having one or more polypeptides (such as peptides or antibodies) that can bind to one or more of the proteins encoded by the genes mentioned above. In one embodiment, the PCR method is qRT-PCR. In one embodiment, the PCR method is multiplex-PCR. In some embodiments, gene expression is measured by microarray. In some embodiments, gene expression is measured by qRT-PCR. In some embodiments, expression is measured by multiplex-PCR. In some embodiments, the stromal gene signature comprises one or more of *FAP, FN1, MMP2, PDGFRB,* or *THY.*

Methods for the evaluation of mRNAs in cells are well known and include, for example, hybridization assays using complementary DNA probes (such as in situ hybridization using labeled riboprobes specific for the one or more genes, Northern blot and related techniques) and various nucleic acid amplification assays (such as RT-PCR using complementary primers specific for one or more of the genes, and other amplification type detection methods, such as, for example, branched DNA, SISBA, TMA and the like).

Samples from mammals can be conveniently assayed for mRNAs using Northern, dot blot or PCR analysis. In addition, such methods can include one or more steps that allow one to determine the levels of target mRNA in a biological sample {*e.g.,* by simultaneously examining the levels a comparative control mRNA sequence of a "housekeeping" gene such as an actin family member). Optionally, the sequence of the amplified target cDNA can be determined.

Optional methods include protocols which examine or detect mRNAs, such as target mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes whose expression correlates with increased or reduced clinical benefit of anti-angiogenic therapy may be arrayed on a solid support. Hybridization of a labeled probe with a particular array member indicates that the sample from which the probe was derived expresses that gene.

In certain embodiments, the presence and/or expression level/amount of the stromal gene signature proteins in a sample is examined using IHC and staining protocols. IHC staining of tissue sections has been shown to be a reliable method of determining or detecting presence of proteins in a sample. In some embodiments, stromal gene signature is detected by immunohistochemistry. In some embodiments, elevated expression of a stromal gene signature in a sample from an individual is elevated protein expression and, in further embodiments, is determined using IHC. In one embodiment, expression level of the stromal gene signature is determined using a method comprising: (a) performing IHC analysis of a sample (such as a subject cancer sample) with an antibody; and b) determining expression level of the stromal gene signature in the sample. In some embodiments, IHC staining intensity is determined relative to a reference. In some embodiments, the reference is a reference value (*e.g.,* from a database of complete responders and partial responders to an immunotherapy). In some embodiments, the stromal gene signature comprises one or more of FAP, FN1, MMP2, PDGFRB, or THY.

IHC may be performed in combination with additional techniques such as morphological staining and/or fluorescence in-situ hybridization. Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibodies used for IHC typically will be labeled with a detectable moiety. Numerous labels are available which can be generally grouped into the following categories: (a) Radioisotopes, such as $^{35}S$, $^{14}C$, $^{125}I$, $^{3}H$, and $^{131}I$; (b) colloidal gold particles; (c) fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above; (d) various enzyme-substrate labels are available and U.S. Patent No. 4,275,149 provides a review of some of these. Examples of enzymatic labels include luciferases (*e.g.*, firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (*e.g.*, glucose oxidase, galactose oxidase, and glucose-6-phosphate dehydrogenase), heterocyclic oxidases (such as uricase and xanthine oxidase), lactoperoxidase, microperoxidase, and the like.

Examples of enzyme-substrate combinations include, for example, horseradish peroxidase (HRPO) with hydrogen peroxidase as a substrate; alkaline phosphatase (AP) with para-Nitrophenyl phosphate as chromogenic substrate; and β-D-galactosidase (β-D-Gal) with a chromogenic substrate (*e.g.,* p-nitrophenyl-D-galactosidase) or fluorogenic substrate (*e.g., 4*-methylumbelliferyl- -D-galactosidase). For a general review of these, see U.S. Patent Nos. 4,275,149 and 4,318,980.

In some embodiments of any of the methods, one or more genes of the stromal gene signature is detected by immunohistochemistry using a stromal gene diagnostic antibodies (*i.e.,* primary antibody). In some embodiments, the stromal diagnostic antibody specifically binds human stromal-associated genes. In some embodiments, the stromal diagnostic antibody is a nonhuman antibody. In some embodiments, the stromal diagnostic antibody is a rat, mouse, or rabbit antibody. In some embodiments, the stromal diagnostic antibody is a monoclonal antibody. In some embodiments, the stromal diagnostic antibody is directly labeled. In some embodiments, the stromal gene signature comprises one or more of *FAP, FN1, MMP2, PDGFRB,* or *THY.*

Specimens thus prepared may be mounted and coverslipped. Slide evaluation is then determined, *e.g.*, using a microscope, and staining intensity criteria, routinely used in the art, may be employed. In one embodiment, it is understood that when cells and/or tissue from a tumor is examined using IHC, staining is generally determined or assessed in tumor cell and/or tissue (as opposed to stromal or surrounding tissue that may be present in the sample). In some embodiments, it is understood that when cells and/or tissue from a tumor is examined using IHC, staining includes determining or assessing in tumor infiltrating immune cells, including intratumoral or peritumoral immune cells. In some embodiments, the presence of a PD-L1 biomarker is detected by IHC in >0 % of the sample, in at least 1% of the sample, in at least 5% of the sample, in at least 10% of the sample.

In alternative methods, the sample may be contacted with an antibody specific for said biomarker under conditions sufficient for an antibody-biomarker complex to form, and then detecting said complex. The presence of the biomarker may be detected in a number of ways, such as by Western blotting and ELISA procedures for assaying a wide variety of tissues and samples, including plasma or serum. A wide range of immunoassay techniques using such an assay format are available, see, *e.g.,* U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labeled antibody to a target biomarker.

Presence and/or expression level/amount of a selected stromal gene signature in a tissue or cell sample may also be examined by way of functional or activity-based assays. For instance, if the biomarker is an enzyme, one may conduct assays known in the art to determine or detect the presence of the given enzymatic activity in the tissue or cell sample.

In certain embodiments, the samples are normalized for both differences in the amount of the stromal gene assayed and variability in the quality of the samples used, and variability between assay runs. Such normalization may be accomplished by detecting and incorporating the expression of certain normalizing biomarkers, including well known housekeeping genes. Alternatively, normalization can be based on the mean or median signal of all of the assayed genes or a large subset thereof (global normalization approach). On a gene-by-gene basis, measured normalized amount of a subject tumor mRNA or protein is compared to the amount found in a reference set. Normalized expression levels for each mRNA or protein per tested tumor per subject can be expressed as a percentage of the expression level measured in the reference set. The presence and/or expression level/amount measured in a particular subject sample to be analyzed will fall at some percentile within this range, which can be determined by methods well known in the art. In some embodiments, the stromal gene signature comprises one or more of *FAP, FN1, MMP2, PDGFRB,* or *THY.*

In one embodiment, the sample is a clinical sample. In another embodiment, the sample is used in a diagnostic assay. In some embodiments, the sample is obtained from a primary or metastatic tumor. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood. Genes or gene products can be detected from cancer or tumor tissue or from other body samples such as urine, sputum, serum or plasma. The same techniques discussed above for detection of target genes or gene products in cancerous samples can be applied to other body samples. Cancer cells may be sloughed off from cancer lesions and appear in such body samples. By screening such body samples, a simple early diagnosis can be achieved for these cancers. In addition, the progress of therapy can be monitored more easily by testing such body samples for target genes or gene products.

In certain embodiments, a reference sample, reference cell, reference tissue, control sample, control cell, or control tissue is a single sample or combined multiple samples from the same subject or individual that are obtained at one or more different time points than when the test sample is obtained. For example, a reference sample, reference cell, reference tissue, control sample, control cell, or control tissue is obtained at an earlier time point from the same subject or individual than when the test sample is obtained. Such reference sample, reference cell, reference tissue, control sample, control cell, or control tissue may be useful if the reference sample is obtained during initial diagnosis of cancer and the test sample is later obtained when the cancer becomes metastatic.

In certain embodiments, a reference sample, reference cell, reference tissue, control sample, control cell, or control tissue is a combined multiple samples from one or more healthy individuals who are not the subject or individual. In certain embodiments, a reference sample, reference cell, reference tissue, control sample, control cell, or control tissue is a combined multiple samples from one or more individuals with a disease or disorder (*e.g.,* cancer) who are not the subject or individual. In certain embodiments, a reference sample, reference cell, reference tissue, control sample, control cell, or control tissue is pooled RNA samples from normal tissues or pooled plasma or serum samples from one or more individuals who are not the subject or individual. In certain embodiments, a reference sample, reference cell, reference tissue, control sample, control cell, or control tissue is pooled RNA samples from tumor tissues or pooled plasma or serum samples from one or more individuals with a disease or disorder (*e.g.,* cancer) who are not the subject or individual.

In certain embodiments, a reference sample, reference cell, reference tissue, control sample, control cell, or control tissue is a combined multiple samples from one or more individuals who are not the subject or individual but were complete responders or partial responders to an immunotherapy. In certain embodiments, a reference sample, reference cell, reference tissue, control sample, control cell, or control tissue is a combined multiple samples from one or more individuals with a disease or disorder (*e.g.,* cancer) who are not the subject or individual. In certain embodiments, a reference sample, reference cell, reference tissue, control sample, control cell, or control tissue is pooled RNA samples from tissues or pooled plasma or serum samples from one or more individuals who are not the subject or individual bur were complete responders or partial responders to an immunotherapy. In certain embodiments, a reference sample, reference cell, reference tissue, control sample, control cell, or control tissue is pooled RNA samples from tumor tissues or pooled plasma or serum samples from one or more individuals with a disease or disorder (*e.g.,* cancer) who are not the subject or individual who were complete responders or partial responders to an immunotherapy.

In some embodiments, the sample is a tissue sample from the individual. In some embodiments, the tissue sample is a tumor tissue sample (*e.g.*, biopsy tissue). In some embodiments, the tissue sample is lung tissue. In some embodiments, the tissue sample is renal tissue. In some embodiments, the tissue sample is skin tissue. In some embodiments, the tissue sample is pancreatic tissue. In some embodiments, the tissue sample is gastric tissue. In some embodiments, the tissue sample is bladder tissue. In some embodiments, the tissue sample is esophageal tissue. In some embodiments, the tissue sample is mesothelial tissue. In some embodiments, the tissue sample is breast tissue. In some embodiments, the tissue sample is thyroid tissue. In some embodiments, the tissue sample is colorectal tissue. In some embodiments, the tissue sample is head and neck tissue. In some embodiments, the tissue sample is osteosarcoma tissue. In some embodiments, the tissue sample is prostate tissue. In some embodiments, the tissue sample is ovarian tissue, HCC (liver), blood cells, lymph nodes, bone/bone marrow.

In some embodiments of any of the methods, the disease or disorder is a tumor. In some embodiments, the tumor is a malignant cancerous tumor (*i.e.,* cancer). In some embodiments, the tumor and/or cancer is a solid tumor or a non-solid or soft tissue tumor. Examples of soft tissue tumors include leukemia (*e.g.,* chronic myelogenous leukemia, acute myelogenous leukemia, adult acute lymphoblastic leukemia, acute myelogenous leukemia, mature B-cell acute lymphoblastic leukemia, chronic lymphocytic leukemia, polymphocytic leukemia, or hairy cell leukemia) or lymphoma (*e.g.*, non-Hodgkin's lymphoma, cutaneous T-cell lymphoma, or Hodgkin's disease). A solid tumor includes any cancer of body tissues other than blood, bone marrow, or the lymphatic system. Solid tumors can be further divided into those of epithelial cell origin and those of non-epithelial cell origin. Examples of epithelial cell solid tumors include tumors of the gastrointestinal tract, colon, colorectal (*e.g.*, basaloid colorectal carcinoma), breast (*e.g.,* triple negative breast cancer), prostate, lung, kidney, liver, pancreas, ovary (*e.g.*, endometrioid ovarian carcinoma), head and neck, oral cavity, stomach, duodenum, small intestine, large intestine, anus, gall bladder, labium, nasopharynx, skin, uterus, male genital organ, urinary organs (*e.g.* , urothelial bladder cancer, urothelium carcinoma, dysplastic urothelium carcinoma, transitional cell carcinoma), bladder, and skin. Solid tumors of non-epithelial origin include sarcomas, brain tumors, and bone tumors. In some embodiments, the cancer is non-small cell lung cancer (NSCLC). In some embodiments, the cancer is second-line or third-line locally advanced or metastatic non-small cell lung cancer. In some embodiments, the cancer is adenocarcinoma. In some embodiments, the cancer is squamous cell carcinoma.

In some embodiments, one or more of the genes of the stromal gene signature is detected in the sample using a method selected from the group consisting of FACS, Western blot, ELISA, immunoprecipitation, immunohistochemistry, immunofluorescence, radioimmunoassay, dot blotting, immunodetection methods, HPLC, surface plasmon resonance, optical spectroscopy, mass spectrometery, HPLC, qPCR, RT-qPCR, multiplex qPCR or RT-qPCR, RNA-seq, microarray analysis, SAGE, MassARRAY technique, and FISH, and combinations thereof. In some embodiments, the stromal gene signature is detected using FACS analysis. In some embodiments, the stromal gene signature comprises one or more of

*FAP, FN1, MMP2, PDGFRB,* or *THY.*

Preferably, the expression level of a stromal gene signature is assessed in a biological sample that contains or is suspected to contain cancer cells. The sample may be, for example, a tissue resection, a tissue biopsy, or a metastatic lesion obtained from a patient suffering from, suspected to suffer from, or diagnosed with cancer (*e.g.,* bladder cancer (*e.g.,* urothelial bladder cancer), breast cancer (*e.g.,* triple negative breast cancer), renal cell carcinoma, colorectal cancer, gastric cancer, liver cancer, melanoma, lung cancer (*e.g.,* non-small cell lung carcinoma), ovarian cancer, or pancreatic cancer). Preferably, the sample is a sample of a tissue, a resection or biopsy of a tumor, a known or suspected metastatic cancer lesion or section, or a blood sample, *e.g.,* a peripheral blood sample, known or suspected to comprise circulating cancer cells. The sample may comprise both cancer cells (*i.e.,* tumor cells), and non-cancerous cells (*e.g.,* stromal cells), and, in certain embodiments, comprises both cancerous and non-cancerous cells. In aspects of the invention comprising the determination of gene expression in stroma components, the sample comprises both cancer/tumor cells and non-cancerous cells that are, *e.g.,* associated with the cancer/tumor cells (*e.g.,* tumor associated fibroblasts, endothelial cells, pericytes, the extra-cellular matrix, and/or various classes of leukocytes). In other aspects, the skilled artisan, *e.g.,* a pathologist, can readily discern cancer cells from non-cancerous (*e.g.,* stromal cells, endothelial cells, *etc.*). Methods of obtaining biological samples including tissue resections, biopsies, and body fluids, *e.g.,* blood samples comprising cancer/tumor cells, are well known in the art. In some embodiments, the sample obtained from the patient is collected prior to beginning any immunotherapy or other treatment regimen or therapy, *e.g.,* chemotherapy or radiation therapy for the treatment of cancer or the management or amelioration of a symptom thereof. In some embodiments, the sample obtained from the patient is collected after beginning an immunotherapy but before treatment with a suppressive stromal antagonist. In some embodiments, the patient has failed treatment with an immunotherapy prior to treatment with a suppressive stromal antagonist. Therefore, in some embodiments, the sample is collected before the administration of immunotherapeutic agents or other agents, or the start of immunotherapy or treatment with a suppressive stromal antagonist. In some embodiments, the sample is collected after the administration of immunotherapeutic agents or other agents, but before the start of treatment with a suppressive stromal antagonist

In addition to the methods described above, the invention also encompasses further immunohistochemical methods for assessing the expression level of one or more stromal gene signatures, such as by Western blotting and ELISA-based detection. As is understood in the art, the expression level of the marker/indicator proteins of the invention may also be assessed at the mRNA level by any suitable method known in the art, such as Northern blotting, real time PCR, and RT PCR. Immunohistochemical- and mRNA-based detection methods and systems are well known in the art and can be deduced from standard textbooks, such as Lottspeich (Bioanalytik, Spektrum Akademisher Verlag, 1998) or Sambrook and Russell (Molecular Cloning: A Laboratory Manual, CSH Press, Cold Spring Harbor, N.Y., U.S.A., 2001). The described methods are of particular use for determining the expression levels of a stromal gene signature in a patient or group of patients relative to control levels established in a population diagnosed with advanced stages of a cancer (*e.g.,* bladder cancer, breast cancer, colorectal cancer, gastric cancer, liver cancer, melanoma, lung cancer (*e.g.,* non-small cell lung carcinoma), ovarian cancer, or renal cell carcinoma).

For use in the detection methods described herein, the skilled person has the ability to label the polypeptides or oligonucleotides encompassed by the present invention. As routinely practiced in the art, hybridization probes for use in detecting mRNA levels and/or antibodies or antibody fragments for use in IHC methods can be labeled and visualized according to standard methods known in the art. Non-limiting examples of commonly used systems include the use of radiolabels, enzyme labels, fluorescent tags, biotin-avidin complexes, chemiluminescence, and the like.

The expression level of one or more stromal gene signature can also be determined on the protein level by taking advantage of immunoagglutination, immunoprecipitation (*e.g.,* immunodiffusion, immunelectrophoresis, immune fixation), western blotting techniques (*e.g.,* in situ immunohistochemistry, in situ immunocytochemistry, affinity chromatography, enzyme immunoassays), and the like. Amounts of purified polypeptide may also be determined by physical methods, *e.g.,* photometry. Methods of quantifying a particular polypeptide in a mixture usually rely on specific binding, *e.g.,* of antibodies.

As mentioned above, the expression level of the marker/indicator proteins according to the present invention may also be reflected in increased or decreased expression of the corresponding gene(s) encoding the stromal gene signature. Therefore, a quantitative assessment of the gene product prior to translation (*e.g.* spliced, unspliced or partially spliced mRNA) can be performed in order to evaluate the expression of the corresponding gene(s). The person skilled in the art is aware of standard methods to be used in this context or may deduce these methods from standard textbooks (*e.g.* Sambrook, 2001). For example, quantitative data on the respective concentration/amounts of mRNA encoding one or more of an immune cell gene signature as described herein can be obtained by Northern Blot, Real Time PCR, and the like.

The invention further provides methods for administering an immunotherapy to patients with a cancer (*e.g.,* bladder cancer (*e.g.,* a urothelial bladder cancer, breast cancer (*e.g.,* a triple negative breast cancer), colorectal cancer, gastric cancer, liver cancer, melanoma, lung cancer (*e.g.,* non-small cell lung carcinoma), ovarian cancer, or renal cell carcinoma that is chemotherapy-resistant, chemotherapy-sensitive, refractory, primary, advanced, or recurrent), if the patient is determined to have a change in the level of expression of one or more immune cell gene signatures in any of the gene sets. In one embodiment, the patient is administered a suppressive stromal antagonist in combination with an immunotherapy if there is an increase in expression level of one or more stromal gene signatures (*i.e.,* one or more of *FAP, FN1, MMP2, PDGFRB,* or *THY*).

In some embodiments, the activating immunotherapy includes a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist. In particular embodiments, the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) increases, enhances, or stimulates an immune response or function in a patient having cancer. In some embodiments, the agonist modulates the expression and/or activity of a ligand (*e.g.,* a T cell receptor ligand), and/or increases or stimulates the interaction of the ligand with its immune receptor, and/or increases or stimulates the intracellular signaling mediated by ligand binding to the immune receptor. In other embodiments, the suppressing immunotherapy includes a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist. In particular embodiments, the antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) is an agent that inhibits and/or blocks the interaction of a ligand (*e.g.,* a T cell receptor ligand) with its immune receptor or is an antagonist of ligand and/or receptor expression and/or activity, or is an agent that blocks the intracellular signaling mediated by a ligand (*e.g.,* a T cell receptor ligand) with its immune receptor. In some embodiments, the immunotherapy is administered in combination with a suppressive stromal antagonist. In some embodiments, the immunotherapy is administered in combination with a suppressive stromal antagonist where a sample from the individual indicated the presence of a stromal gene signature as described herein.

In some embodiments, the methods of the invention may further comprise administering the activating immunotherapy (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist agonist) and a suppressive stromal antagonist with an additional therapy. The additional therapy may be radiation therapy, surgery, chemotherapy, gene therapy, DNA therapy, viral therapy, RNA therapy, bone marrow transplantation, nanotherapy, monoclonal antibody therapy, or a combination of the foregoing. The additional therapy may be in the form of an adjuvant or neoadjuvant therapy. In some embodiments, the additional therapy is the administration of side-effect limiting agents (*e.g.*, agents intended to lessen the occurrence and/or severity of side effects of treatment, such as anti-nausea agents, *etc.*). In some embodiments, the additional therapy is radiation therapy. In some embodiments, the additional therapy is surgery. In some embodiments, the additional therapy may be one or more of the chemotherapeutic agents described hereinabove. For example, these methods involve the co-administration of the activating immunotherapy (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or the suppressing immunotherapy (*e.g.*, a CTLA-4, PD-1 axis, TIM-3, BTLA, VISTA, LAG-3, B7H4, CD96, TIGIT, CD226, prostaglandin, VEGF, endothelin B, IDO, arginase, MICA/-MICB, TIM-3, IL-10, IL-4, or IL-13 antagonist) with one or more additional chemotherapeutic agents (*e.g.,* carboplatin and/or paclitaxel), as described further below. Immunotherapy optionally in combination with one or more chemotherapeutic agents (*e.g.*, carboplatin and/or paclitaxel) preferably extends and/or improves survival, including progression free survival (PFS) and/or overall survival (OS). In one embodiment, immunotherapy extends survival at least about 20% more than survival achieved by administering an approved anti-tumor agent, or standard of care, for the cancer being treated.

In one embodiment, a fixed dose of the immunotherapy is administered. The fixed dose may suitably be administered to the patient at one time or over a series of treatments. Where a fixed dose is administered, preferably it is in the range from about 20 mg to about 2000 mg. For example, the fixed dose may be approximately 420 mg, approximately 525 mg, approximately 840 mg, or approximately 1050 mg of the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist). Where a series of doses are administered, these may, for example, be administered approximately every week, approximately every 2 weeks, approximately every 3 weeks, or approximately every 4 weeks, but preferably approximately every 3 weeks. The fixed doses may, for example, continue to be administered until disease progression, adverse event, or other time as determined by the physician. For example, from about two, three, or four, up to about 17 or more fixed doses may be administered.

In one embodiment, one or more loading dose(s) of the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) are administered, followed by one or more maintenance dose(s). In another embodiment, a plurality of the same dose is administered to the patient.

In one embodiment, one or more loading dose(s) of the suppressive stromal antagonist (*e.g.,* a TGFβ antagonist) is administered, followed by one or more maintenance dose(s). In another embodiment, a plurality of the same dose is administered to the patient.

While the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) may be administered as a single anti-tumor agent, the patient is optionally treated with a combination of agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) and a suppressive stromal antagonist depending on the presence of a stromal gene signature.

In other embodiments, the immunotherapy and the suppressive stromal antagonist is administered in combination with a chemotherapeutic agent. Exemplary chemotherapeutic agents herein include: gemcitabine, carboplatin, oxaliplatin, irinotecan, fluoropyrimidine (*e.g.*, 5-FU), paclitaxel (*e.g.*, nab-paclitaxel), docetaxel, topotecan, capecitabine, temozolomide, interferon-alpha, and/or liposomal doxorubicin (*e.g.*, pegylated liposomal doxorubicin). The combined administration includes co-administration or concurrent administration, using separate formulations or a single pharmaceutical formulation, and consecutive administration in either order, wherein preferably there is a time period while both (or all) active agents simultaneously exert their biological activities. Thus, the chemotherapeutic agent may be administered prior to, or following, administration of the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) and/or prior to, or following administration of a suppressive stromal antagonist. In this embodiment, the timing between at least one administration of the chemotherapeutic agent and at least one administration of the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist), antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) and/or suppressive stromal antagonist is preferably approximately 1 month or less (3 weeks, 2, weeks, 1 week, 6 days, 5, days, 4 days, 3 days, 2 days, 1 day). Alternatively, the chemotherapeutic agent and the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist), and suppressive stromal antagonist are administered concurrently to the patient, in a single formulation or separate formulations. Treatment with the combination of the chemotherapeutic agent (*e.g.,* carboplatin and/or paclitaxel) and the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) and/or suppressive stromal antagonist may result in a synergistic, or greater than additive, therapeutic benefit to the patient.

Particularly desired chemotherapeutic agents for combining with the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) and suppressive stromal antagonist, *e.g.* for therapy of ovarian cancer, include: a chemotherapeutic agent such as a platinum compound (*e.g.*, carboplatin), a taxol such as paclitaxel or docetaxel, topotecan, or liposomal doxorubicin.

Particularly desired chemotherapeutic agents for combining with the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) and suppressive stromal antagonist, *e.g.*, for therapy of breast cancer, include: chemotherapeutic agents such as capecitabine, and a taxol such as paclitaxel (*e.g.*, nab-paclitaxel) or docetaxel.

Particularly desired chemotherapeutic agents for combining with the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) and suppressive stromal antagonist, *e.g.*, for therapy of colorectal cancer, include: chemotherapeutic agents such as a fluoropyrimidine (*e.g.*, 5-FU), paclitaxel, cisplatin, topotecan, irinotecan, fluoropyrimidine-oxaliplatin, fluoropyrimidine-irinotecan, FOLFOX4 (5-FU, lecovorin, oxaliplatin), and IFL (ironotecan, 5-FU, leucovorin).

Particularly desired chemotherapeutic agents for combining with the agonist (*e.g.,* a CD28, OX40, GITR, CD137, CD27, ICOS, HVEM, NKG2D, MICA, 2B4, IL-2, IL-12, IFNγ, IFNα, TNFα, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) and suppressive stromal antagonist, *e.g.*, for therapy of renal cell carcinoma, include: chemotherapeutic agents such as interferon-alpha2a.

A chemotherapeutic agent, if administered, is usually administered at dosages known therefore, or optionally lowered due to combined action of the drugs or negative side effects attributable to administration of the chemotherapeutic agent. Preparation and dosing schedules for such chemotherapeutic agents may be used according to manufacturers' instructions or as determined empirically by the skilled practitioner. Where the chemotherapeutic agent is paclitaxel, preferably, it is administered at a dose between about 130 mg/m$^2$ to 200 mg/m$^2$ (for example approximately 175 mg/m$^2$), for instance, over 3 hours, once every 3 weeks. Where the chemotherapeutic agent is carboplatin, preferably it is administered by calculating the dose of carboplatin using the Calvert formula which is based on a patient's preexisting renal function or renal function and desired platelet nadir. Renal excretion is the major route of elimination for carboplatin. The use of this dosing formula, as compared to empirical dose calculation based on body surface area, allows compensation for patient variations in pretreatment renal function that might otherwise result in either underdosing (in patients with above average renal function) or overdosing (in patients with impaired renal function). The target AUC of 4-6 mg/mL/min using single agent carboplatin appears to provide the most appropriate dose range in previously treated patients.

Aside from the agonist (*e.g.,* a CD28, OX40, GITR, CD137, CD27, ICOS, HVEM, NKG2D, MICA, 2B4, IL-2, IL-12, IFN$\gamma$, IFN$\alpha$, TNF$\alpha$, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist), suppressive stromal antagonist and chemotherapeutic agent, other therapeutic regimens may be combined therewith. For example, a second (third, fourth, *etc.*) chemotherapeutic agent(s) may be administered, wherein the second chemotherapeutic agent is an antimetabolite chemotherapeutic agent, or a chemotherapeutic agent that is not an antimetabolite. For example, the second chemotherapeutic agent may be a taxane (such as paclitaxel or docetaxel), capecitabine, or platinum-based chemotherapeutic agent (such as carboplatin, cisplatin, or oxaliplatin), anthracycline (such as doxorubicin, including, liposomal doxorubicin), topotecan, pemetrexed, vinca alkaloid (such as vinorelbine), and TLK 286. "Cocktails" of different chemotherapeutic agents may be administered.

Other therapeutic agents that may be combined with the agonist (*e.g.,* a CD28, OX40, GITR, CD137, CD27, ICOS, HVEM, NKG2D, MICA, 2B4, IL-2, IL-12, IFN$\gamma$, IFN$\alpha$, TNF$\alpha$, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist), and/or chemotherapeutic agent include any one or more of: a HER inhibitor, HER dimerization inhibitor (for example, a growth inhibitory HER2 antibody such as trastuzumab, or a HER2 antibody which induces apoptosis of a HER2-overexpressing cell, such as 7C2, 7F3 or humanized variants thereof); an antibody directed against a different tumor associated antigen, such as EGFR, HER3, HE R4; anti-hormonal compound, *e.g.,* an anti-estrogen compound such as tamoxifen, or an aromatase inhibitor; a cardioprotectant (to prevent or reduce any myocardial dysfunction associated with the therapy); a cytokine; an EGFR-targeted drug (such as TARCEVA® IRESSA® or cetuximab); a tyrosine kinase inhibitor; a COX inhibitor (for instance a COX-1 or COX-2 inhibitor); non-steroidal anti-inflammatory drug, celecoxib (CELEBREX®); farnesyl transferase inhibitor (for example, Tipifarnib/-ZARNESTRA® R115777 available from Johnson and Johnson or Lonafarnib SCH66336 available from Schering-Plough); antibody that binds oncofetal protein CA 125 such as Oregovomab (MoAb B43.13); HER2 vaccine (such as HER2AutoVac vaccine from Pharmexia, or APC8024 protein vaccine from Dendreon, or HER2 peptide vaccine from GSK/Corixa); another HER targeting therapy (e.g. trastuzumab, cetuximab, ABX-EGF, EMD7200, gefitinib, erlotinib, CP724714, CI1033, GW572016, IMC-11F8, TAK165, *etc*); Raf and/or ras inhibitor (see, for example, WO 2003/86467); doxorubicin HCl liposome injection (DOXIL®); topoisomerase 1 inhibitor such as topotecan; taxane; HER2 and EGFR dual tyrosine kinase inhibitor such as lapatinib/GW572016; TLK286 (TELCYTA®); EMD-7200; a medicament that treats nausea such as a serotonin antagonist, steroid, or benzodiazepine; a medicament that prevents or treats skin rash or standard acne therapies, including topical or oral antibiotic; a medicament that treats or prevents diarrhea; a body temperature-reducing medicament such as acetaminophen, diphenhydramine, or meperidine; hematopoietic growth factor, *etc.*

Suitable dosages for any of the above-noted co-administered agents are those presently used and may be lowered due to the combined action (synergy) of the agent and the agonist (*e.g.,* a CD28, OX40, GITR, CD137, CD27, ICOS, HVEM, NKG2D, MICA, 2B4, IL-2, IL-12, IFN$\gamma$, IFN$\alpha$, TNF$\alpha$, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) and suppressive stromal antagonist. In addition to the above therapeutic regimes, the patient may be subjected to surgical removal of tumors and/or cancer cells, and/or radiation therapy.

Where the agonist (*e.g.,* a CD28, OX40, GITR, CD137, CD27, ICOS, HVEM, NKG2D, MICA, 2B4, IL-2, IL-12, IFN$\gamma$, IFN$\alpha$, TNF$\alpha$, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) is an antibody, preferably the administered antibody is a naked antibody. The agonist (*e.g.,* a CD28, OX40, GITR, CD137, CD27, ICOS, HVEM, NKG2D, MICA, 2B4, IL-2, IL-12, IFN$\gamma$, IFN$\alpha$, TNF$\alpha$, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) administered may be conjugated with a cytotoxic agent. Preferably, the conjugate and/or antigen to which it is bound is/are internalized by the cell, resulting in increased therapeutic efficacy of the conjugate in killing the cancer cell to which it binds. In a preferred embodiment, the cytotoxic agent targets or interferes with nucleic acid in the cancer cell. Examples of such cytotoxic agents include maytansinoids, calicheamicins, ribonucleases, and DNA endonucleases.

The agonist (*e.g.,* a CD28, OX40, GITR, CD137, CD27, ICOS, HVEM, NKG2D, MICA, 2B4, IL-2, IL-12, IFN$\gamma$, IFN$\alpha$, TNF$\alpha$, IL-1, CDN, HMBG1, or TLR agonist) or antagonist (*e.g.,* a CTLA-4, PD-1 axis, TIM-3, BTLA, VISTA, LAG-3, B7H4, CD96, TIGIT, CD226, prostaglandin, VEGF, endothelin B, IDO, arginase, MICA/MICB, TIM-3, IL-10, IL-4, or IL-13 antagonist) can be administered by gene therapy. See, for example, WO 96/07321 published Mar. 14, 1996 concerning the use of gene therapy to generate intracellular antibodies. There are two major approaches to getting the nucleic acid (optionally contained in a vector) into the patient's cells; in vivo and ex vivo. For in vivo delivery the nucleic acid is injected directly into the patient, usually at the site where the antibody is required. For ex vivo treatment, the patient's cells are removed, the nucleic acid is introduced into these isolated cells and the modified cells are administered to the patient either directly or, for example, encapsulated within porous membranes which are implanted into the patient (see, *e.g.* U.S. Pat. Nos. 4,892,538 and 5,283,187). There are a variety of techniques available for introducing nucleic acids into viable cells. The techniques vary depending upon whether the nucleic acid is transferred into cultured cells in vitro or in vivo in the cells of the intended host. Techniques suitable for the transfer of nucleic acid into mammalian cells in vitro include the use of liposomes, electroporation, microinjection, cell fusion, DEAE-dextran, the calcium phosphate precipitation method, *etc.* A commonly used vector for ex vivo delivery of the gene is a retrovirus. The currently preferred in vivo nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). In some situations it is desirable to provide the nucleic acid source with an agent that targets the target cells, such as an antibody specific for a cell surface membrane protein or the target cell, a ligand for a receptor on the target cell, *etc.* Where liposomes are employed, proteins which bind to a cell surface membrane protein associated with endocytosis may be used for targeting and/or to facilitate uptake, *e.g.* capsid proteins or fragments thereof tropic for a particular cell type, antibodies for proteins which undergo internalization in cycling, and proteins that target intracellular localization and enhance intracellular half-life. The technique of receptor-mediated endocytosis is described, for example, by Wu et al., J. Biol. Chem. 262:44294432 (1987); and Wagner et al., Proc. Natl. Acad. Sci. USA 87:3410-3414 (1990). For review of the currently known gene marking and gene therapy protocols see Anderson et al., Science 256:808-813 (1992). See also WO 93/25673 and the references cited therein.

## III. Antibodies for use in the methods of the invention

In some embodiments of the invention, the immunotherapy and/or the suppressive stromal antagonist is an antibody. The invention provides various antibodies for use in the diagnosis and/or treatment of an individual that may benefit from the combined treatment of an immunotherapy and a suppressive stromal antagonist.

*Monoclonal antibodies*

In some embodiments, antibodies are monoclonal antibodies. Monoclonal antibodies are obtained from a population of substantially homogeneous antibodies, *i.e.,* the individual antibodies comprising the population are identical and/or bind the same epitope except for possible variants that arise during production of the monoclonal antibody, such variants generally being present in minor amounts. Thus, the modifier "monoclonal" indicates the character of the antibody as not being a mixture of discrete or polyclonal antibodies.

For example, the monoclonal antibodies may be made using the hybridoma method first described by Kohler et al., Nature 256:495 (1975), or may be made by recombinant DNA methods (U.S. Patent No. 4,816,567).

In the hybridoma method, a mouse or other appropriate host animal, is immunized as herein described to elicit lymphocytes that produce or are capable of producing antibodies that will specifically bind to the polypeptide used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986)).

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

In some embodiments, the myeloma cells are those that fuse efficiently, support stable high-level production of antibody by the selected antibody-producing cells, and are sensitive to a medium such as HAT medium. Among these, in some embodiments, the myeloma cell lines are murine myeloma lines, such as those derived from MOPC-21 and MPC-11 mouse tumors available from the Salk Institute Cell Distribution Center, San Diego, California USA, and SP-2 or X63-Ag8-653 cells available from the American Type Culture Collection, Rockville, Maryland USA. Human myeloma and mouse-human heteromyeloma cell lines also have been described for the production of human monoclonal antibodies (Kozbor, J. Immunol. 133:3001 (1984); Brodeur et al., Monoclonal Antibody Production Techniques and Applications pp. 51-63 (Marcel Dekker, Inc., New York, 1987)).

Culture medium in which hybridoma cells are growing is assayed for production of monoclonal antibodies directed against the antigen. In some embodiments, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

The binding affinity of the monoclonal antibody can, for example, be determined by the Scatchard analysis of Munson et al., Anal. Biochem. 107:220 (1980).

After hybridoma cells are identified that produce antibodies of the desired specificity, affinity, and/or activity, the clones may be subcloned by limiting dilution procedures and grown by standard methods (Goding, Monoclonal Antibodies: Principles and Practice pp. 59-103 (Academic Press, 1986)). Suitable culture media for this purpose include, for example, D-MEM or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, polypeptide A-Sepharose®, hydroxylapatite chromatography, gel electrophoresis, dialysis, or affinity chromatography.

DNA encoding the monoclonal antibodies is readily isolated and sequenced using conventional procedures (*e.g.,* by using oligonucleotide probes that are capable of binding specifically to genes encoding the heavy and light chains of murine antibodies). In some embodiments, the hybridoma cells serve as a source of such DNA. Once isolated, the DNA may be placed into expression vectors, which are then transfected into host cells such as *E. coli* cells, simian COS cells, human embryonic kidney (HEK) 293 cells, Chinese Hamster Ovary (CHO) cells, or myeloma cells that do not otherwise produce immunoglobulin polypeptide, to obtain the synthesis of monoclonal antibodies in the recombinant host cells. Review articles on recombinant expression in bacteria of DNA encoding the antibody include Skerra et al., Curr. Opinion in Immunol. 5:256-262 (1993) and Plückthun, Immunol. Revs., 130:151-188 (1992).

In a further embodiment, antibodies or antibody fragments can be isolated from antibody phage libraries generated using the techniques described in McCafferty et al., Nature 348:552-554 (1990). Clackson et al., Nature 352:624-628 (1991) and Marks et al., J. Mol. Biol. 222:581-597 (1991) describe the isolation of murine and human antibodies, respectively, using phage libraries. Subsequent publications describe the production of high affinity (nM range) human antibodies by chain shuffling (Marks et al., Bio/Technology 10:779-783 (1992)), as well as combinatorial infection and in vivo recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA also may be modified, for example, by substituting the coding sequence for human heavy- and light chain constant domains in place of the homologous murine sequences (U.S. Patent No. 4,816,567; Morrison et al., Proc. Natl Acad. Sci. USA 81:6851 (1984)), or by covalently joining to the immunoglobulin coding sequence all or part of the coding sequence for a non-immunoglobulin polypeptide.

Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

In some embodiments of any of the methods described herein, the antibody is IgA, IgD, IgE, IgG, or IgM. In some embodiments, the antibody is an IgG monoclonal antibody.

*Antibody fragments*

In some embodiments, an antibody is an antibody fragment. Various techniques have been developed for the production of antibody fragments. Traditionally, these fragments were derived via proteolytic digestion of intact antibodies (see, *e.g.,* Morimoto et al., Journal of Biochemical and Biophysical Methods 24:107-117 (1992) and Brennan et al., Science 229:81 (1985)). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form F(ab')2 fragments (Carter et al., Bio/Technology 10:163-167 (1992)). According to another approach, F(ab')2 fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent No. 5,571,894; and US Patent No. 5,587,458. The antibody fragment may also be a "linear antibody," *e.g.,* as described in US Patent 5,641,870 for example. Such linear antibody fragments may be monospecific or bispecific.

In some embodiments, fragments of the antibodies described herein are provided. In some embodiments, the antibody fragment is an antigen binding fragment. In some embodiments, the antigen binding fragment is selected from the group consisting of a Fab fragment, a Fab' fragment, a F(ab')2 fragment, a scFv, a Fv, and a diabody.

Polypeptide Variants and Modifications

In certain embodiments, amino acid sequence variants of the proteins herein are contemplated. For example, it may be desirable to improve the binding affinity and/or other biological properties of the protein. Amino acid sequence variants of a protein may be prepared by introducing appropriate modifications into the nucleotide sequence encoding the protein, or by peptide synthesis. Such modifications include, for example, deletions from, and/or insertions into and/or substitutions of residues within the amino acid sequences of the protein. Any combination of deletion, insertion, and substitution can be made to arrive at the final construct, provided that the final construct possesses the desired characteristics.

*Variant Polypeptides*

"Polypeptide variant" means a polypeptide, for example, an active polypeptide, as defined herein having at least about 80% amino acid sequence identity with a full-length native sequence of the polypeptide, a polypeptide sequence lacking the signal peptide, an extracellular domain of a polypeptide, with or without the signal peptide. Such polypeptide variants include, for instance, polypeptides wherein one or more amino acid residues are added, or deleted, at the N or C-terminus of the full-length native amino acid sequence. Ordinarily, a polypeptide variant will have at least about 80% amino acid sequence identity, alternatively at least about any of 85%, 90%, 95%, 96%, 97%, 98%, or 99% amino acid sequence identity, to a full-length native sequence polypeptide sequence, a polypeptide sequence lacking the signal peptide, an extracellular domain of a polypeptide, with or without the signal peptide. Optionally, variant polypeptides will have no more than one conservative amino acid substitution as compared to the native polypeptide sequence, alternatively no more than about any of 2, 3, 4, 5, 6, 7, 8, 9, or 10 conservative amino acid substitution as compared to the native polypeptide sequence.

The variant polypeptide may be truncated at the N-terminus or C-terminus, or may lack internal residues, for example, when compared with a full length native polypeptide. Certain variant polypeptides may lack amino acid residues that are not essential for a desired biological activity. These variant polypeptides with truncations, deletions, and insertions may be prepared by any of a number of conventional techniques. Desired variant polypeptides may be chemically synthesized. Another suitable technique involves isolating and amplifying a nucleic acid fragment encoding a desired variant polypeptide, by polymerase chain reaction (PCR). Oligonucleotides that define the desired termini of the nucleic acid fragment are employed at the 5' and 3' primers in the PCR. Preferably, variant polypeptides share at least one biological and/or immunological activity with the native polypeptide disclosed herein.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme or a polypeptide which increases the serum half-life of the antibody.

For example, it may be desirable to improve the binding affinity and/or other biological properties of the polypeptide. Amino acid sequence variants of the polypeptide are prepared by introducing appropriate nucleotide changes into the antibody nucleic acid, or by peptide synthesis. Such modifications include, for example, deletions from, and/or insertions into and/or substitutions of, residues within the amino acid sequences of the polypeptide. Any combination of deletion, insertion, and substitution is made to arrive at the final construct, provided that the final construct possesses the desired characteristics. The amino acid changes also may alter post-translational processes of the polypeptide (*e.g.*, antibody), such as changing the number or position of glycosylation sites.

Guidance in determining which amino acid residue may be inserted, substituted or deleted without adversely affecting the desired activity may be found by comparing the sequence of the polypeptide with that of homologous known polypeptide molecules and minimizing the number of amino acid sequence changes made in regions of high homology.

A useful method for identification of certain residues or regions of the polypeptide (*e.g.,* antibody) that are preferred locations for mutagenesis is called "alanine scanning mutagenesis" as described by Cunningham and Wells, Science 244:1081-1085 (1989). Here, a residue or group of target residues are identified (*e.g.,* charged residues such as Arg, Asp, His, Lys, and Glu) and replaced by a neutral or negatively charged amino acid (most preferably Alanine or Polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at the target codon or region and the expressed antibody variants are screened for the desired activity.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. If such substitutions result in a change in biological activity, then more substantial changes, denominated "exemplary substitutions" in the Table 1, or as further described below in reference to amino acid classes, may be introduced and the products screened.

Table 1.

| Original Residue | Exemplary Substitutions | Conservative Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in the biological properties of the polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, Biochemistry second ed., pp. 73-75, Worth Publishers, New York (1975)):

(1) non-polar: Ala (A), Val (V), Leu (L), Ile (I), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the polypeptide to improve its stability (particularly where the antibody is an antibody fragment such as an Fv fragment).

One example of substitutional variant involves substituting one or more hypervariable region residues of a parent antibody (*e.g.,* a humanized antibody). Generally, the resulting variant(s) selected for further development will have improved biological properties relative to the parent antibody from which they are generated. A convenient way for generating such substitutional variants involves affinity maturation using phage display. Briefly, several hypervariable region sites (*e.g.,* 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (*e.g.,* binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and target. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the polypeptide alters the original glycosylation pattern of the antibody. The polypeptide may comprise non-amino acid moieties. For example, the polypeptide may be glycosylated. Such glycosylation may occur naturally during expression of the polypeptide in the host cell or host organism, or may be a deliberate modification arising from human intervention. By altering is meant deleting one or more carbohydrate moieties found in the polypeptide, and/or adding one or more glycosylation sites that are not present in the polypeptide.

Glycosylation of polypeptide is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the polypeptide is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Removal of carbohydrate moieties present on the polypeptide may be accomplished chemically or enzymatically or by mutational substitution of codons encoding for amino acid residues that serve as targets for glycosylation. Enzymatic cleavage of carbohydrate moieties on polypeptides can be achieved by the use of a variety of endo- and exo-glycosidases.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the γ-amino groups of lysine, arginine, and histidine side chains, acetylation of the N-terminal amine, and amidation of any C-terminal carboxyl group.

*Chimeric Polypeptides*

The polypeptide described herein may be modified in a way to form chimeric molecules comprising the polypeptide fused to another, heterologous polypeptide or amino acid sequence. In some embodiments, a chimeric molecule comprises a fusion of the polypeptide with a tag polypeptide which provides an epitope to which an anti-tag antibody can selectively bind. The epitope tag is generally placed at the amino- or carboxyl-terminus of the polypeptide. The presence of such epitope-tagged forms of the polypeptide can be detected using an antibody against the tag polypeptide. Also, provision of the epitope tag enables the polypeptide to be readily purified by affinity purification using an anti-tag antibody or another type of affinity matrix that binds to the epitope tag.

*Multispecific Antibodies*

In certain embodiments, an antibody provided herein is a multispecific antibody, *e.g.* a bispecific antibody. In certain embodiments, multispecific antibodies are monoclonal antibodies that have binding specificities for at least two different sites. In certain embodiments, one of the binding specificities is for one antigen and the other is for any other antigen. In certain embodiments, bispecific antibodies may bind to two different epitopes of the same antigen. Bispecific antibodies may also be used to localize cytotoxic agents to cells which express a particular antigen. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983), WO 93/08829, and Traunecker et al., EMBO J. 10: 3655 (1991)), and "knob-in-hole" engineering (see, *e.g.,* U.S. Patent No. 5,731,168), and in addition exchanging one or more heavy chain and light chain domains within the antigen-binding fragment (Fab) of one half of the bi-specific antibody (CrossMab, see WO2009/080251, WO2009/080252, WO2009/080253, and WO2009/080254). Multi-specific antibodies may also be made by engineering electrostatic steering effects for making antibody Fc-heterodimeric molecules (WO 2009/089004A1); cross-linking two or more antibodies or fragments (see, *e.g.,* US Patent No. 4,676,980, and Brennan et al., Science, 229: 81 (1985)); using leucine zippers to produce bi-specific antibodies (see, *e.g.,* Kostelny et al., J. Immunol., 148(5):1547-1553 (1992)); using "diabody" technology for making bispecific antibody fragments (see, *e.g.,* Hollinger et al., Proc. Natl. Acad. Sci. USA, 90:6444-6448 (1993)); and using single-chain Fv (sFv) dimers (see, *e.g.* Gruber et al., J. Immunol., 152:5368 (1994)); and preparing trispecific antibodies as described, *e.g.,* in Tutt et al. J. Immunol. 147: 60 (1991).

Engineered antibodies with three or more functional antigen binding sites, including "Octopus antibodies," are also included herein (see, *e.g.* US 2006/0025576A1).

The antibody or fragment herein also includes a "Dual Acting FAb" or "DAF" comprising an antigen binding site that binds to one antigen as well as another, different antigen (see, US 2008/0069820, for example).

**IV. Vectors, Host Cells, and Recombinant Methods**

For recombinant production of a heterologous polypeptide (*e.g.,* an antibody), the nucleic acid encoding it is isolated and inserted into a replicable vector for further cloning (amplification of the DNA) or for expression. DNA encoding the polypeptide (*e.g.,* antibody) is readily isolated and sequenced using conventional procedures (*e.g.,* by using oligonucleotide probes that are capable of binding specifically to genes encoding the heavy and light chains of the antibody). Many vectors are available. The choice of vector depends in part on the host cell to be used. Generally, preferred host cells are of either prokaryotic origin. It will be appreciated that constant regions of any isotype can be used for this purpose, including IgG, IgM, IgA, IgD, and IgE constant regions, and that such constant regions can be obtained from any human or animal species.

A. Generating antibodies using prokaryotic host cells

*i. Vector Construction*

Polynucleotide sequences encoding polypeptide components of the polypeptide (*e.g.,* antibody) of the invention can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the polypeptides are inserted into a recombinant vector capable of replicating and expressing heterologous polynucleotides in prokaryotic hosts. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication, a selection marker gene, a promoter, a ribosome binding site (RBS), a signal sequence, the heterologous nucleic acid insert and a transcription termination sequence.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, *E. coli* is typically transformed using pBR322, a plasmid derived from an *E. coli* species. pBR322 contains genes encoding ampicillin (Amp) and tetracycline (Tet) resistance and thus provides easy means for identifying transformed cells. pBR322, its derivatives, or other microbial plasmids or bacteriophage may also contain, or be modified to contain, promoters which can be used by the microbial organism for expression of endogenous proteins. Examples of pBR322 derivatives used for expression of particular antibodies are described in detail in Carter et al., U.S. Patent No. 5,648,237.

In addition, phage vectors containing replicon and control sequences that are compatible with the host microorganism can be used as transforming vectors in connection with these hosts. For example, bacteriophage such as GEM™-11 may be utilized in making a recombinant vector which can be used to transform susceptible host cells such as *E. coli* LE392.

The expression vector of the invention may comprise two or more promoter-cistron pairs, encoding each of the polypeptide components. A promoter is an untranslated regulatory sequence located upstream (5') to a cistron that modulates its expression. Prokaryotic promoters typically fall into two classes, inducible and constitutive. Inducible promoter is a promoter that initiates increased levels of transcription of the cistron under its control in response to changes in the culture condition, *e.g.,* the presence or absence of a nutrient or a change in temperature.

A large number of promoters recognized by a variety of potential host cells are well known. The selected promoter can be operably linked to cistron DNA encoding the light or heavy chain by removing the promoter from the source DNA via restriction enzyme digestion and inserting the isolated promoter sequence into the vector of the invention. Both the native promoter sequence and many heterologous promoters may be used to direct amplification and/or expression of the target genes. In some embodiments, heterologous promoters are utilized, as they generally permit greater transcription and higher yields of expressed target gene as compared to the native target polypeptide promoter.

Promoters suitable for use with prokaryotic hosts include the PhoA promoter, the - lactamase and lactose promoter systems, a tryptophan (trp) promoter system and hybrid promoters such as the tac or the trc promoter. However, other promoters that are functional in bacteria (such as other known bacterial or phage promoters) are suitable as well. Their nucleotide sequences have been published, thereby enabling a skilled worker operably to ligate them to cistrons encoding the target light and heavy chains (Siebenlist et al., (1980) Cell 20: 269) using linkers or adaptors to supply any required restriction sites.

The translational initiation region (TIR) is a major determinant of the overall translation level of a protein. The TIR includes the polynucleotide that encodes the signal sequence, and extends from immediately upstream of the Shine-Delgarno sequence to approximately twenty nucleotides downstream of the initiation codon. Generally, the vector will comprise a TIR, TIRs and variant TIRs are known in the art and methods for generating TIRs are known in in the art A series of nucleic acid sequence variants can be created with a range of translational strengths, thereby providing a convenient means by which to adjust this factor for the optimal secretion of many different polypeptides. The use of a reporter gene fused to these variants, such as PhoA, provides a method to quantitate the relative translational strengths of different translation initiation regions. The variant or mutant TIRs can be provided in the background of a plasmid vector thereby providing a set of plasmids into which a gene of interest may be inserted and its expression measured, so as to establish an optimum range of translational strengths for maximal expression of mature polypeptide. Variant TIRs are disclosed in USP 8,241,901.

In one aspect of the invention, each cistron within the recombinant vector comprises a secretion signal sequence component that directs translocation of the expressed polypeptides across a membrane. In general, the signal sequence may be a component of the vector, or it may be a part of the target polypeptide DNA that is inserted into the vector. The signal sequence selected for the purpose of this invention should be one that is recognized and processed (*i.e.,* cleaved by a signal peptidase) by the host cell. For prokaryotic host cells that do not recognize and process the signal sequences native to the heterologous polypeptides, the signal sequence is substituted by a prokaryotic signal sequence selected, for example, from the signal polypeptides of the present invention. In addition, the vector may comprise a signal sequence selected from the group consisting of alkaline phosphatase, penicillinase, Lpp, or heat-stable enterotoxin II (STII) leaders, LamB, PhoE, PelB, OmpA, and MBP.

In one aspect, one or more polynucleotides (*e.g.*, expression vectors) collectively encode an antibody. In one embodiment, a single polynucleotide encodes the light chain of the antibody and a separate polynucleotide encodes the heavy chain of the antibody. In one embodiment, a single polynucleotide encodes the light chain and heavy chain of the antibody. In some embodiments, one or more polynucleotides (*e.g.*, expression vectors) collectively encode a one-armed antibody. In one embodiment, a single polynucleotide encodes (a) the light and heavy chain of the one armed antibody, and (b) the Fc polypeptide. In one embodiment, a single polynucleotide encodes the light and heavy chain of the one armed antibody, and a separate polynucleotide encodes the Fc polypeptide. In one embodiment, separate polynucleotides encode the light chain component of the one-armed antibody, the heavy chain component of the one-armed antibody and the Fc polypeptide, respectively. Production of a one-armed antibody is described in, for example, in WO2005063816.

Prokaryotic host cells suitable for expressing antibodies of the invention include Archaebacteria and Eubacteria, such as Gram-negative or Gram-positive organisms. Examples of useful bacteria include Escherichia (*e.g., E. coli*), Bacilli (*e.g., B. subtilis*), *Enterobacteria, Pseudomonas* species (*e.g., P. aeruginosa*), *Salmonella typhimurium, Serratia marcescans,* Klebsiella, Proteus, Shigella, Rhizobia, Vitreoscilla, or Paracoccus. In one embodiment, gram-negative cells are used. In one embodiment, *E. coli* cells are used as hosts for the invention. Examples of *E. coli* strains include strain W3110 (Bachmann, Cellular and Molecular Biology, vol. 2 (Washington, D.C.: American Society for Microbiology, 1987), pp. 1190-1219; ATCC Deposit No. 27,325) and derivatives thereof, including strain 33D3 having genotype W3110 ΔfhuA (ΔtonA) ptr3 lac Iq lacL8 ΔompTΔ (nmpc-fepE) degP41 kanR (U.S. Pat. No. 5,639,635) and strains 63C1 and 64B4. In some embodiment, the *E. coli* strain is a W3110 derivative named 62A7 (ΔfhuA (ΔtonA) ptr3, lacIq, lacL8, ompTΔ(nmpc-fepE) ΔdegP ilvG repaired). Other strains and derivatives thereof, such as *E. coli* 294 (ATCC 31,446), *E. coli* B, *E. coli* λ 1776 (ATCC 31,537) and *E. coli* RV308(ATCC 31,608) are also suitable. These examples are illustrative rather than limiting. Methods for constructing derivatives of any of the above-mentioned bacteria having defined genotypes are known in the art and described in, for example, Bass et al., Proteins, 8:309-314 (1990). It is generally necessary to select the appropriate bacteria taking into consideration replicability of the replicon in the cells of a bacterium. For example, *E. coli,* Serratia, or Salmonella species can be suitably used as the host when well known plasmids such as pBR322, pBR325, pACYC177, or pKN410 are used to supply the replicon. Typically the host cell should secrete minimal amounts of proteolytic enzymes, and additional protease inhibitors may desirably be incorporated in the cell culture.

To improve the production yield and quality of the polypeptides in bacterial cultures, the bacterial cells can be modified. For example, to improve the proper assembly and folding of the secreted antibody polypeptides, the bacteria host cell may comprise additional vectors expressing chaperone proteins, such as FkpA and Dsb proteins (DsbB, DsbC, DsbD, and/or DsbG) can be used to co-transform the host prokaryotic cells. The chaperone proteins have been demonstrated to facilitate the proper folding and solubility of heterologous proteins produced in bacterial host cells.

*ii. Antibody Production*

Host cells are transformed with the above-described expression vectors and cultured in conventional nutrient media modified as appropriate for inducing promoters, selecting transformants, or amplifying the genes encoding the desired sequences.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

Prokaryotic cells used to produce the polypeptides of the invention are grown in media known in the art and suitable for culture of the selected host cells. Examples of suitable media include Luria broth (LB) plus necessary nutrient supplements. In some embodiments, the media also contains a selection agent, chosen based on the construction of the expression vector, to selectively permit growth of prokaryotic cells containing the expression vector. For example, ampicillin is added to media for growth of cells expressing ampicillin resistant gene.

Any necessary supplements besides carbon, nitrogen, and inorganic phosphate sources may also be included at appropriate concentrations introduced alone or as a mixture with another supplement or medium such as a complex nitrogen source. Optionally the culture medium may contain one or more reducing agents selected from the group consisting of glutathione, cysteine, cystamine, thioglycollate, dithioerythritol and dithiothreitol.

The prokaryotic host cells are cultured at suitable temperatures. For *E. coli* growth, for example, the preferred temperature ranges from about 20 °C to about 39 °C, more preferably from about 25 °C to about 37 °C, even more preferably at about 30 °C. The pH of the medium may be any pH ranging from about 5 to about 9, depending mainly on the host organism. For *E. coli,* the pH is preferably from about 6.8 to about 7.4, and more preferably about 7.0.

If an inducible promoter is used in the expression vector of the invention, protein expression is induced under conditions suitable for the activation of the promoter. In one aspect of the invention, PhoA promoters are used for controlling transcription of the polypeptides. Accordingly, the transformed host cells are cultured in a phosphate-limiting medium for induction. Preferably, the phosphate-limiting medium is the C.R.A.P medium (see, *e.g.,* Simmons et al., J. Immunol. Methods (2002), 263:133-147) or media described in WO2002/061090. A variety of other inducers may be used, according to the vector construct employed, as is known in the art.

In one embodiment, the expressed polypeptides of the present invention are secreted into and recovered from the periplasm of the host cells. Protein recovery typically involves disrupting the microorganism, generally by such means as osmotic shock, sonication or lysis. Once cells are disrupted, cell debris or whole cells may be removed by centrifugation or filtration. The proteins may be further purified, for example, by affinity resin chromatography. Alternatively, proteins can be transported into the culture media and isolated therein. Cells may be removed from the culture and the culture supernatant being filtered and concentrated for further purification of the proteins produced. The expressed polypeptides can be further isolated and identified using commonly known methods such as polyacrylamide gel electrophoresis (PAGE) and Western blot assay.

In one aspect of the invention, antibody production is conducted in large quantity by a fermentation process. Various large-scale fed-batch fermentation procedures are available for production of recombinant polypeptides. Large-scale fermentations have at least 1000 liters of capacity, preferably about 1,000 to 100,000 liters of capacity. These fermentors use agitator impellers to distribute oxygen and nutrients, especially glucose (the preferred carbon/energy source). Small scale fermentation refers generally to fermentation in a fermenter that is no more than approximately 100 liters in volumetric capacity, and can range from about 1 liter to about 100 liters.

In a fermentation process, induction of protein expression is typically initiated after the cells have been grown under suitable conditions to a desired density, *e.g.,* an $OD_{550}$ of about 180-220, at which stage the cells are in the early stationary phase. A variety of inducers may be used, according to the vector construct employed, as is known in the art and described above. Cells may be grown for shorter periods prior to induction. Cells are usually induced for about 12-50 hours, although longer or shorter induction time may be used.

To minimize proteolysis of expressed heterologous proteins (especially those that are proteolytically sensitive), certain host strains deficient for proteolytic enzymes can be used for the present invention. For example, host cell strains may be modified to effect genetic mutation(s) in the genes encoding known bacterial proteases such as Protease III, OmpT, DegP, Tsp, Protease I, Protease Mi, Protease V, Protease VI, and combinations thereof. Some *E. coli* protease-deficient strains are available and described in, for example, Joly *et al.,* (1998), *supra*; Georgiou et al., U.S. Patent No. 5,264,365; Georgiou et al., U.S. Patent No. 5,508,192; Hara et al., Microbial Drug Resistance, 2:63-72 (1996).

In one embodiment, *E. coli* strains deficient for proteolytic enzymes and transformed with plasmids expressing one or more chaperone proteins are used as host cells in the expression system of the invention.

*iii. Antibody Purification*

Standard protein purification methods known in the art can be employed. The following procedures are exemplary of suitable purification procedures: fractionation on immunoaffinity or ion-exchange columns, ethanol precipitation, reverse phase HPLC, chromatography on silica or on a cation-exchange resin such as SP or an anion exchange such as DEAE, chromatofocusing, SDS-PAGE, ammonium sulfate precipitation, and gel filtration using, for example, Sephadex G-75.

In one aspect, Protein A immobilized on a solid phase is used for immunoaffinity purification of the antibody products of the invention. Protein A is a 41kD cell wall protein from *Staphylococcus aureas* which binds with a high affinity to the Fc region of antibodies. Lindmark et al., (1983) J. Immunol. Meth. 62:1-13. The solid phase to which Protein A is immobilized is preferably a column comprising a glass or silica surface, more preferably a controlled pore glass column or a silicic acid column. In some applications, the column has been coated with a reagent, such as glycerol, in an attempt to prevent nonspecific adherence of impurities.

As the first step of purification, the preparation derived from the cell culture as described above is applied onto the Protein A immobilized solid phase to allow specific binding of the antibody of interest to Protein A. The solid phase is then washed to remove impurities non-specifically bound to the solid phase. Finally the antibody of interest is recovered from the solid phase by elution.

The invention also provides immunoconjugates (interchangeably termed "antibody-drug conjugates" or "ADC"), comprising any of the antibodies described herein conjugated to, *e.g.,* a cytotoxic agent such as a chemotherapeutic agent, a drug, a growth inhibitory agent, a toxin (*e.g.,* an enzymatically active toxin of bacterial, fungal, plant, or animal origin, or fragments thereof), or a radioactive isotope (*i.e.,* a radioconjugate).

**V. Diagnostic Kits, Assays and Articles of Manufacture**

Provided herein are diagnostic kits comprising one or more reagent for determining the presence of a stromal gene signature in a sample from an individual with a disease or disorder, wherein the presence of a stromal gene signature means a higher likelihood of efficacy when the individual is treated with a combination of an immunotherapy and a suppressive stromal antagonist. In some embodiments, the article of manufacture further comprises an immunotherapy. Optionally, the kit further comprises instructions to use the kit to select a medicament (*e.g.* a suppressive stromal antagonist, such as an anti-TGFβ antibody) for treating the disease or disorder if the individual expresses the stromal gene signature.

Provided herein are also assay for identifying an individual with a disease or disorder to receive a suppressive stromal antagonist in combination with an immunotherapy, the method comprising: determining the presence of a suppressive stromal antagonist in a sample from the individual, and recommending a suppressive stromal antagonist based on the presence of a stromal gene signature.

Provided herein are also articles of manufacture comprising, packaged together, a suppressive stromal antagonist in a pharmaceutically acceptable carrier and a package insert indicating that the suppressive stromal antagonist (*e.g.*, anti-TGFβ antibodies) is for treating a patient with a disease or disorder based on expression of a stromal gene signature. In some embodiments, the article of manufacture further comprises an immunotherapy. Treatment methods include any of the treatment methods disclosed herein. Further provided are the invention concerns a method for manufacturing an article of manufacture comprising combining in a package a pharmaceutical composition comprising a suppressive stromal antagonist (*e.g.*, anti-TGFβ antibodies), optionally an immunotherapy, and a package insert indicating that the pharmaceutical composition is for treating a patient with a disease or disorder based on expression of stromal gene signature.

The article of manufacture comprises a container and a label or package insert on or associated with the container. Suitable containers include, for example, bottles, vials, syringes, *etc.* The containers may be formed from a variety of materials such as glass or plastic. The container holds or contains a composition comprising the cancer medicament as the active agent and may have a sterile access port (for example the container may be an intravenous solution bag or a vial having a stopper pierceable by a hypodermic injection needle).

The article of manufacture may further comprise a second container comprising a pharmaceutically-acceptable diluent buffer, such as bacteriostatic water for injection (BWFI), phosphate buffered saline, Ringer's solution and dextrose solution. The article of manufacture may further include other materials desirable from a commercial and user standpoint, including other buffers, diluents, filters, needles, and syringes.

The article of manufacture of the present invention also includes information, for example in the form of a package insert, indicating that the composition is used for treating cancer based on expression level of the stromal gene signature herein. The insert or label may take any form, such as paper or on electronic media such as a magnetically recorded medium (*e.g.,* floppy disk) or a CD-ROM. The label or insert may also include other information concerning the pharmaceutical compositions and dosage forms in the kit or article of manufacture.

The invention also concerns a method for manufacturing an article of manufacture comprising combining in a package a pharmaceutical composition comprising a suppressive stromal antagonist (*e.g.*, an anti-TGFβ antibody), optionally an immunotherapy, and a package insert indicating that the pharmaceutical composition is for treating a patient with cancer (such as NSCLC) based on expression of a stromal gene signature.

The article of manufacture may further comprise an additional container comprising a pharmaceutically acceptable diluent buffer, such as bacteriostatic water for injection (BWFI), phosphate buffered saline, Ringer's solution, and/or dextrose solution. The article of manufacture may further include other materials desirable from a commercial and user standpoint, including other buffers, diluents, filters, needles, and syringes.

## VI. Exemplary embodiments

Further details of the invention are illustrated by the following non-limiting Examples.

EXAMPLES

The examples below are intended to be purely exemplary of the invention and should therefore not be considered to limit the invention in any way. The following examples and detailed description are offered by way of illustration and not by way of limitation.

## Example 1: Tumor infiltrating stromal gene signatures across urothelial carcinoma (UC) and their association with resistance to anti-PD-L1 antibody treatment

*Introduction*

To evaluate and understand the complexity of factors that may modulate or inhibit anti-tumor immunity and thus contribute to resistance to immune modulatory therapy, a highly sensitive immune gene expression assay was performed to interrogate the tumor microenvironment (TME) of pretreatment tumor tissues from urothelial carcinoma patients.

*Materials and Methods*

The Illumina TruSeq RNA Access RNA-seq kit was performed to interrogate the tumor microenvironment (TME) of pretreatment tumor tissues from urothelial carcinoma patients (n=217). The Illumina TruSeq RNA Access RNA-seq captures and interrogates genes across the whole human genomes (> 20,000 genes).

RNA was extracted from formalin-fixed paraffin embedded archival tissues. The tumor tissues were clinical collections from the Phase II IMvigor210 study (ClinicalTrials.gov number, NCT02108652) of Atezolizumab (MPDL3280A; anti-PD-L1 antibody). Appropriate patient informed consents were obtained from the institutional review boards for the exploratory evaluation of biomarkers.

Genes associated with stromal elements in the tumors were specifically interrogated for their association with overall response rate per RECIST v1.1. The extended gene-set (Set A) for the urothelial stroma-associated genes included: PDPN, FAP, TGFB1, NNMT, TNFAIP6, DKK3, MMP2, MMP8, MMP9, BGN, COL4A1, COL4A2, COL5A1, PDGFRB, NUAK1, FN1, FGF1, PDLIM4 and LRR32C. Gene Set B for the urothelial stroma-associated genes included: TGFB1, DKK3, PDGFRB, NUAK1, FGF1, PDLIM4, and LRRC32.

Response categories included complete response (CR); partial response (PR); stable disease (SD) and disease progression (PD). CR and PR patients were categorized as "responders". Statistical testing was performed using Kruskal-Wallis (KW) test, a non-parametric test used to compare three or more independent data groups.

*Results*

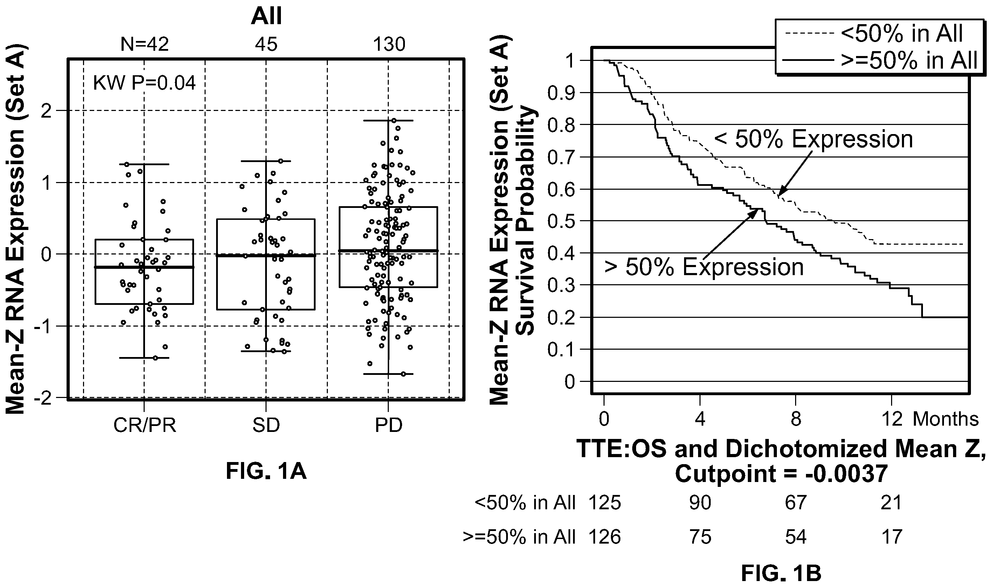

The mean expression (mean-Z) of the urothelial stroma-associated gene Set A was higher in the PD population compared to the responders (CR/PR) (**FIG. 1A**). Furthermore, when overall survival (OS) was computed based on the level of urothelial stroma-associated gene expression, patients with higher than 50% mean-Z expression did poorly with a hazard ratio (HR) of 1.47 (95%CI 1.07-2.02) compared to those with lower than 50% mean-Z expression with a HR of 0.68 (95%CI 0.49- 0.93) (**FIG. 1B**).

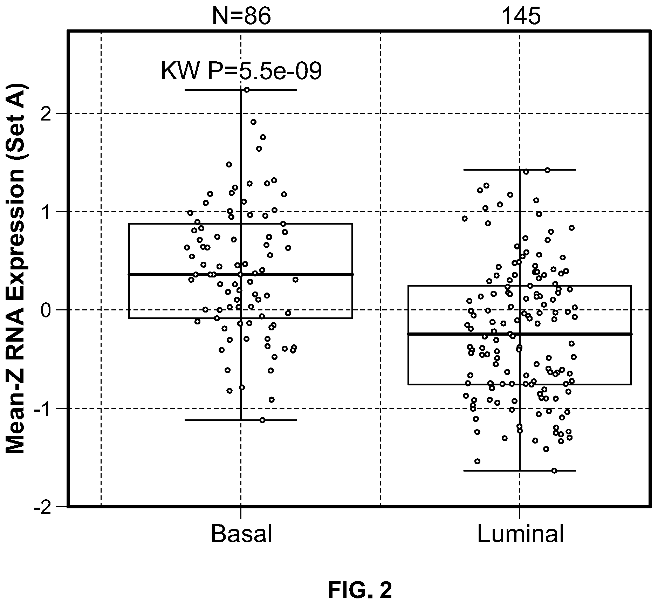

Expression of the urothelial stroma-associated gene set expression in luminal versus basal urothelial carcinoma molecular subtyping suggested by The Cancer Genome Atlas (TCGA) was evaluated. Patient tumor samples were characterized as luminals or basals based on the differential expression of FGFR3, CDKN2A, KRT5, KRT14, EGFR, GATA3, FOXA1, and ERBB2. Urothelial stroma-associated gene Set A expression was higher in the basal molecular subtype compared to the luminals (**FIG. 2**).

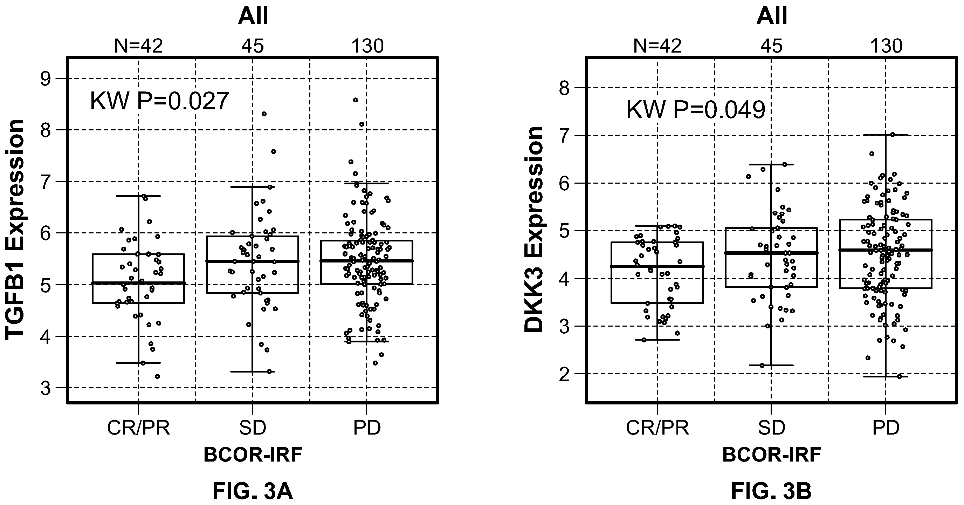

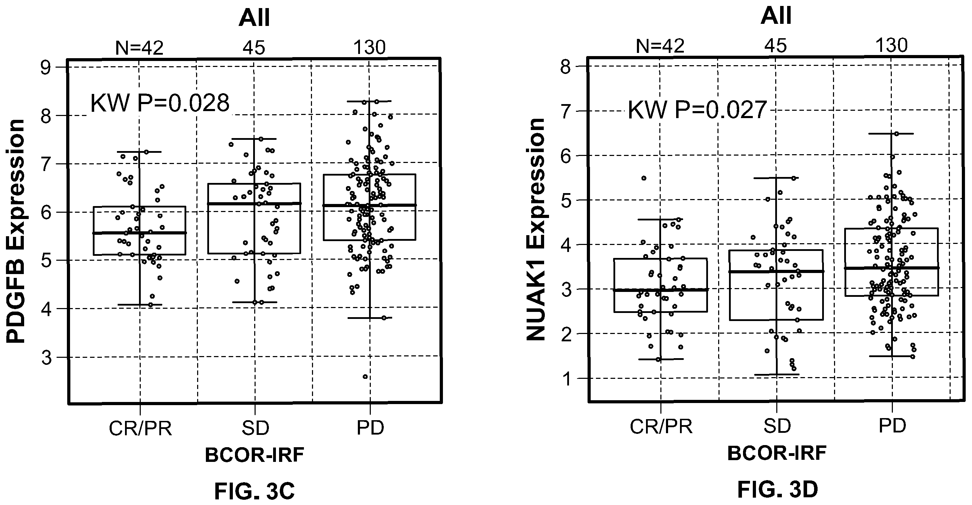

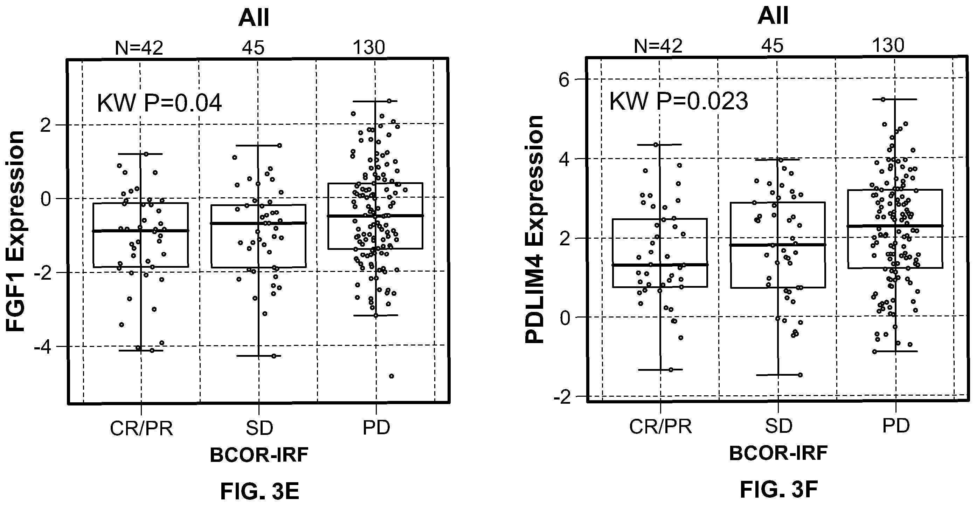

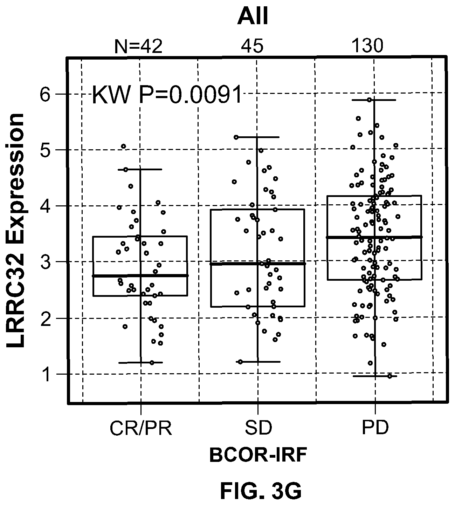

Association between individual genes within the urothelial stroma-associated gene Set A and response to anti-PD-L1 therapy was evaluated. Among these genes, TGFB1 (p=0.027), DKK3 (p=0.049), PDGFRB (p=0.028), NUAK1 (p=0.027), FGF1 (p=0.04), PDLIM4 (p=0.027) and LRRC32 (0.009) showed significantly lower expression among the responders (CR/PR), compared to patients with progressive disease (PD) (**FIGS. 3A-FIG. 3G**, respectively).

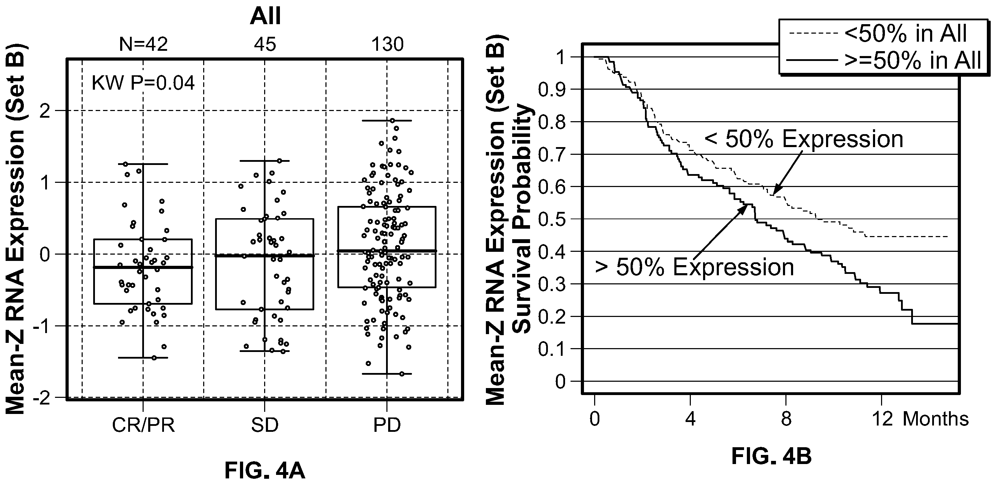

These individual highly significant genes were then combined to make up a more concise urothelial stroma-associated genes set (Set B: TGFB1, DKK3, PDGFRB, NUAK1, FGF1, PDLIM4, and LRRC32). This set was able to better distinguish the responders (CR/PR) from those with either stable disease (SD) or progressive disease (PD) (p=0.0064) (**FIG. 4A**). Furthermore, when overall survival (OS) was computed based on the level of urothelial stroma-associated gene expression, patients with higher than 50% mean-Z expression did poorly with a hazard ratio (HR) of 1.49 (95%CI 1.09-2.05) compared to those with lower than 50% mean-Z expression with a HR of 0.67 (95%CI 0.49- 0.92) (**FIG. 4B**).

**Example 2: Tumor stromal signatures across five cancer types and their association with disease prognostic factors**

*Introduction*

To further evaluate and understand the complexity of factors that may modulate or inhibit anti-tumor immunity and thus contribute to response or resistance to immune modulatory therapy, a highly sensitive immune gene expression assay was performed to interrogate the tumor microenvironment (TME) of pretreatment tumor tissues from breast cancer (BC), lung cancer, melanoma, RCC, and bladder cancer.

*Materials and Methods*

The Illumina TruSeq RNA Access RNA-seq kit was performed to interrogate the tumor microenvironment (TME) of pretreatment tumor tissues from BC (n=73), lung (n=59), melanoma (n=34), RCC (n=55), and bladder cancer (n=44) patients. The Illumina TruSeq RNA Access RNA-seq captures and interrogates genes across the whole human genomes (> 20,000 genes).

RNA was extracted from formalin-fixed paraffin embedded archival tissues that were derived from the ongoing Phase I study of MPDL3280A (anti-PD-L1 antibody). Appropriate patient informed consents were obtained from the institutional review boards for the exploratory evaluation of biomarkers.

Genes associated with stromal elements in the tumors were specifically interrogated for their association with overall response rate per RECIST v1.1. The extended gene-set (Set A) for the urothelial stroma-associated genes included: FAP, FN1, MMP2, BGN, LOXL2, PDPN, PDGFRB, COL12a1, COL5A1, COL8A2, THY1, and PALLD.

Response categories included complete response (CR); partial response (PR); stable disease (SD) and disease progression (PD). CR and PR patients were categorized as "responders". Statistical testing was performed using Kruskal-Wallis (KW) test, a non-parametric test used to compare three or more independent data groups.

*Results*

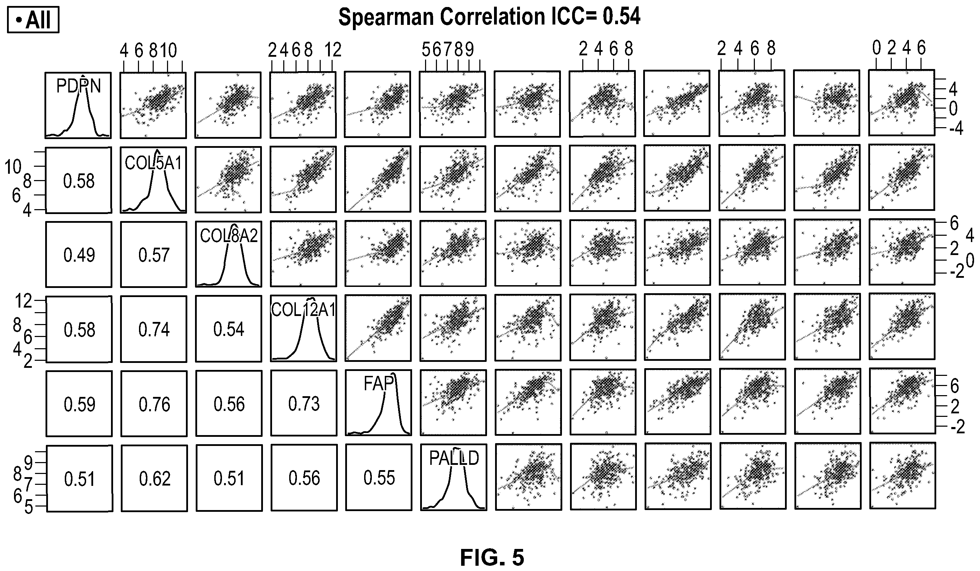

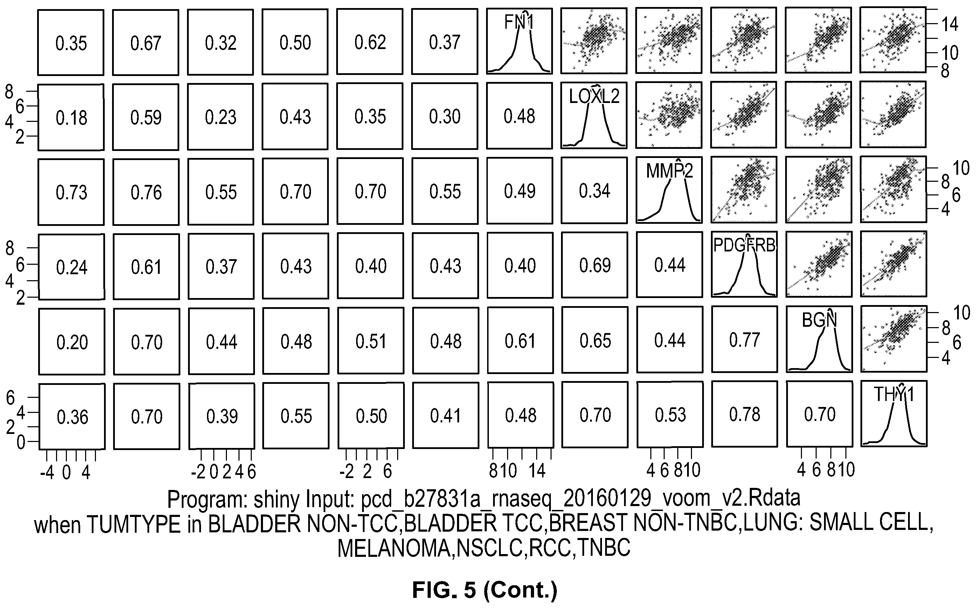

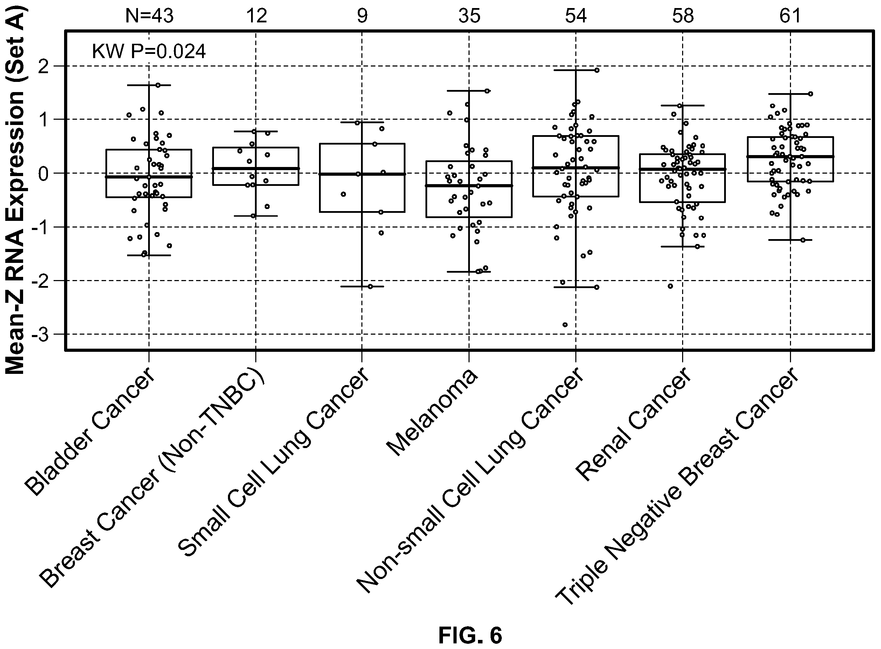

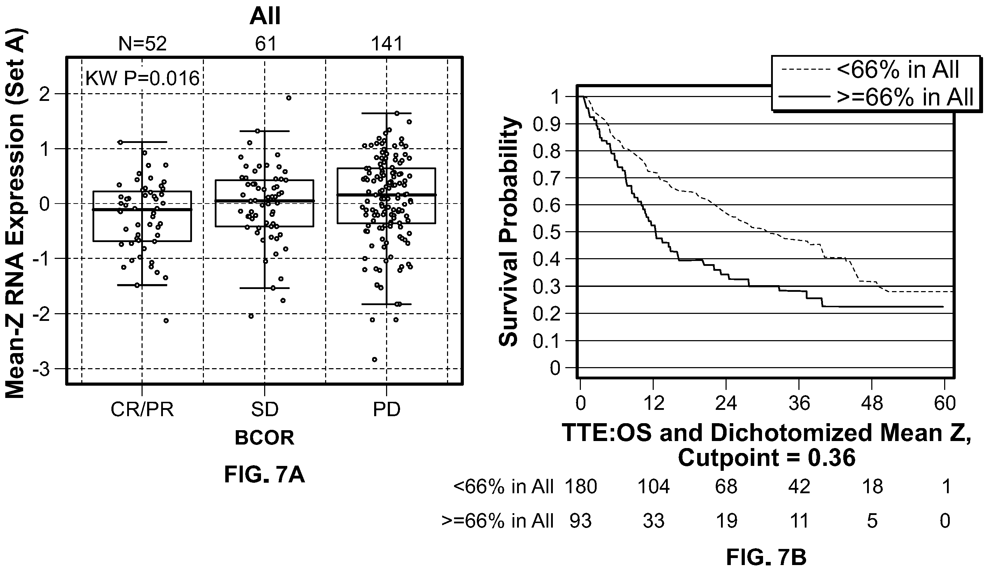

Variability of expression of the signature between tumor indications as well as intra-indications was observed, indicating a dynamic expression range of the Set A stromal signature (**FIG. 5** and **FIG. 6**). The mean expression (mean-Z) of the stroma-associated gene Set A was higher in the PD population compared to the responders (CR/PR) (**FIG. 7A**). Furthermore, when overall survival (OS) was computed based on the level of urothelial stroma-associated gene expression, patients with higher than 0.36 mean-Z expression did poorly with a hazard ratio (HR) of 1.66 (95%CI 1.18 - 2.33) compared to those with lower than 0.36 mean-Z expression cutoff (**FIG. 7B**).

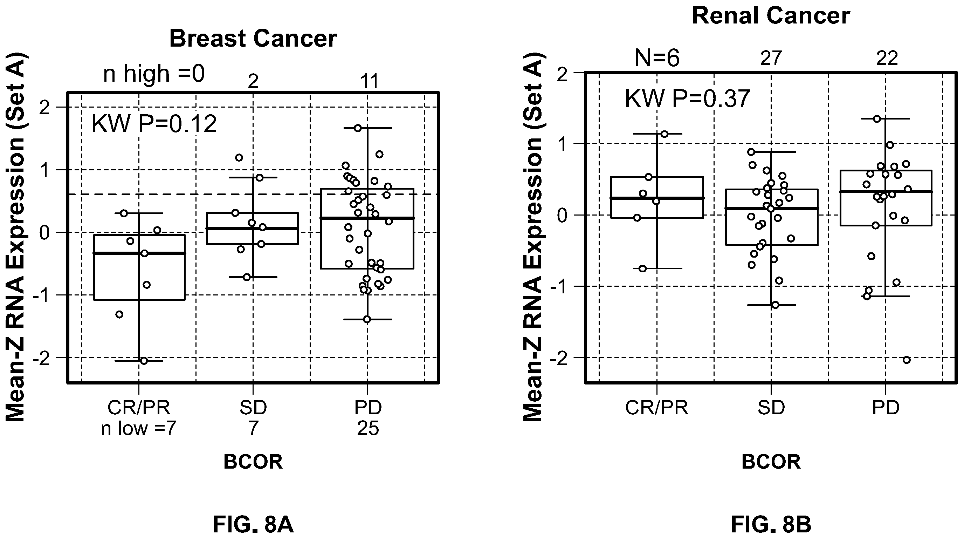

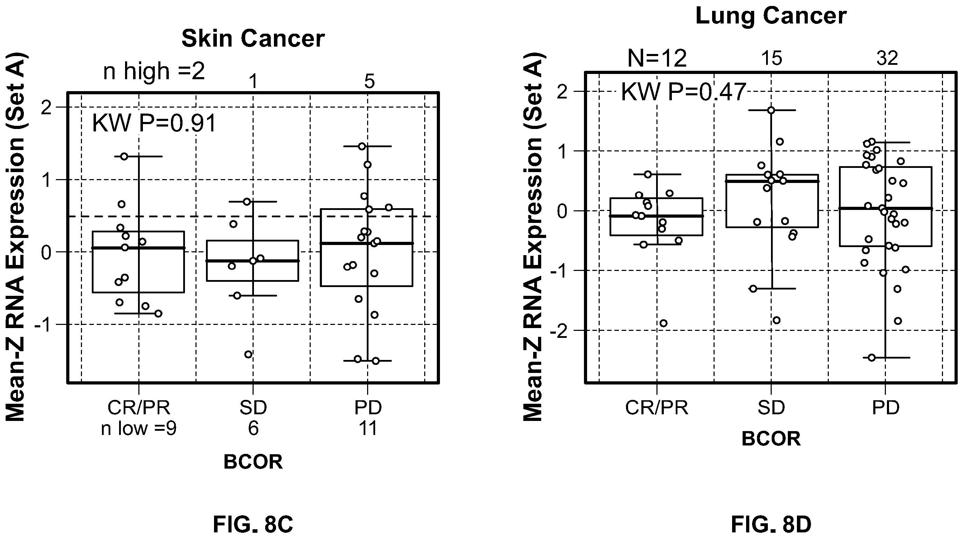

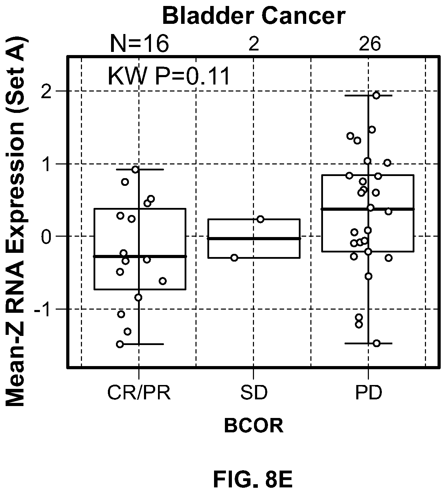

Analysis of the stromal signature in particular tumor types showed trends to response in breast (**FIG. 8A**) and bladder cancer (**FIG. 8E**), but not in melanoma skin cancer (**FIG. 8C**), lung cancer (**FIG. 8D**) or renal cancer (**FIG. 8B**).

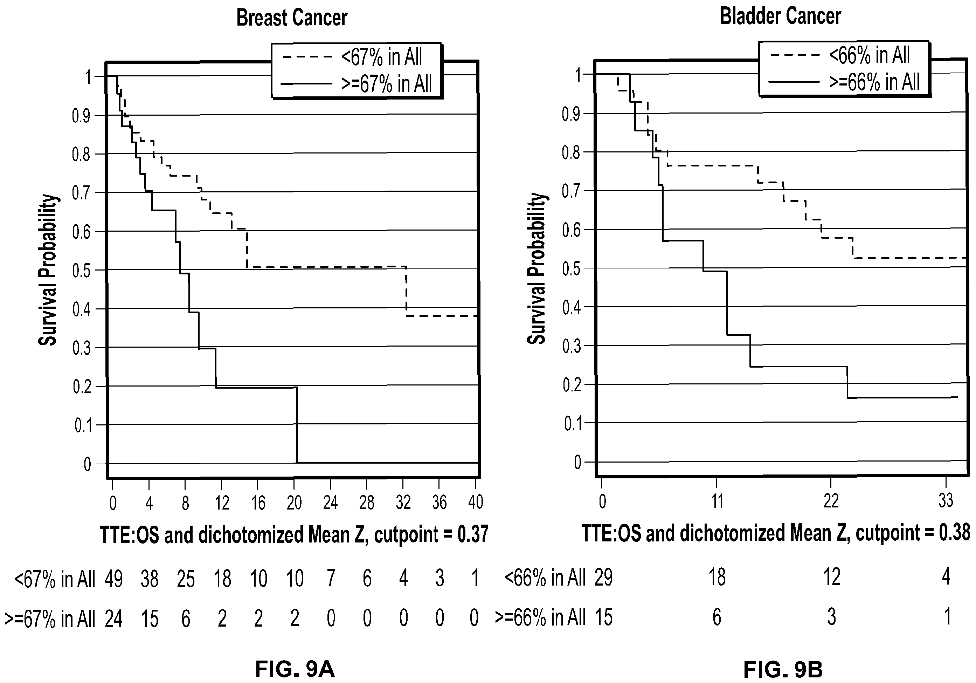

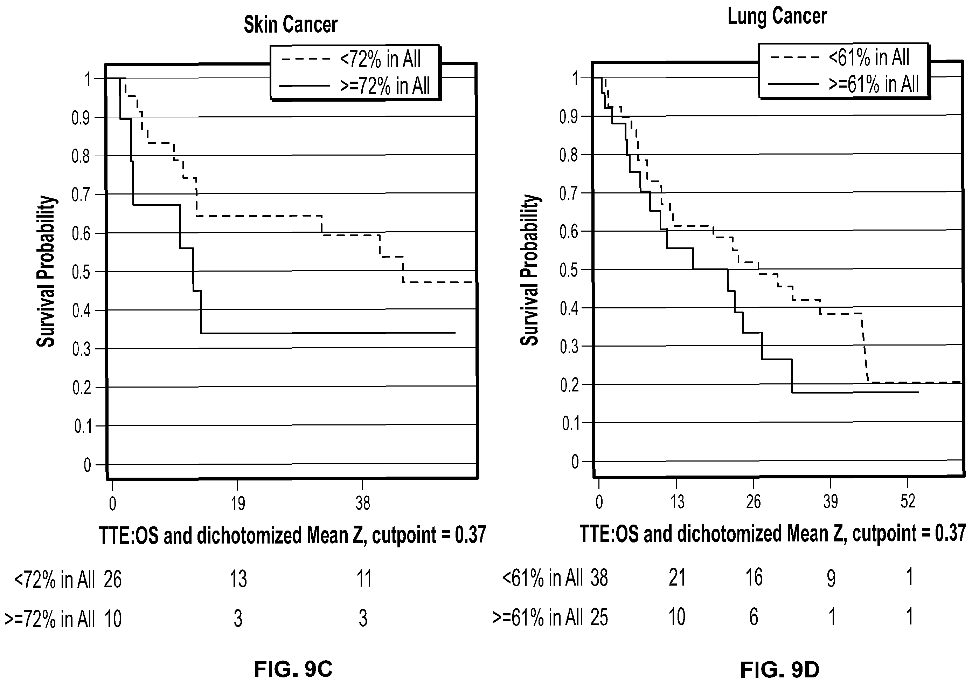

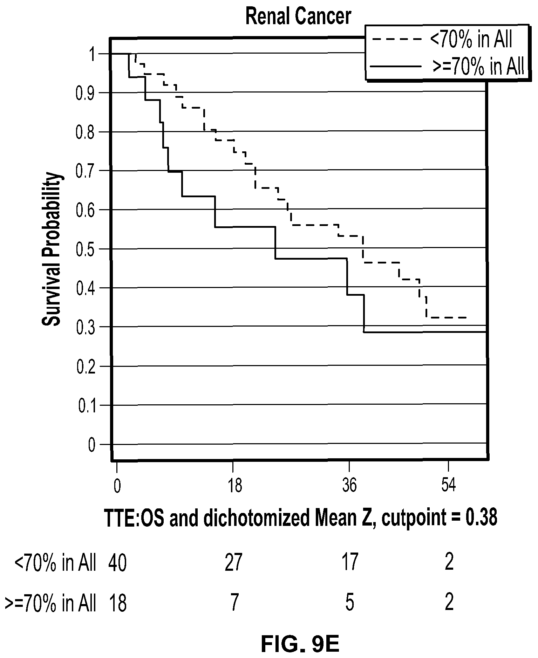

The impact of the gene expression signature in overall survival (OS) for individual indications of patients treated with anti-PD-L1 was evaluated. Using a cutoff of 0.36 for k-means, breast (**FIG. 9A**), bladder (**FIG. 9B**) and skin (**FIG. 9C**) cancer patients had OS associated to this signature, while in lung (**FIG. 9D**) and renal (**FIG. 9E**) cancer patients there was a non-significant trend. Hazard ratio values for patients treated with anti-PD-L1 are shown in Table 2.

**Table 2:** Hazard Ratios for Patients Treated with anti-PD-L1

| Tumor Type | Hazard Ratio at Mean Z = 0.36 | Confidence Interval 95% |
|---|---|---|
| Breast Cancer (n=73) | 2.52 | 1.23-5.16 |
| Bladder Cancer (n=44) | 2.76 | 1.18-6.49 |
| Renal Cancer (n=58) | 1.47 | 0.69-3.13 |
| Skin Cancer (n=36) | 1.89 | 0.7-5.13 |
| Lung Cancer (n=63) | 1.51 | 0.79-2.87 |

**Example 3: TGFb blockade improves anti-PDL1 efficacy in the EMT6 breast tumor model**

*Introduction*

The impact of TGFb blockade on the efficacy of anti-PDL1 treatment was evaluated in a mouse breast tumor model.

*Material and Methods*

*Efficacy Mouse Study 1*

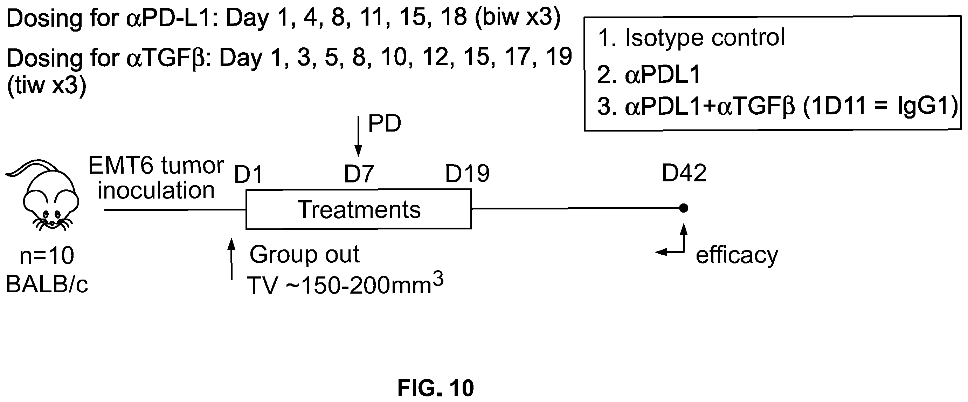

70 Balb/c mice from Charles River Laboratories were inoculated on the 5th mammary fat pad with 0.1 million EMT6 cells in 100 microliters of HBSS+matrigel (1:1). When tumors achieved a mean tumor volume of approximately 150-200 mm$^3$ (approximately 7-8 days after tumor cell inoculation), the animals were recruited into the treatment groups outlined below (Day 0). Mice not recruited into the treatment groups due to dissimilar tumor volume were euthanized. Treatment was initiated the next day after grouping out the mice (Day 1). 'Tiw' indicates dosage three times per week, while 'biw' indicates dosage two times per week. An exemplary experimental diagram is shown in **FIG. 10.**

> **Group 1:** Mu IgG1 anti-gp120 (9338), 10 mg/kg IV first dose, followed by 5 mg/kg IP biwx3 + Mu IgG2a anti-gp120 (9239), 10 mg/kg IV first dose, followed by IP tiwx3, n=10.
> **Group 2:** Mu IgG1 anti-PD-L1 (6E11), 10 mg/kg IV first dose, followed by 5 mg/kg IP biwx3, n=10.
> **Group 3:** Mu IgG1 anti-PD-L1 (6E11), 10 mg/kg IV first dose, followed by 5 mg/kg IP biwx3 + Mu IgG2b anti-TGFβ (2G7.5A9), 10 mg/kg IV first dose, followed by IP tiwx3, n=10.
> **Group 4:** Mu IgG1 anti-PD-L1 (6E11), 10 mg/kg IV first dose, followed by 5 mg/kg IP biwx3 + Mu IgG1 anti-TGFβ (1D11), 10 mg/kg IV first dose, followed by IP tiwx3, n=10.

Mu IgG1 anti-PD-L1 and Mu IgG1 anti-gp120 antibodies were administered on Days 1, 4, 8, 11, 15, 18; Mu IgG2b and Mu IgG1 anti-TGFβ antibodies were dosed on Days 1, 3, 5, 8, 10, 12, 15, 17, 19.

All antibodies were diluted in 20 mM histidine acetate, 240 mM sucrose, 0.02% Polysorbate 20 (Tween-20), pH 5.5. Total daily dosed volume was 350 μL or less. Tumor measurements and body weights were collected 2X/week. Animals exhibiting weight loss of > 15% were weighed daily and euthanized if they lost >20% of body weight. Animals were observed for clinical issues 2x/week. Animals showing adverse clinical issues were observed more frequently, up to daily depending on severity, and euthanized if moribund. Mice were euthanized if tumor volumes exceeded 2,000 mm$^3$, or after 3 months if tumors did not form.

*Efficacy Mouse Study 2*

70 Balb/c mice from Charles River Laboratories were inoculated on the 5th mammary fat pad with 0.1 million EMT6 cells in 100 microliters of HBSS+matrigel (1:1). When tumors achieved a mean tumor volume of approximately 150-200 mm$^3$ (approximately 7-8 days after inoculation), animals were recruited into the treatment groups outlined below (Day 0). Mice not recruited into the treatment groups due to dissimilar tumor volume were euthanized. Treatment was initiated on Day 1. 'Tiw' indicates dosage three times per week, while 'biw' indicates dosage two times per week.

> **Group 1:** Mu IgG1 anti-gp120 (9338), 10 mg/kg IV first dose, followed by 5 mg/kg IP biwx3, + Mu IgG2a anti-gp120 (9239), 10 mg/kg IV first dose, followed by IP biwx3, n=10.
> **Group 2:** Mu IgG1 anti-PD-L1 (6E11), 10 mg/kg IV first dose, followed by 5 mg/kg IP biwx3, n=10.
> **Group 3:** Mu IgG2b anti-TGFβ (2G7.5A9), 10 mg/kg IV first dose, followed by IP biwx3, n=10.
> **Group 4:** Mu IgG1 anti-TGFβ (1D11) 10 mg/kg IV first, followed by IP, biwx3, n=10.
> **Group 5:** Mu IgG1 anti-PD-L1 (6E11), 10 mg/kg IV first dose, followed by 5 mg/kg IP biwx3 + Mu IgG2b anti-TGFβ (2G7.5A9), 10 mg/kg IV first dose, followed by IP biwx3, n=10.

Mu IgG1 anti-PD-L1 and Mu IgG1 anti-gp120 antibodies were administered on Day 1, 4, 8, 11, 15, 18; Mu IgG2b and Mu IgG1 anti-TGFβ antibodies were dosed on Day 1, 3, 5, 8, 10, 12, 15, 17, 19.

All antibodies were diluted in 20 mM histidine acetate, 240 mM sucrose, 0.02% Polysorbate 20 (Tween-20), pH 5.5. Total daily dosed volume was 350 μL or less. Tumor measurements and body weights were collected 2X/week. Animals exhibiting weight loss of > 15% were weighed daily and euthanized if they lost >20% of body weight. Animals were observed for clinical issues 2x/week. Animals showing adverse clinical issues were observed more frequently, up to daily depending on severity, and euthanized if moribund. Mice were euthanized if tumor volumes exceeded 2,000 mm$^3$, or after 3 months if tumors did not form.

*Tumor processing*

Tumors were collected, cut into small pieces, and digested with dispase (0.8 mg/mL), collagenase P (0.2 mg/mL), and DNaseI (0.1 mg/mL) in RPMI + 2% FBS. The digestion was done as described previously (Fletcher, *et al.* 2011). Briefly, the tumor pieces were incubated at 37°C in the enzymes and mixed every 5 minutes. Every 15 minutes, the samples were pipetted up and down through a wide bore pipette tip. Pieces were allowed to settle to the bottom and any cells in suspension were collected. Then new digestion media was added to the sample. This process was repeated until the tumor was completely digested, usually ~75 minutes total. Then the single cell suspension was filtered, and the cells were counted.

*Flow cytometry*

For FACS staining, 2 million cells were stained with antibodies against T cell markers for 15 minutes on ice. For intracellular stains, the eBioscience FoxP3 kit was used to fix and permeabilize the cells before the addition of antibodies specific for granzyme B or Ki67. For the pSMAD phosflow staining, the cells were immediately fixed in BD phosflow Lyse/Fix buffer for 10 minutes at 37°C. Then they were permeabilized for 30 minutes with ice cold BD Phosflow Perm/Wash buffer III. Finally, the cells were washed and stained with antibodies against CD45 and pSMAD2/3 for 45 minutes on ice. All samples were acquired on a BD Fortessa and analyzed with FlowJo X software.

*Results*

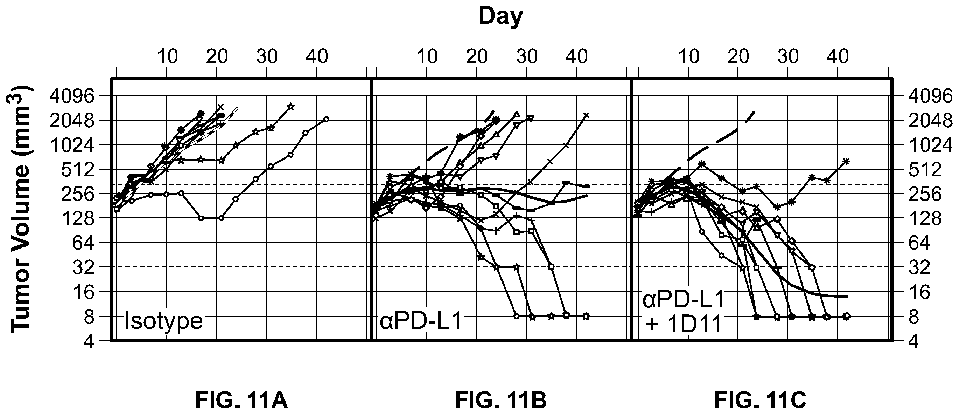

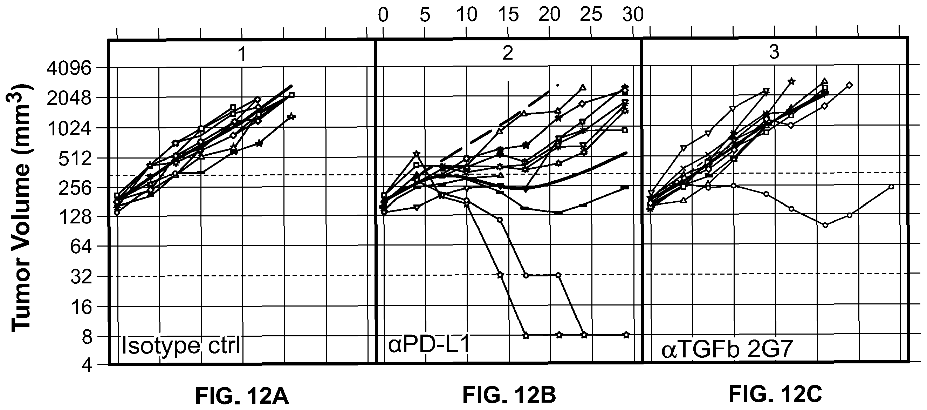

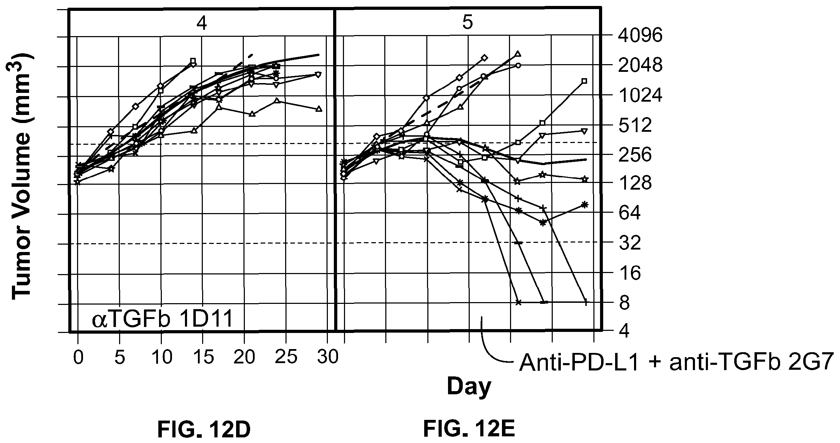

TGFb blockade with 1D11 antibody improved anti-PDL1 efficacy in the EMT6 breast tumor model (Efficacy Mouse Study 1), as indicated by decreased tumor volume in animals treated with both anti-TGFb 1D11 and anti-PDL1 (**FIG. 11C**) as compared to animals treated with anti-PDL1 alone (**FIG. 11B**) or isotype control (**FIG. 11A**). Similarly, 2G7 anti-TGFb antibody improved anti-PDL1efficacy (Efficacy Mouse Study 2), as indicated by decreased tumor volume in animals treated with both anti-TGFb 2G7 and anti-PDL1 (**FIG. 12E**) as compared to animals treated with anti-PDL1 alone (**FIG. 12B**), anti-TGFb 2G7 alone (**FIG. 12C**), anti-TGFb 1D11 alone (**FIG. 12D**), or isotype control (**FIG. 12A**).

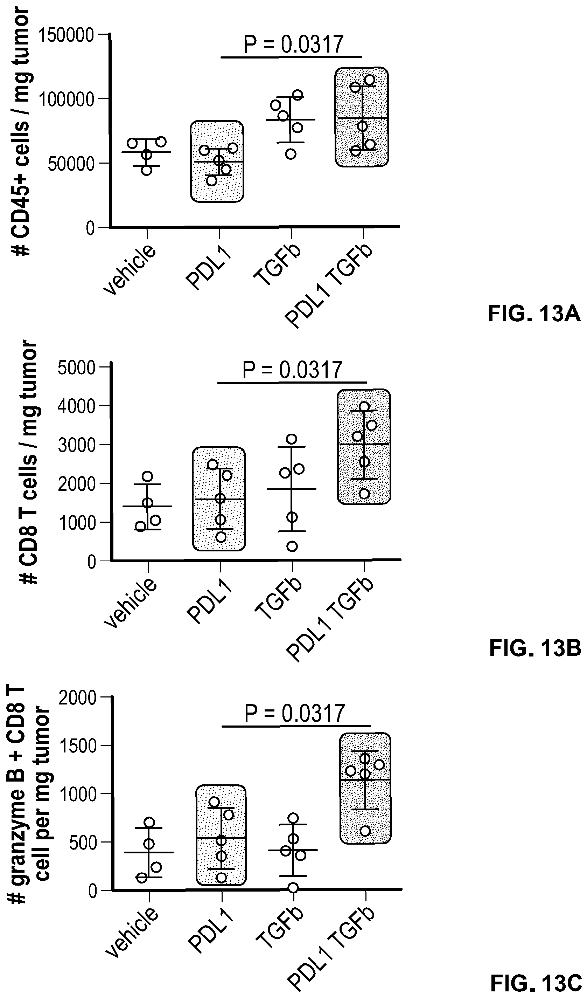

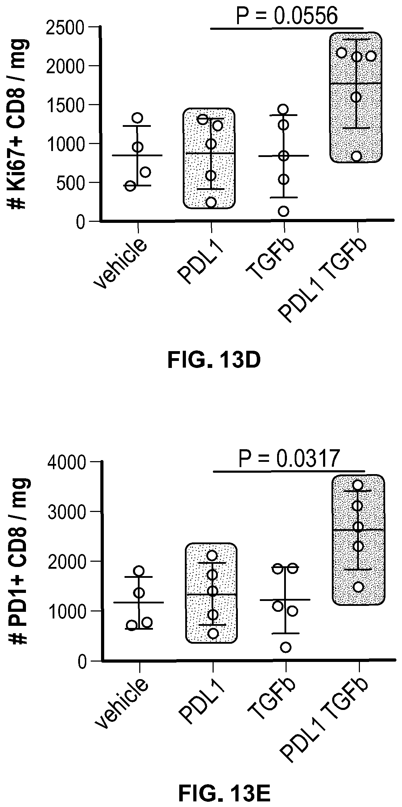

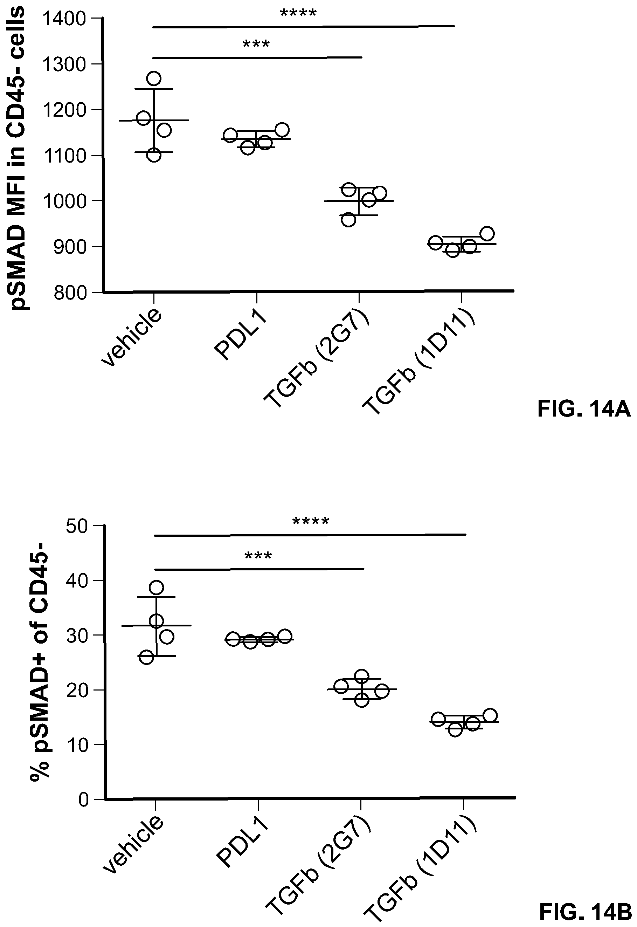

TGFb blockade with 1D11 antibody in combination with anti-PDL1 enhanced CD8 T cell abundance (**FIG. 13B**) and activation, as indicated by the increased abundance of CD45+ cells (**FIG. 13A**), granzyme B+ cells (**FIG. 13C**), Ki67+ cells (**FIG. 13D**), and PD1+ cells (**FIG. 13E**), over anti-PDL1 alone. In addition, TGFb blockade reduced SMAD2/3 phosphorylation in CD45- cells in EMT6 tumors, as indicated by decreased pSMAD MFI (**FIG. 14A**) and decreased percentage of pSMAD+ cells (**FIG. 14B**).

## Example 4: Synergistic effect of TGFb blockade and anti-PDL1 antibody

*Introduction*

The impact of TGFb blockade on the efficacy of anti-PDL1 treatment was evaluated in a mouse breast tumor model.

*Material and Methods*

*Efficacy Mouse Study 3*

70 Balb/c mice from Charles River Laboratories were inoculated on the 5th mammary fat pad with 0.1 million EMT6 cells in 100 microliters of HBSS+matrigel (1:1). When tumors achieved a mean tumor volume of approximately 150-200 mm$^3$ (approximately 7-8 days after tumor cell inoculation), the animals were recruited into the treatment groups outlined below (Day 0). Mice not recruited into the treatment groups due to dissimilar tumor volume were euthanized. Treatment was initiated the next day after grouping out the mice (Day 1). 'BIWx3' indicates dosing bi-weekly for 3 weeks. 'TIWx3' indicates dosing 3 times a week for 3 weeks. 'TIWx4' indicates dosing 3 times a week for 4 weeks.

> **Group 1:** Mu IgG1 anti-PD-L1 (6E11), 10 mg/kg IV first dose, followed by 5 mg/kg IP biwx3, n=10.
> **Group 2:** Mu IgG1 anti-PD-L1 (6E11), 10 mg/kg IV first dose, followed by 5 mg/kg IP tiwx4, n=10.
> **Group 3:** Mu IgG1 anti-PD-L1 (6E11), 10 mg/kg IV first dose, followed by 5 mg/kg IP biwx3, n=10.
> **Group 4:** Mu IgG1 anti-PD-L1 (6E11), 10 mg/kg IV first dose, followed by 5 mg/kg IP biwx3 + Mu IgG2b anti-TGFβ (2G7.5A9), 10 mg/kg IV first dose, followed by 10 mg/kg IP biwx3, n=10.
> **Group 5:** Mu IgG1 anti-PD-L1 (6E11), 10 mg/kg IV first dose, followed by 5 mg/kg IP tiwx4 + Mu IgG2b anti-TGFβ (2G7.5A9), 10 mg/kg IV first dose, followed by 10 mg/kg IP tiwx4, n=10.
> **Group 6:** Mu IgG1 anti-PD-L1 (6E11), 10 mg/kg IV first dose, followed by 5 mg/kg IP biwx3 + Mu IgG2b anti-TGFβ (2G7.5A9), 10 mg/kg IV first dose, followed by 10 mg/kg IP tiwx3, n=10.

Mu IgG1 anti-PD-L1 and Mu IgG1 anti-gp120 antibodies were administered on Days 1, 4, 8, 11, 15, 18; Mu IgG2b and Mu IgG1 anti-TGFβ antibodies were dosed on Days 1, 3, 5, 8, 10, 12, 15, 17, 19.

All antibodies were diluted in 20 mM histidine acetate, 240 mM sucrose, 0.02% Polysorbate 20 (Tween-20), pH 5.5. Total daily dosed volume was 350 μL or less. The anti-TGFb antibody used was clone 2G7 in IgG2b format. Tumor measurements and body weights were collected 2X/week. Animals exhibiting weight loss of > 15% were weighed daily and euthanized if they lost >20% of body weight. Animals were observed for clinical issues 2x/week. Animals showing adverse clinical issues were observed more frequently, up to daily depending on severity, and euthanized if moribund. Mice were euthanized if tumor volumes exceeded 2,000 mm$^3$, or after 3 months if tumors did not form.

*Results*

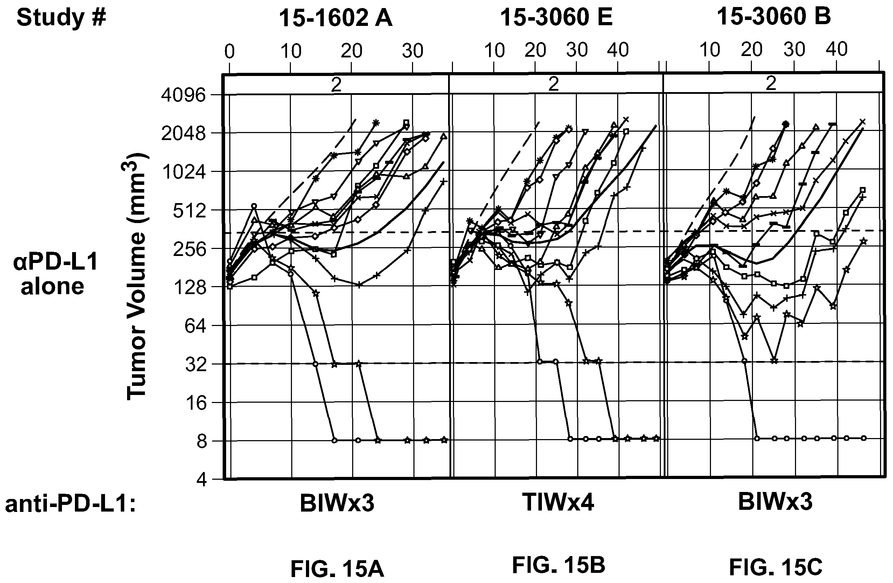

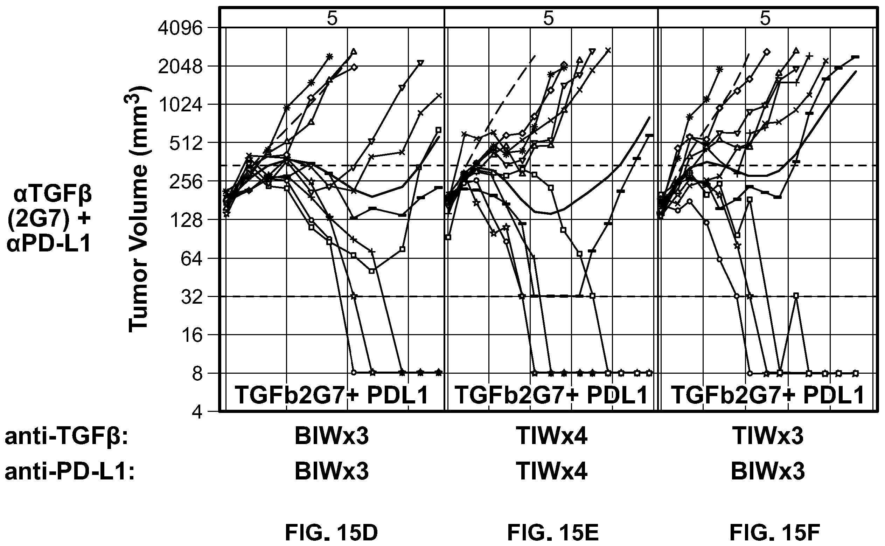

TGFb blockade with 2G7 antibody improved anti-PDL1 efficacy in the EMT6 breast tumor model, as indicated by decreased tumor volume in animals treated with both anti-TGFb 2G7 and anti-PDL1 (**FIG**. **15D, FIG. 15E,** and **FIG. 15F**) as compared to animals treated with anti-PDL1 alone (**FIG**. **15A, FIG. 15B,** and **FIG. 15C**).

## Claims

1. A method for identifying an individual with cancer who is more likely to exhibit benefit from treatment with an immunotherapy and a suppressive stromal antagonist, the method comprising determining the presence of a stromal gene signature in a sample from the individual, said signature comprises *FAP, FN1, MMP2, PDGFRB,* and *THY1,* wherein an increase in the level of expression of the one or more genes in the stroma gene signature relative to a median level identifies an individual having a suppressive stroma wherein the presence of a stromal gene signature in a sample from the individual indicates the individual is more likely to exhibit an increased clinical benefit from an immunotherapy and a suppressive stromal antagonist,

   > wherein the immunotherapy comprises a PD-L1 binding antibody, and
   > wherein the suppressive stromal antagonist is a TGFβ binding antibody.

2. The method of claim 1, wherein the increased clinical benefit further comprises a relative increase in one or more of the following: overall survival (OS), progression free survival (PFS), complete response (CR), partial response (PR) and combinations thereof.

3. The method of claim 1 or 2, wherein the cancer is selected from the group consisting of non-small cell lung cancer, small cell lung cancer, renal cell cancer, colorectal cancer, ovarian cancer, breast cancer, metastatic breast cancer, triple-negative breast cancer, melanoma, pancreatic cancer, gastric carcinoma, bladder cancer, urothelial bladder cancer, esophageal cancer, mesothelioma, melanoma, head and neck cancer, thyroid cancer, sarcoma, prostate cancer, glioblastoma, cervical cancer, thymic carcinoma, leukemia, lymphomas, myelomas, mycoses fungoids, merkel cell cancer, and other hematologic malignancies.

4. The method of claim 3, wherein:

a) the cancer is urothelial bladder cancer (UBC) and the stromal gene signature further comprises one or more of *DKK3, PDGFB, NUAK1, FGF1, PDLIM4* or *LRRC32*

b) wherein the cancer is renal cell cancer (RCC) and the stromal gene signature further comprises *LUM* and/or *POSTN*

c) wherein the cancer is triple-negative breast cancer (TNBC) and the stromal gene signature further comprises one or more of *MMP11, BGN,* or *COL5A1,*or

d) wherein the cancer is ovarian cancer and the stromal gene signature further comprises one or more of *POSTN, LOX,* or *TIMP3.*

5. The method of claim 4, wherein the stromal gene signature further comprises *TGFβ.*

6. The method of any one of claims 1-5, wherein the sample obtained from the individual is a tumor tissue sample, optionally wherein the tumor tissue sample comprises tumor cells, tumor infiltrating immune cells, stromal cells and any combinations thereof; or a whole blood sample, optionally wherein the whole blood comprises immune cells, circulating tumor cells and any combinations thereof.

7. The method of any of claims 1-6, wherein the stromal gene signature is detected in the sample using a method selected from the group consisting of FACS, Western blot, ELISA, immunoprecipitation, immunohistochemistry, immunofluorescence, radioimmunoassay, dot blotting, immunodetection methods, HPLC, surface plasmon resonance, optical spectroscopy, one or more reagents for determining the presence of a stromal gene signature in a sample from an individual mass spectrometery, HPLC, qPCR, RT-qPCR, multiplex qPCR or RT-qPCR, RNA-seq, microarray analysis, SAGE, MassARRAY technique, and FISH, and combinations thereof.

8. The method of claim 7, wherein the stromal gene signature is detected on tumor cells, tumor infiltrating immune cells, stromal cells and any combinations thereof, optionally wherein staining is membrane staining, cytoplasmic staining and combinations thereof.

9. The method of any of claims 1-8, wherein the median levels of the stromal gene signature is selected from the group consisting of (1) the level of the stromal gene signature from a reference population; (2) the level of the stromal gene signature from a population of complete responders and/or partial responders to the immunotherapy; and (3) the level of the stromal gene signature from the individual at a second time point prior to the first time point.

10. A pharmaceutical composition comprising an immunotherapy and a suppressive stromal antagonist for use in treatment of an individual with cancer, wherein the individual is determined to be more likely to exhibit an increased clinical benefit from an immunotherapy and a suppressive stromal antagonist in accordance with the method of any one of claims 1-9, ,

wherein the immunotherapy comprises a PD-L1 binding antibody, and
wherein the suppressive stromal antagonist is a TGFβ binding antibody.

**Patentansprüche**

1. Verfahren zum Identifizieren eines Individuums mit Krebs, das mit höherer Wahrscheinlichkeit einen Nutzen aus einer Behandlung mit einer Immuntherapie und einem suppressiven Stromaantagonisten zieht, wobei das Verfahren das Bestimmen der Gegenwart einer Stromagensignatur in einer Probe von dem Individuum umfasst, wobei die Signatur *FAP, FN1, MMP2, PDGFRB* und *THY1* umfasst, wobei eine Erhöhung des Expressionsniveaus des einen Gens oder der mehreren Gene in der Stromagensignatur in Bezug auf ein medianes Niveau ein Individuum mit einem suppressiven Stroma identifiziert, wobei die Gegenwart einer Stromagensignatur in einer Probe von dem Individuum angibt, dass das Individuum mit höherer Wahrscheinlichkeit einen größeren klinischen Nutzen aus einer Immuntherapie und einem suppressiven Stromaantagonisten zieht,

wobei die Immuntherapie einen PD-L1-bindenden Antikörper umfasst und
wobei der suppressive Stromaantagonist ein TGFb-bindender Antikörper ist.

2. Verfahren nach Anspruch 1, wobei der größere klinische Nutzen ferner eine relative Erhöhung bei einem oder mehreren von Folgenden umfasst: Gesamtüberleben (OS), progressionsfreies Überleben (PFS), vollständiges Ansprechen (CR), teilweises Ansprechen (PR) und Kombinationen davon.

3. Verfahren nach Anspruch 1 oder 2, wobei der Krebs aus der Gruppe ausgewählt ist, die aus nicht-kleinzelligem Lungenkrebs, kleinzelligem Lungenkrebs, Nierenzellenkrebs, Dickdarmkrebs, Eierstockkrebs, Brustkrebs, metastatischem Brustkrebs, dreifach negativem Brustkrebs, Melanom, Bauchspeicheldrüsenkrebs, Magenkarzinom, Blasenkrebs, Harnblasenkrebs, Speiseröhrenkrebs, Mesotheliom, Melanom, Kopf- und Halskrebs, Schilddrüsenkrebs, Sarkom, Prostatakrebs, Glioblastom, Gebärmutterhalskrebs, Thymuskarzinom, Leukämie, Lymphomen, Myelomen, Mycosis fungoides, Merkelzellenkarzinom und anderen hämatologischen Malignitäten besteht.

4. Verfahren nach Anspruch 3, wobei:

a) der Krebs Harnblasenkrebs (UBC) ist und die Stromagensignatur ferner eine oder mehrere von *DKK3, PDGFB, NUAK1, FGF1, PDL1M4* oder *LRRC32* umfasst;
b) wobei der Krebs Nierenzellenkrebs (RCC) ist und die Stromagensignatur ferner *LUM* und/oder *POSTN* umfasst;
c) wobei der Krebs dreifach negativer Brustkrebs (TNBC) ist und die Stromagensignatur ferner eine oder mehrere von *MMP11, BGN* oder *COL5AI* umfasst, oder
d) wobei der Krebs Eierstockkrebs ist und die Stromagensignatur ferner eine oder mehrere von *POSTN, LOX* oder *TIMP3* umfasst.

5. Verfahren nach Anspruch 4, wobei die Stromagensignatur ferner *TGFb* umfasst.

6. Verfahren nach einem der Ansprüche 1-5, wobei die von dem Individuum erhaltene Probe eine Tumorgewebeprobe ist, gegebenenfalls wobei die Tumorgewebeprobe Tumorzellen, tumorinfiltrierende Immunzellen, Stromazellen und jegliche Kombinationen davon umfasst; oder
eine Vollblutprobe ist, gegebenenfalls wobei das Vollblut Immunzellen, zirkulierende Tumorzellen und jegliche Kombinationen davon umfasst.

7. Verfahren nach einem der Ansprüche 1-6, wobei die Stromagensignatur in der Probe unter Anwendung eines Verfahrens nachgewiesen wird, das aus der Gruppe ausgewählt ist, die aus FACS, Western Blot, ELISA, Immunpräzipitation, Immunhistochemie, Immunfluoreszenz, Radioimmunassay, Dot-Blotting, Immunnachweisverfahren, HPLC, Oberflächen-Plasmonresonanz, optischer Spektroskopie, Massenspektrometrie mit einem oder mehreren Reagenz(ien) zum Bestimmen der Gegenwart einer Stromagensignatur in einer Probe von einem Individuum, HPLC, qPCR, RT-qPCR, Multiplex-qPCR oder RT-qPCR, RNA-seq, Mikroarrayanalyse, SAGE, MassARRAY-Technik und FISH und Kombinationen davon besteht.

8. Verfahren nach Anspruch 7, wobei die Stromagensignatur in Tumorzellen, tumorinfiltrierenden Immunzellen, Stromazellen und jeglichen Kombinationen davon nachgewiesen wird, gegebenenfalls wobei Färben Membranfärben, zytoplasmatisches Färben und Kombinationen davon ist.

9. Verfahren nach einem der Ansprüche 1-8, wobei das mediane Niveau der Stromagensignatur aus der Gruppe ausgewählt ist, die aus (1) dem Niveau der Stromagensignatur aus einer Referenzpopulation; (2) dem Niveau der Stromagensignatur aus einer Population von Individuen mit vollständigem und/oder teilweisem Ansprechen auf die Immuntherapie und (3) dem Niveau der Stromagensignatur von dem Individuum zu einem zweiten Zeitpunkt vor dem ersten Zeitpunkt besteht.

10. Pharmazeutische Zusammensetzung, umfassend eine Immuntherapie und einen suppressiven Stromaantagonisten, zur Verwendung bei der Behandlung eines Individuums mit Krebs, wobei gemäß dem Verfahren nach einem der Ansprüche 1-9 bestimmt wurde, dass das Individuum mit höherer Wahrscheinlichkeit einen größeren klinischen Nutzen aus einer Immuntherapie und einem suppressiven Stromaantagonisten zieht,

wobei die Immuntherapie einen PD-L1-bindenden Antikörper umfasst und
wobei der suppressive Stromaantagonist ein TGFb-bindender Antikörper ist.

**Revendications**

1. Procédé destiné à l'identification d'un individu atteint d'un cancer qui est plus susceptible de présenter un bénéfice suite à un traitement avec une immunothérapie et un antagoniste stromal suppressif, le procédé comprenant la détermination de la présence d'une signature génétique stromale dans un échantillon provenant de l'individu, ladite signature comprend *FAP, FN1, MMP2, PDGFRB* et *THY1,* dans lequel une augmentation du niveau d'expression du ou des gènes dans la signature génétique stromale par rapport à un niveau médian identifie un individu ayant un stroma suppressif dans lequel la présence d'une signature génétique stromale dans un échantillon provenant de l'individu indique que l'individu est plus susceptible de présenter un bénéfice clinique accru suite à une immunothérapie et un antagoniste stromal suppressif,

   dans lequel l'immunothérapie comprend un anticorps de liaison à PD-L1, et
   dans lequel l'antagoniste stromal suppressif est un anticorps de liaison à TGFβ.

2. Procédé selon la revendication 1, dans lequel le bénéfice clinique accru comprend en outre une augmentation relative d'une ou plusieurs des suivantes : la survie globale (SG), la survie sans progression (SSP), la réponse complète (RC), la réponse partielle (RP) et des combinaisons de celles-ci.

3. Procédé selon la revendication 1 ou 2, dans lequel le cancer est choisi dans le groupe constitué par un cancer du poumon non à petites cellules, un cancer du poumon à petites cellules, un cancer à cellules rénales, un cancer colorectal, un cancer de l'ovaire, un cancer du sein, un cancer du sein métastatique, un cancer du sein triple négatif, un mélanome, un cancer du pancréas, un carcinome gastrique, un cancer de la vessie, un cancer de la vessie urothélial, un cancer de l'œsophage, un mésothéliome, un mélanome, un cancer de la tête et du cou, un cancer de la thyroïde, un sarcome, un cancer de la prostate, un glioblastome, un cancer du col de l'utérus, un carcinome thymique, une leucémie, des lymphomes, des myélomes, des mycoses fongoïdes, un cancer à cellules de Merkel et d'autres malignités hématologiques.

4. Procédé selon la revendication 3, dans lequel :

   a) le cancer est un cancer urothélial de la vessie (CUV) et la signature génétique stromale comprend en outre un ou plusieurs parmi *DKK3, PDGFB, NUAK1, FGF1, PDL1M4* ou *LRRC32*
   b) dans lequel le cancer est un cancer à cellules rénales (CCR) et la signature génétique stromale comprend en outre *LUM* et/ou *POSTN*
   c) dans lequel le cancer est un cancer du sein triple négatif (CSTN) et la signature génétique stromale comprend en outre un ou plusieurs parmi *MMP11, BGN* ou *COL5A1,* ou
   d) dans lequel le cancer est un cancer de l'ovaire et la signature génétique stromale comprend en outre un ou plusieurs parmi *POSTN, LOX* ou *TIMP3.*

5. Procédé selon la revendication 4, dans lequel la signature génétique stromale comprend en outre *TGFβ*.

6. Procédé selon l'une quelconque des revendications 1 à 5, dans lequel l'échantillon obtenu auprès de l'individu est un échantillon de tissu tumoral, éventuellement dans lequel l'échantillon de tissu tumoral comprend des cellules tumorales, des cellules immunitaires infiltrant la tumeur, des cellules stromales et de quelconques combinaisons de celles-ci ; ou un échantillon de sang total, éventuellement dans lequel le sang total comprend des cellules immunitaires, des cellules tumorales circulantes et de quelconques combinaisons de celles-ci.

7. Procédé selon l'une quelconque des revendications 1 à 6, dans lequel la signature génétique stromale est détectée dans l'échantillon en utilisant une méthode choisie dans le groupe constitué par le FACS, le transfert de Western, l'ELISA, l'immunoprécipitation, l'immunohistochimie, l'immunofluorescence, le dosage radio-immunologique, le transfert en point, les méthodes d'immunodétection, la HPLC, la résonance plasmonique de surface, la spectroscopie optique, un ou plusieurs réactifs pour déterminer la présence d'une signature génétique stromale dans un échantillon provenant d'un individu, la spectrométrie de masse, la HPLC, la qPCR, la RT-qPCR, la qPCR ou la RT-qPCR multiplexe, le séquençage d'ARN, l'analyse par microréseau, la méthode SAGE, la technique MassARRAY et la FISH ou des combinaisons de ceux-ci.

8. Procédé selon la revendication 7, dans lequel la signature génétique stromale est détectée sur des cellules tumorales, des cellules immunitaires infiltrant la tumeur, des cellules stromales et de quelconques combinaisons de celles-ci, éventuellement dans lequel la coloration est une coloration de membrane, une coloration cytoplasmique et des combinaisons de celles-ci.

9. Procédé selon l'une quelconque des revendications 1 à 8, dans lequel le niveau médian de la signature génétique stromale est choisi dans le groupe constitué par (1) le niveau de la signature génétique stromale d'une population de référence ; (2) le niveau de la signature génétique stromale provenant d'une population de répondants complets et/ou de répondants partiels à l'immunothérapie ; et (3) le niveau de la signature génétique stromale provenant de l'individu en un second point de temps antérieur au premier point de temps.

10. Composition pharmaceutique comprenant une immunothérapie et un antagoniste stromal suppressif destinée à une utilisation dans le traitement d'un individu atteint d'un cancer, dans laquelle l'individu est déterminé comme étant plus susceptible de présenter un bénéfice clinique accru suite à une immunothérapie et un antagoniste stromal suppressif conformément au procédé selon l'une quelconque des revendications 1 à 9,

dans laquelle l'immunothérapie comprend un anticorps de liaison à PD-L1, et
dans laquelle l'antagoniste stromal suppressif est un anticorps de liaison à TGFβ.

**All**

FIG. 1A

TTE:OS and Dichotomized Mean Z,
Cutpoint = -0.0037

| | | | | |
|---|---|---|---|---|
| <50% in All | 125 | 90 | 67 | 21 |
| >=50% in All | 126 | 75 | 54 | 17 |

FIG. 1B

EP 3 458 608 B1

**FIG. 2**

**All**

N=42     45     130

KW P=0.027

TGFB1 Expression

CR/PR     SD     PD

**BCOR-IRF**

**FIG. 3A**

**All**

N=42     45     130

KW P=0.049

DKK3 Expression

CR/PR     SD     PD

**BCOR-IRF**

**FIG. 3B**

EP 3 458 608 B1

**FIG. 3C**

**FIG. 3D**

FIG. 3F

FIG. 3E

**All**

**FIG. 3G**

FIG. 4B

FIG. 4A

**FIG. 5**

Program: shiny Input: pcd_b27831a_rnaseq_20160129_voom_v2.Rdata
when TUMTYPE in BLADDER NON-TCC,BLADDER TCC,BREAST NON-TNBC,LUNG: SMALL CELL,
MELANOMA,NSCLC,RCC,TNBC

**FIG. 5 (Cont.)**

EP 3 458 608 B1

FIG. 6

**All**

N=52  61  141

KW P=0.016

Mean-Z RNA Expression (Set A)

CR/PR    SD    PD

BCOR

**FIG. 7A**

‑‑‑‑ <66% in All
——— >=66% in All

Survival Probability

TTE:OS and Dichotomized Mean Z,
Cutpoint = 0.36

| | 0 | 12 | 24 | 36 | 48 | 60 |
|---|---|---|---|---|---|---|
| <66% in All | 180 | 104 | 68 | 42 | 18 | 1 |
| >=66% in All | 93 | 33 | 19 | 11 | 5 | 0 |

**FIG. 7B**

EP 3 458 608 B1

**Breast Cancer**

FIG. 8A

**Renal Cancer**

FIG. 8B

EP 3 458 608 B1

FIG. 8D

FIG. 8C

EP 3 458 608 B1

FIG. 8E

**Breast Cancer**

TTE:OS and dichotomized Mean Z, cutpoint = 0.37

| <67% in All | 49 | 38 | 25 | 18 | 10 | 10 | 7 | 6 | 4 | 3 | 1 |
| >=67% in All | 24 | 15 | 6 | 2 | 2 | 2 | 0 | 0 | 0 | 0 | 0 |

**FIG. 9A**

**Bladder Cancer**

TTE:OS and dichotomized Mean Z, cutpoint = 0.38

| <66% in All | 29 | 18 | 12 | 4 |
| >=66% in All | 15 | 6 | 3 | 1 |

**FIG. 9B**

FIG. 9D

FIG. 9C

**FIG. 9E**

EP 3 458 608 B1

Dosing for αPD-L1: Day 1, 4, 8, 11, 15, 18 (biw x3)

Dosing for αTGFβ: Day 1, 3, 5, 8, 10, 12, 15, 17, 19 (tiw x3)

1. Isotype control
2. αPDL1
3. αPDL1+αTGFβ (1D11 = IgG1)

**FIG. 10**

**Day**

FIG. 11A          FIG. 11B          FIG. 11C

EP 3 458 608 B1

FIG. 12A

FIG. 12B

FIG. 12C

FIG. 12D

FIG. 12E

**FIG. 13A**

**FIG. 13B**

**FIG. 13C**

FIG. 13D

FIG. 13E

FIG. 14A

FIG. 14B

FIG. 15A      FIG. 15B      FIG. 15C

FIG. 15D          FIG. 15E          FIG. 15F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014151006 A2
- US 5641870 A
- EP 404097 A
- WO 9311161 A
- WO 2005035572 A
- WO 03035694 A
- WO 0029004 A
- WO 02051870 A
- US 4816567 A
- US 5693780 A
- US 5500362 A
- US 5821337 A
- WO 2010077634 A1
- US 5571714 A
- WO 9208480 A
- WO 9200330 A
- WO 9526203 A
- WO 9713844 A
- WO 00066631 A
- WO 05097832 A
- WO 06086469 A
- WO 06116002 A
- WO 07076391 A
- WO 12167143 A
- WO 13134365 A
- WO 14164709 A
- US 4275149 A
- US 4737456 A
- US 4318980 A

- US 4016043 A
- US 4424279 A
- US 4018653 A
- WO 200386467 A
- WO 9607321 A
- US 4892538 A
- US 5283187 A
- WO 9325673 A
- WO 9316185 A
- US 5571894 A
- US 5587458 A
- WO 9308829 A
- US 5731168 A
- WO 2009080251 A
- WO 2009080252 A
- WO 2009080253 A
- WO 2009080254 A
- WO 2009089004 A1
- US 4676980 A
- US 20060025576 A1
- US 20080069820 A
- US 5648237 A, Carter
- US 8241901 P
- WO 2005063816 A
- US 5639635 A
- WO 2002061090 A
- US 5264365 A, Georgiou
- US 5508192 A, Georgiou

**Non-patent literature cited in the description**

- **TURLEY, S.J et al.** *Nature Reviews Immunology*, 2015, vol. 15, 669-682
- **JOYCE, J. A** ; **POLLARD, J. W**. *Nat. Rev. Cancer*, 2009, vol. 9, 239-252
- **POLYAK, K et al.** *Trends Genet*, 2009, vol. 25, 30-38
- **NAKASONE, E. S et al.** *Cancer Cell*, 2012, vol. 21, 488-503
- **PIVARCSI, A et al.** *Proc. Natl Acad. Sci. USA*, 2007, vol. 104, 19055-19060
- **POWLES, T et al.** *Nature*, 2014, vol. 515, 558-562
- **HERBST, R. S et al.** *Nature*, 2014, vol. 515, 563-567
- **TUMEH, P. C et al.** *Nature*, 2014, vol. 515, 568-571

- **NICOLAOU et al.** *Angew. Chem Intl. Ed. Engl.*, 1994, vol. 33, 183-186
- **WILMAN**. Prodrugs in Cancer Chemotherapy. *Biochemical Society Transactions*, 1986, vol. 14, 375-382
- Prodrugs: A Chemical Approach to Targeted Drug Delivery. **STELLA et al.** Directed Drug Delivery. Humana Press, 1985, 247-267
- Cell cycle regulation, oncogenes, and antineoplastic drugs. **MURAKAMI et al.** The Molecular Basis of Cancer. WB Saunders, 1995, 13
- **KABAT et al.** Sequences of Proteins of Immunological Interest. Public Health Service, National Institutes of Health, 1991
- **CHOTHIA** ; **LESK**. *J. Mol. Biol*, 1987, vol. 196, 901-917

- **BURTON**. *Molec. Immunol*, 1985, vol. 22, 161-206
- **ZAPATA et al.** *Protein Eng.*, 1995, vol. 8 (10), 1057-1062
- **PLÜCKTHUN**. The Pharmacology of Monoclonal Antibodies. Springer-Verlag, 1994, vol. 113, 269-315
- **HOLLINGER et al.** *Proc. Natl. Acad. Sci. USA*, 1993, vol. 90, 6444-6448
- *Nature*, 1989, vol. 341, 544-546
- *Dev Comp Immunol*, 2006, vol. 30, 43-56
- *Trend Biochem Sci*, 2001, vol. 26, 230-235
- *Trends Biotechnol*, 2003, vol. 21, 484-490
- *FEBS Lett*, 1994, vol. 339, 285-290
- **KOHLER et al.** *Nature*, 1975, vol. 256, 495
- **CLACKSON et al.** *Nature*, 1991, vol. 352, 624-628
- **MARKS et al.** *J. Mol. Biol.*, 1991, vol. 222, 581-597
- **MORRISON et al.** *Proc. Natl. Acad. Sci. USA*, 1984, vol. 81, 6851-6855
- **JONES et al.** *Nature*, 1986, vol. 321, 522-525
- **RIECHMANN et al.** *Nature*, 1988, vol. 332, 323-329
- **PRESTA**. *Curr. Op. Struct. Biol*, 1992, vol. 2, 593-596
- **GAZZANO-SANTORO et al.** *J. Immunol. Methods*, 1996, vol. 202, 163
- **RAVETCH** ; **KINET**. *Annu. Rev. Immunol*, 1991, vol. 9, 457-92
- **CLYNES et al.** *Proc. Natl. Acad. Sci. (USA)*, 1998, vol. 95, 652-656
- **DAËRON**. *Annu. Rev. Immunol.*, 1997, vol. 15, 203-234
- **CAPEL et al.** *Immunomethods*, 1994, vol. 4, 25-34
- **HAAS et al.** *J. Lab. Clin. Med.*, 1995, vol. 126, 330-41
- **GUYER et al.** *J. Immunol.*, 1976, vol. 117, 587
- **KIM et al.** *J. Immunol.*, 1994, vol. 24, 249
- **KINDT et al.** Kuby Immunology. W.H. Freeman and Co, 2007, 91
- **PORTOLANO et al.** *J. Immunol.*, 1993, vol. 150, 880-887
- **CLARKSON et al.** *Nature*, 1991, vol. 352, 624-628
- **CHEN, DS** ; **MELLMAN**. *Immunity*, 2013, vol. 39, 1-10
- **ROSENBLOOM, J et al.** *Biochimica et Biophysica Acta*, 2013, vol. 1832, 1088-1103
- *Current Protocols In Molecular Biology*, 1995
- **LOTTSPEICH**. Bioanalytik. Spektrum Akademisher Verlag, 1998
- **SAMBROOK** ; **RUSSELL**. Molecular Cloning: A Laboratory Manual, CSH Press. Cold Spring Harbor, 2001
- **WU et al.** *J. Biol. Chem.*, 1987, vol. 262, 44294432
- **WAGNER et al.** *Proc. Natl. Acad. Sci. USA*, 1990, vol. 87, 3410-3414
- **ANDERSON et al.** *Science*, 1992, vol. 256, 808-813
- **GODING**. Monoclonal Antibodies: Principles and Practice. Academic Press, 1986, 59-103
- **KOZBOR**. *J. Immunol.*, 1984, vol. 133, 3001
- **BRODEUR et al.** Monoclonal Antibody Production Techniques and Applications. Marcel Dekker, Inc, 1987, 51-63
- **MUNSON et al.** *Anal. Biochem.*, 1980, vol. 107, 220
- **SKERRA et al.** *Curr. Opinion in Immunol*, 1993, vol. 5, 256-262
- **PLÜCKTHUN**. *Immunol. Revs.*, 1992, vol. 130, 151-188
- **MCCAFFERTY et al.** *Nature*, 1990, vol. 348, 552-554
- **MARKS et al.** *Bio/Technology*, 1992, vol. 10, 779-783
- **WATERHOUSE et al.** *Nuc. Acids. Res.*, 1993, vol. 21, 2265-2266
- **MORRISON et al.** *Proc. Natl Acad. Sci. USA*, 1984, vol. 81, 6851
- **MORIMOTO et al.** *Journal of Biochemical and Biophysical Methods*, 1992, vol. 24, 107-117
- **BRENNAN et al.** *Science*, 1985, vol. 229, 81
- **CARTER et al.** *Bio/Technology*, 1992, vol. 10, 163-167
- **CUNNINGHAM** ; **WELLS**. *Science*, 1989, vol. 244, 1081-1085
- **A. L. LEHNINGER**. Biochemistry. Worth Publishers, 1975, 73-75
- **MILSTEIN** ; **CUELLO**. *Nature*, 1983, vol. 305, 537
- **TRAUNECKER et al.** *EMBO J.*, 1991, vol. 10, 3655
- **KOSTELNY et al.** *J. Immunol.*, 1992, vol. 148 (5), 1547-1553
- **GRUBER et al.** *J. Immunol.*, 1994, vol. 152, 5368
- **TUTT et al.** *J. Immunol.*, 1991, vol. 147, 60
- **SIEBENLIST et al.** *Cell*, 1980, vol. 20, 269
- Cellular and Molecular Biology. **BACHMANN**. American Society for Microbiology. Washington, D.C, 1987, vol. 2, 1190-1219
- **BASS et al.** *Proteins*, 1990, vol. 8, 309-314
- **SIMMONS et al.** *J. Immunol. Methods*, 2002, vol. 263, 133-147
- **HARA et al.** *Microbial Drug Resistance*, 1996, vol. 2, 63-72
- **LINDMARK et al.** *J. Immunol. Meth.*, 1983, vol. 62, 1-13